# EUROPEAN PATENT SPECIFICATION

(11) **EP 3 601 226 B1**
(45) Date of publication and mention of the grant of the patent: **28.10.2026**
(21) Application number: 18770931.6
(22) Date of filing: 20.03.2018
(51) Int. Cl.: C07D 211/56, C07D 209/40, C07D 213/75, C07D 487/04, C07D 471/04, C07D 215/38, C07D 261/20, C07D 231/40, C07C 311/21, C07C 311/29, C07C 259/06, C07C 231/02, C07C 235/60, A61K 31/44, A61K 31/403, A61K 31/167, A61P 35/00, C07C 233/75, C07D 295/192

(54) **HEAT SHOCK PROTEIN 90 INHIBITORS**
INHIBITOREN DES HITZESCHOCKPROTEINS 90
INHIBITEURS DE LA PROTÉINE DE CHOC THERMIQUE 90

(30) Priority: 20.03.2017 US 201762473708 P
(43) Date of publication of application: 05.02.2020
(73) Proprietor: Taipei Medical University, Taipei City 110 (TW)
(72) Inventor: LIOU, Jing-ping, Taipei City 110 (TW); TENG, Che-ming, Taipei City 110 (TW); PAN, Shiow-lin, Taipei City 106 (TW)
(74) Representative: J A Kemp LLP
(86) International application number: PCT/CN2018/079563
(87) International publication number: WO 2018/171575

(56) References cited:
- EP-A1- 1 512 396
- WO-A1-01/44172
- WO-A1-2006/109085
- WO-A1-2006/117669
- WO-A1-2015/040425
- WO-A2-2006/109075
- WO-A2-2008/044027
- WO-A2-2009/066060
- JU HUI JEONG ET AL: "Targeting the entry region of Hsp90's ATP binding pocket with a novel 6,7-dihydrothieno[3,2-c]pyridin-5(4H)-yl amide", EUROPEAN JOURNAL OF MEDICINAL CHEMISTRY, vol. 124, 1 November 2016 (2016-11-01), NL, pages 1069 - 1080, XP055540911, ISSN: 0223-5234, DOI: 10.1016/j.ejmech.2016.10.038
- CIKOTIENE I ET AL: "5-Aryl-4-(5-substituted-2,4-dihydroxyphenyl)-1,2,3-thiadiazoles as inhibitors of Hsp90 chaperone", BIORGANIC & MEDICINAL CHEMISTRY LETTERS, ELSEVIER, AMSTERDAM , NL, vol. 19, no. 4, 15 February 2009 (2009-02-15), pages 1089 - 1092, XP025937219, ISSN: 0960-894X, [retrieved on 20090109], DOI: 10.1016/J.BMCL.2009.01.003
- JIAN-MIN JIA ET AL: "Hybrids of the Benzofuran Core from Natural Products and the 2,4-Dihydroxy-5-isopropylbenzene Fragment as Potent Hsp90 Inhibitors: Design, Synthesis and Bioevaluation", MOLECULAR INFORMATICS, vol. 33, no. 8, 1 August 2014 (2014-08-01), pages 495 - 502, XP55757376, ISSN: 1868-1743, DOI: 10.1002/minf.201300182
- CHEMICAL CATALOG: "1-(2,4-dihydroxyphenyl)-2-thiazolyl-ethanone derivative", STNNEXT- REGISTRY, 1 November 2000 (2000-11-01), XP55768181
- CHEMICAL CATALOG: "1(2,4-dihydroxyphenyl)-2-thiazolyl-ethanone derivative", STNNEXT-REGISTRY, 2 November 2000 (2000-11-02), XP55768182, Retrieved from the Internet <URL:www.stn.org> [retrieved on 20210125]
- CHEMICAL CATALOG: "1(2,4-dihydroxyphenyl)-2-pyridinyl derivative", STNNEXT-REGISTRY, 2 November 2000 (2000-11-02), XP55768183, Retrieved from the Internet <URL:www.stn.org> [retrieved on 20210125]
- CHEMICAL CATALOG: "1-(2,4-dihydroxyphenyl)-2-benzimidazolyl-ethanone derivative", REGISTRY, 3 November 2000 (2000-11-03), XP55768185, Retrieved from the Internet <URL:www.stn.org> [retrieved on 20210125]
- CHEMICAL CATALOG: "1-(2,4-dighydroxyphenyl)-2-benzofuranyl-ethanone derivative", STNNEXT-REGISTRY, 9 January 2001 (2001-01-09), XP55768187, Retrieved from the Internet <URL:www.stn.org> [retrieved on 20210125]
- JU HUI JEONG ET AL.: "Targeting the entry region of Hsp90's ATP binding pocket with a novel 6, 7-dihydrothieno[3, 2-c]pyridin-5(4H)-yl amide", EUROPEAN JOURNAL OF MEDICINAL CHEMISTRY, vol. 124, 18 October 2016 (2016-10-18), pages 1069 - 1080, XP055540911, ISSN: 0223-5234

## Description

### BACKGROUND

Molecular chaperones guide polypeptides to fold correctly so that they lower the risk of protein assembly while exposing to the protein-rich intracellular environment. Heat shock protein 90 (HSP90), a molecular chaperone, functions as a stabilizer and activator of HSP90 client proteins, such as protein kinase B, cyclin-dependent kinase 4, and fms-like tyrosine kinase 3. Certain HSP90 client proteins, crucial in cell growth, are termed oncoproteins.

HSP90 has become an attractive target due to its close connection with numerous client proteins. One of the advantages of inhibiting HSP90 is that the inhibition causes simultaneous shut-down of several oncoproteins. As a result, inhibition of HSP90 leads to gradual degradation of certain oncoproteins and eventually suppresses cancer growth.

Several HSP90 inhibitors have been identified and tested in clinical trials. In general, HSP90 inhibitors are categorized into two groups: natural product and synthetic molecule. Natural HSP90 inhibitors derive from 17-allylamino-17-desmethoxygeldanamycin (17-AAG), of which the structures typically have a symbolic quinone ring fused with a macrocycle ring. Synthetic HSP90 inhibitors are classified as purine derivatives (e.g., BIIB021 from Biogen Idec and NVP-HSP990 from Novatis), resorcinol derivatives (e.g., NVP-AUY922 from Novatis and AT13387 from Astex), and benzamide derivatives (e.g., SNX-2112 from Pfizer). These HSP90 inhibitors have certain drawbacks, including difficult synthesis, low efficacy, and poor safety margin.

There is a need to develop new HSP90 inhibitors that do not have the above-described drawbacks.
WO2006109075 describes pharmaceutical compounds for use as inhibitors of HSP90.

### SUMMARY

The present invention is based on an unexpected discovery that certain substituted aromatic compounds effectively inhibit HSP90 with good safety profiles.

In one aspect, this invention relates to substituted aromatic compounds of formula (I):

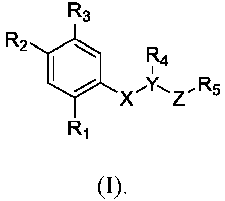

In this formula:
X is CO;
Y is N;
Z is:
   (i) aryl selected from phenyl, 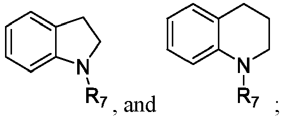 or
   (ii) heteroaryl selected from the group consisting of 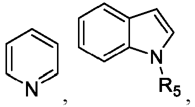 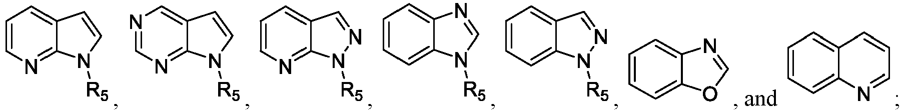
each of R₁ and R₂, independently, is OH or NH₂;
R₃ is C₁₋₆ alkyl or halogen;
R₄ is C₁₋₆ alkyl, C₃₋₈ cycloalkyl, C₁₋₆ alkylamine, or C₁₋₆ alkylalcohol; and
R₅ is CF₃, CN, NO₂, NR₆R₇, -NHCO(CH₂)ₙCONHOH, -CONH(CH₂)ₙCONHOH, CONHOH, CO₂NH₂, C₁₋₆ alkoxyl, C₃₋₁₀ cycloalkyl, C₃₋₁₀ heterocycloalkyl, aryl, or heteroaryl when Z is aryl, providing that when Z is phenyl, R₅ is -NHCO(CH₂)ₙCONHOH or - CONH(CH₂)ₙCONHOH; or
R₅ is H, halogen, CF₃, CN, NO₂, NR₆R₇, -NHCO(CH₂)ₙCONHOH, - CONH(CH₂)ₙCONHOH, CONHOH, CO₂NH₂, CH₂NR₆R₇, C₁₋₆ alkyl, C₁₋₆ alkoxyl, C₃₋₁₀ cycloalkyl, C₃₋₁₀ heterocycloalkyl, aryl, or heteroaryl when Z is heteroaryl,
in which
each of R₆ and R₇, independently, is H, C₁₋₆ alkyl, C₁₋₆ alkoxyl, C₃₋₁₀ cycloalkyl, C₃₋₁₀ heterocycloalkyl, aryl, or heteroaryl; or R₆, together with R₇ and the nitrogen atom bonded to R₆ and R₇, is C₃₋₁₀ heterocycloalkyl; or R₆, together with Z and the nitrogen atom bonded to R₆ and R₇, forms a fused bicycle; and
n is 5 to 7.

The term "alkyl" herein refers to a saturated, linear or branched hydrocarbon moiety, such as -CH₃ or branched -C₃H₇. The term "cycloalkyl" refers to a non-aromatic, monocyclic, bicyclic, tricyclic, or tetracyclic hydrocarbon moiety, such as cyclohexyl, or adamantyl. The term "alkoxyl" refers to an -O-alkyl radical. Examples of alkoxy include, but are not limited to, methoxy, ethoxy, n-propoxy, isopropoxy, n-butoxy, iso-butoxy, secbutoxy, and tert-butoxy. The term "heterocycloalkyl" refers to a non-aromatic, monocyclic, bicyclic, tricyclic, or tetracyclic moiety having one or more ring heteroatoms (e.g., N, O, or S), such as 4-tetrahydropyranyl. The term "aryl" refers to a hydrocarbon moiety having one or more aromatic rings. Examples of aryl include, but are not limited to, phenyl (Ph), phenylene, naphthyl, naphthylene, pyrenyl, anthryl, and phenanthryl. The term "heteroaryl" refers to a moiety having one or more aromatic rings that contain at least one heteroatom (e.g., N, O, or S). Examples of heteroaryl include, but are not limited to, furyl, furylene, fluorenyl, pyrrolyl, thienyl, oxazolyl, imidazolyl, thiazolyl, pyridyl, pyrimidinyl, quinazolinyl, quinolyl, isoquinolyl and indolyl. The term "alkylamine" refers to an alkyl that is substituted with at least one amino group. Examples of alkylamine include aminomethyl and 2-aminoethyl. The term "alkylalcohol" refers to an alkyl that is substituted with at least one hydroxyl group. Examples of alkylalcohol include hydroxyl methyl and hydroxyl ethyl.

The substituted aromatic compounds described above include the compounds themselves, as well as their salts, and solvates, if applicable. A salt, for example, can be formed between an anion and a positively charged group (e.g., amino) on a substituted aromatic compound. Suitable anions include chloride, bromide, iodide, sulfate, nitrate, phosphate, citrate, methanesulfonate, trifluoroacetate, acetate, malate, tosylate, tartrate, fumurate, glutamate, glucuronate, lactate, glutarate, and maleate. Likewise, a salt can also be formed between a cation and a negatively charged group (e.g., carboxylate) on a substituted aromatic compound. Suitable cations include sodium ion, potassium ion, magnesium ion, calcium ion, and an ammonium cation such as tetramethylammonium ion. The substituted aromatic compounds also include those salts containing quaternary nitrogen atoms. A solvate refers to a complex formed between an active substituted aromatic compound and a pharmaceutically acceptable solvent. Examples of a pharmaceutically acceptable solvent include water, ethanol, isopropanol, ethyl acetate, acetic acid, and ethanolamine.

The substituted aromatic compounds may contain a non-aromatic double bond and one or more asymmetric centers. Thus, they can occur as racemates or racemic mixtures, single enantiomers, individual diastereomers, diastereomeric mixtures, or cis- or transisomeric forms. All such isomeric forms are contemplated.

Another aspect of this invention relates to a compound of formula (I) for use in a method for treating a medical condition associated with HSP90, wherein the medical condition is cancer; optionally, wherein the cancer is breast cancer, non-small cell lung cancer, gastric cancer, lymphoma, or multiple myeloma. The method includes administering to a subject in need thereof an effective amount of one or more of the substituted aromatic compounds of formula (I) described above.

Also within the scope of this invention is a pharmaceutical composition containing one or more of the above-described substituted aromatic compounds of formula (I). The pharmaceutical composition can be used for treating a medical condition associated with HSP90. An exemplary medical condition associated with HSP90 is cancer.

Also disclosed herein is use of one or more of the above-described substituted aromatic compounds of formula (I) for the manufacture of a medicament for treating a medical condition associated with HSP90 (e.g., cancer).

The term "treating" or "treatment" refers to administering one or more of the substituted aromatic compounds to a subject, who has an above-described disease, a symptom of such a disease, or a predisposition toward such a disease, with the purpose to confer a therapeutic effect, e.g., to cure, relieve, alter, affect, ameliorate, or prevent the above-described disease, the symptom of it, or the predisposition toward it. "An effective amount" refers to the amount of an active compound that is required to confer the therapeutic effect. Effective doses will vary, as recognized by those skilled in the art, depending on the types of disease treated, route of administration, excipient usage, and the possibility of co-usage with other therapeutic treatment.

To practice the method of the present invention, a composition having one or more of the above-described substituted aromatic compounds can be administered parenterally, orally, nasally, rectally, topically, or buccally. The term "parenteral" as used herein refers to subcutaneous, intracutaneous, intravenous, intraperitoneal, intramuscular, intraarticular, intraarterial, intrasynovial, intrasternal, intrathecal, intralesional, or intracranial injection, as well as any suitable infusion technique.

A sterile injectable composition can be a solution or suspension in a non-toxic parenterally acceptable diluent or solvent, such as a solution in 1,3-butanediol. Among the acceptable vehicles and solvents that can be employed are mannitol, water, Ringer's solution, and isotonic sodium chloride solution. In addition, fixed oils are conventionally employed as a solvent or suspending medium (e.g., synthetic mono- or di-glycerides). Fatty acid, such as oleic acid and its glyceride derivatives are useful in the preparation of injectables, as are natural pharmaceutically acceptable oils, such as olive oil and castor oil, especially in their polyoxyethylated versions. These oil solutions or suspensions can also contain a long chain alcohol diluent or dispersant, carboxymethyl cellulose, or similar dispersing agents. Other commonly used surfactants such as Tweens and Spans or other similar emulsifying agents or bioavailability enhancers which are commonly used in the manufacture of pharmaceutically acceptable solid, liquid, or other dosage forms can also be used for the purpose of formulation.

A composition for oral administration can be any orally acceptable dosage form including capsules, tablets, emulsions and aqueous suspensions, dispersions, and solutions. In the case of tablets, commonly used carriers include lactose and corn starch. Lubricating agents, such as magnesium stearate, are also typically added. For oral administration in a capsule form, useful diluents include lactose and dried corn starch. When aqueous suspensions or emulsions are administered orally, the active ingredient can be suspended or dissolved in an oily phase combined with emulsifying or suspending agents. If desired, certain sweetening, flavoring, or coloring agents can be added.

A nasal aerosol or inhalation composition can be prepared according to techniques well known in the art of pharmaceutical formulation. For example, such a composition can be prepared as a solution in saline, employing any suitable preservative or absorption promoter (e.g., benzyl alcohol) or any solubilizing or dispersing agent (e.g., fluorocarbon).

A composition having one or more of the above-described substituted aromatic compounds can also be administered in the form of suppositories for rectal administration.

The carrier in the pharmaceutical composition must be "acceptable" in the sense that it is compatible with the active ingredient of the composition (and preferably, capable of stabilizing the active ingredient) and not deleterious to the subject to be treated. One or more solubilizing agents can be utilized as pharmaceutical excipients for delivery of an active 1,5-diphenyl-penta-1,4-dien-3-one compound. Examples of other carriers include colloidal silicon oxide, magnesium stearate, cellulose, sodium lauryl sulfate, and D&C Yellow # 10.

The details of one or more embodiments of the invention are set forth in the description below. Other features, objects, and advantages of the invention will be apparent from the description and from the claims.

### DETAILED DESCRIPTION

Disclosed in detail below are substituted aromatic compounds of formula (I):

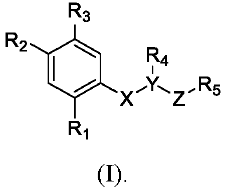

Variables R₁ - R₅, X, Y, and Z are defined in the SUMMARY section above.

One subset of the substituted aromatic compounds of formula (I) includes those in which each of R₁ and R₂ is OH and R₃ is isopropyl. Compounds in this subset can have Z as phenyl and R₅ as -NHCO(CH₂)ₙCONHOH or -CONH(CH₂)ₙCONHOH, or Z as 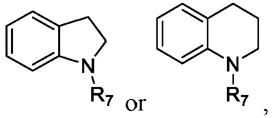 R₇ being C₁₋₆ alkyl. Alternatively, Z can be heteroaryl selected from the group consisting of 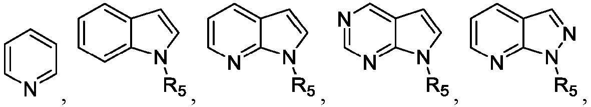 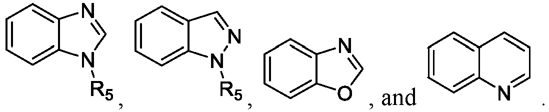 Typically, Z is 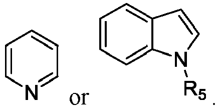 In particular, Z can be 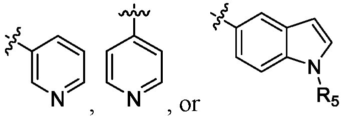 and R₄ can be C₁₋₆ alkyl.
Examples of the substituted aromatic compounds in this subset include, but are not limited to, the following:

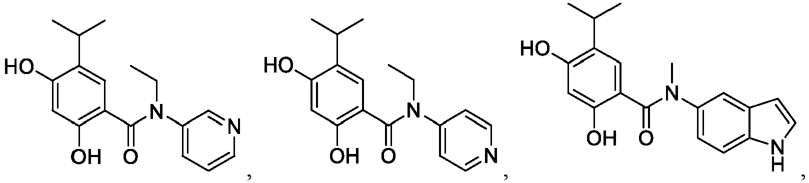

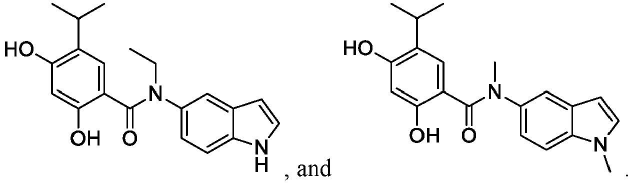

Another subset of the substituted aromatic compounds of formula (I) includes those in which Z is

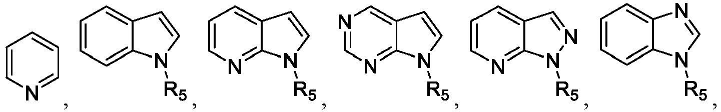

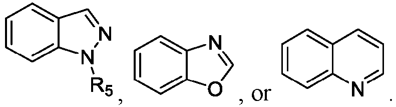

Also within this invention is a pharmaceutical composition containing one or more of the substituted aromatic compounds of formula (I) described above for treating a medical condition associated with HSP90 (e.g., cancer).

Further covered by this invention is a compound of formula (I) for use in treating a a medical condition associated with HSP90, wherein the medical condition is cancer; optionally, wherein the cancer is breast cancer, non-small cell lung cancer, gastric cancer, lymphoma, or multiple myeloma, and optionally, wherein the compound is for oral administration..

Exemplary compounds of formula (I) for use in the above method include, but are not limited to,

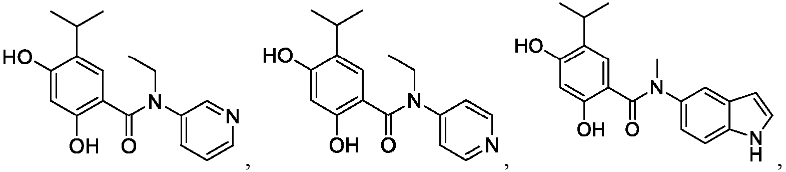

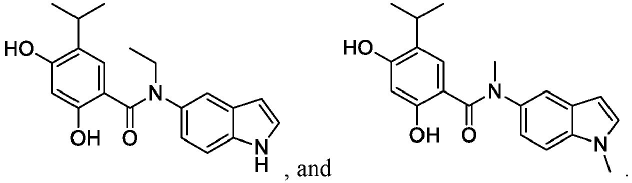

In an exemplary method, compound 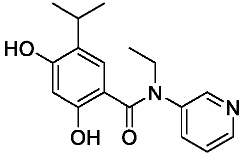 is for use in treating a medical condition associated with HSP90, wherein the medical condition is cancer; optionally, wherein the cancer is breast cancer, non-small cell lung cancer, gastric cancer, lymphoma, or multiple myeloma, and optionally, wherein the compound is for oral administration.

The substituted aromatic compounds of formula (I) described above can be prepared according to methods well known in the field. See, for example, R. Larock, Comprehensive Organic Transformations (2nd Ed., VCH Publishers 1999); P. G. M. Wuts and T. W. Greene, Greene's Protective Groups in Organic Synthesis (4th Ed., John Wiley and Sons 2007); L. Fieser and M. Fieser, Fieser and Fieser's Reagents for Organic Synthesis (John Wiley and Sons 1994); L. Paquette, ed., Encyclopedia of Reagents for Organic Synthesis (2nd ed., John Wiley and Sons 2009); and G. J. Yu et al., J. Med. Chem. 2008, 51, 6044-6054.

Shown in Scheme A below is a synthetic protocol that can be followed to synthesize substituted aromatic compounds of formula (I). More specifically, a variety of benzoic acids (A) are reacted with amino substituted heterocycles in the presence of coupling reagents such as HBTU, EDC, and DCC to afford corresponding amides (B). Aromatic electrophilic substitution like nitration and formylation are performed by respectively reacting with nitric acid and POCl₃/DMF, yielding C and D. Treatment of C with various Grignard reagents followed by oxidation affords benzophenones E. Synthesis of F was fulfilled by the reaction of D with substituted benzoyl chlorides or sulfonyl chlorides. Both E and F are compounds of formula (I).

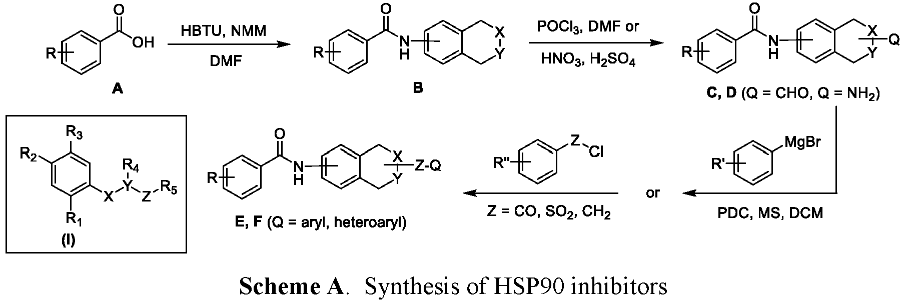

The compounds of formula (I) thus prepared can be initially screened using *in vitro* assays, e.g., the radioligand binding assay described in Example 2 below, for their potency in inhibiting binding of SDF-1 to CXCR4. They can be subsequently evaluated using *in vivo* assays, e.g., a colony-forming assay, for their efficacy in enhancing hematopoietic stem cell mobilization in a mammal. The selected compounds can be further tested to verify their efficacy in treating tissue injury (e.g., acute kidney injury), cancer, inflammatory disease, and autoimmune disease. For example, a compound can be administered to an animal (e.g., a mouse) having ischemic acute kidney injury and its therapeutic effects are then assessed. Based on the results, an appropriate dosage range and administration route can be determined.

Without further elaboration, it is believed that one skilled in the art can, based on the above description, utilize the present invention to its fullest extent. The following specific examples are therefore to be construed as merely illustrative, not limitative, of the remainder of the disclosure in any way.

Shown immediately below are the structures of exemplary compounds of formula (I). The methods for preparing these compounds, as well as the analytical data for the compounds thus prepared, are set forth in EXAMPLE 1 below. The procedures for testing these compounds are described in EXAMPLES 2-4 also below.

In the following examples, compounds 1 to 17, 18 to 32 labelled 'a', 21b, 24b, 26b, 20f, 23f and 36 to 41 are provided for reference only.

| | Number | X | | | R |
|---|---|---|---|---|---|
| | 1a | 4-H | | | H |
| | 1b | | | | Methyl |
| | 1c | | | | Ethyl |
| | 1d | | | | Propyl |
| | 1e | | | | Isopropyl |
| | 2a | 2-F | | | H |
| 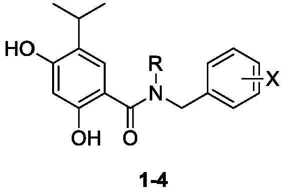 | 2b | | | | Methyl |
| | 2c | | | | Ethyl |
| | 2d | | | | Propyl |
| | 3a | 3-F | | | H |
| | 3b | | | | Methyl |
| | 3c | | | | Ethyl |
| | 3d | | | | Propyl |
| | 3e | | | | Isopropyl |
| | 4a | 4-F | | | H |
| | 4b | | | | Methyl |
| | 4c | | | | Ethyl |
| | 4d | | | | Propyl |
| | 4e | | | | Isopropyl |
| | 5a | 4-H | | | H |
| | 5b | | | | Methyl |
| | 5c | | | | Ethyl |
| | 5d | | | | Propyl |
| 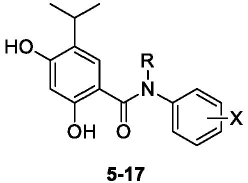 | 5e | | | | Isopropyl |
| | 6a | 2-F | | | H |
| | 6b | | | | Methyl |
| | 6c | | | | Ethyl |
| | 6d | | | | Propyl |
| | 6e | | | | Isopropyl |
| | 7a | 3-F | | | H |
| | 7b | | | | Methyl |
| | 7c | | | | Ethyl |
| | 7d | | | | Propyl |
| | 7e | | | | Isopropyl |
| | 8a | 4-F | | | H |
| 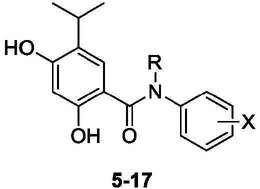 | 8b | | | | Methyl |
| | 8c | | | | Ethyl |
| | 8d | | | | Propyl |
| | 8e | | | | Isopropyl |
| | 9a | 3,4,5-trimethoxy | | | H |
| | 9b | | | | Methyl |
| | 9c | | | | Ethyl |
| | 9d | | | | Propyl |
| | 9e | | | | Isopropyl |
| | 10a | 4-morpholino | | | H |
| | 10b | | | | Methyl |
| | 10c | | | | Ethyl |
| | 10d | | | | Propyl |
| | 10e | | | | Isopropyl |
| | 11a | 2-morpholino | | | H |
| | 11b | | | | Methyl |
| | 11c | | | | Ethyl |
| | 11d | | | | Propyl |
| | 12a | 2-morpholinocarbonyl | | | H |
| | 12b | | | | Methyl |
| | 12c | | | | Ethyl |
| | 12d | | | | Propyl |
| | 13a | 3-morpholinocarbonyl | | | H |
| | 13b | | | | Methyl |
| | 13c | | | | Ethyl |
| | 13d | | | | Propyl |
| | 13e | | | | Isopropyl |
| | 14a | 4-morpholinocarbonyl | | | H |
| | 14b | | | | Methyl |
| | 14c | | | | Ethyl |
| | 14d | | | | Propyl |
| 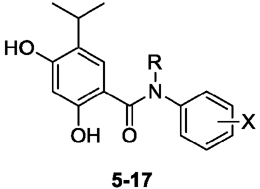 | 14e | | | | Isopropyl |
| | 15a | 2-morpholinomethyl | | | H |
| | 15b | | | | Ethyl |
| | 15c | | | | Propyl |
| | 16a | 3-morpholinomethyl | | | H |
| | 16b | | | | Ethyl |
| | 16c | | | | Propyl |
| | 16d | | | | Isopropyl |
| | 17a | 4-morpholinomethyl | | | H |
| | 17b | | | | Ethyl |
| | 17c | | | | Propyl |
| 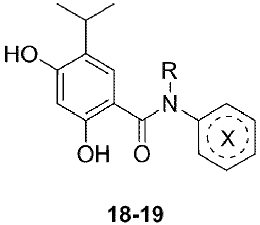 | 18a | 3-aminopyridine | | | H |
| | 18b | | | | Ethyl |
| | 18c | | | | Propyl |
| | 18d | | | | Isopropyl |
| | 19a | 4-aminopyridine | | | H |
| | 19b | | | | Ethyl |
| | 19c | | | | Propyl |
| | 19d | | | | Isopropyl |
| | Number | X | Position | | R |
| 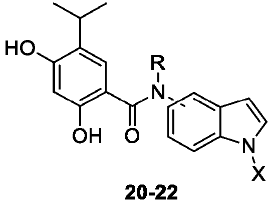 | 20a | H | 5- | | H |
| | 20b | | | | Methyl |
| | 20c | | | | Ethyl |
| | 20d | | | | Propyl |
| | 20e | | | | Isopropyl |
| | 20f | CH₃ | | | H |
| | 20g | | | | Methyl |
| | 20h | | | | Ethyl |
| | 20i | | | | Propyl |
| 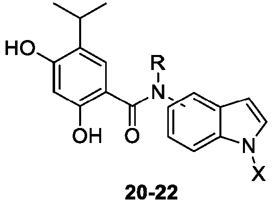 | 21a | H | 6- | | H |
| | 21b | CH₃ | | | H |
| | 21c | | | | Methyl |
| | 21d | | | | Ethyl |
| | 21e | | | | Propyl |
| | 21f | | | | Isopropyl |
| | 22a | CH₃ | 7- | | H |
| | 22b | | | | Methyl |
| | 22c | | | | Ethyl |
| | 22d | | | | Propyl |
| | 22e | | | | Isopropyl |
| 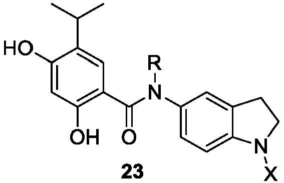 | 23a | H | | | H |
| | 23b | | | | Methyl |
| | 23c | | | | Ethyl |
| | 23d | | | | Propyl |
| | 23e | | | | Isopropyl |
| | 23f | CH₃ | | | H |
| | 23g | | | | Ethyl |
| | 23h | | | | Propyl |
| 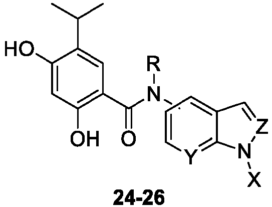 | Number | X | Y | Z | R |
| | 24a | H | N | C | H |
| | 24b | CH₃ | | | H |
| | 24c | | | | Methyl |
| | 24d | | | | Ethyl |
| | 24e | | | | Propyl |
| | 24f | | | | Isopropyl |
| | 25a | H | C | N | H |
| | 25b | CH₃ | | | Methyl |
| | 26a | H | N | N | H |
| | 26b | CH₃ | | | H |
| | 26c | | | | Methyl |
| | 26d | | | | Ethyl |
| | 26e | | | | Propyl |
| | 26f | | | | Isopropyl |

| | Number | | | | R |
|---|---|---|---|---|---|
| 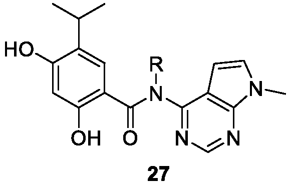 | 27a | | | | H |
| | 27b | | | | Methyl |
| | 27c | | | | Ethyl |
| | 27d | | | | Propyl |
| | 27e | | | | Isopropyl |

| | Number | X | Position | | R |
|---|---|---|---|---|---|
| | 28a | H | 3- | | H |
| | 28b | | | | Methyl |
| | 28c | | | | Ethyl |
| | 28d | | | | Propyl |
| | 29a | H | 5- | | H |
| | 29b | | | | Methyl |
| | 29c | | | | Ethyl |
| 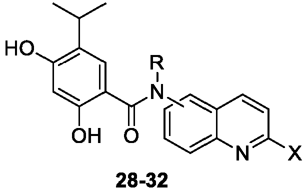 | 30a | H | 6- | | H |
| | 30b | | | | Methyl |
| | 30c | | | | Ethyl |
| | 30d | | | | Propyl |
| | 31a | H | 8- | | H |
| | 31b | | | | Methyl |
| | 31c | | | | Ethyl |
| | 32a | CH₃ | 4- | | H |
| | 32b | | | | Methyl |
| | 32c | | | | Ethyl |

| | Number | X | | | R |
|---|---|---|---|---|---|
| 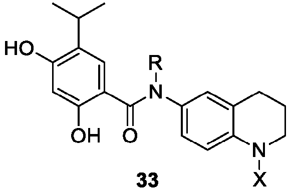 | 33a | H | | | Methyl |
| | 33b | | | | Ethyl |
| | 33c | CH₃ | | | Methyl |
| | 33d | | | | Ethyl |

| | Number | X | | | |
|---|---|---|---|---|---|
| 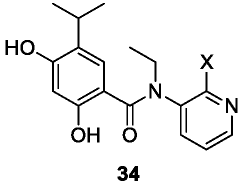 | 34a | Methyl | | | |
| | 34b | O-Benzyl | | | |
| | | | | | |

| | Number | | | | R |
|---|---|---|---|---|---|
| 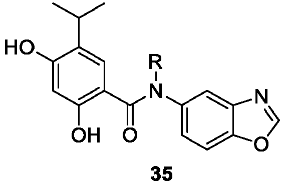 | 35a | | | | Methyl |
| | 35b | | | | Ethyl |

| | Number | X | Y | | R |
|---|---|---|---|---|---|
| | 36a | 3-F | CO | | H |
| | 36b | | | | Methyl |
| | 36c | | | | Ethyl |
| | 36d | | | | Propyl |
| 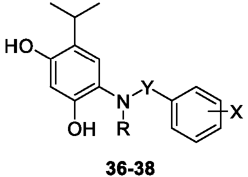 | 36e | | | | Isopropyl |
| | 37a | 4-F | | | H |
| | 37b | | | | Methyl |
| | 37c | | | | Ethyl |
| | 37d | | | | Propyl |
| | 38a | 4-methoxy | SO₂ | | |
| | 38b | 4-F | | | |

| | Number | X | | | |
|---|---|---|---|---|---|
| 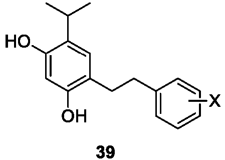 | 39a | 3,4,5-trimethoxy 4-methoxy 4-F | | | |
| | 39b | | | | |
| | 39c | | | | |

| | Number | X | | | |
|---|---|---|---|---|---|
| 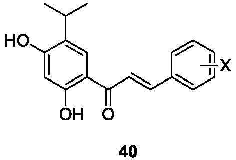 | 40a | 3,4-dimethoxy 2,5-dimethoxy | | | |
| | 40b | | | | |

| | Number | | | | |
|---|---|---|---|---|---|
| 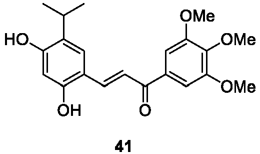 | 41 | | | | |

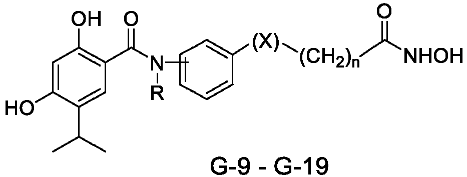

| Number | Position | R | X | n |
|---|---|---|---|---|
| G-9 | Para | H | 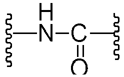 | 6 |
| G-10 | Para | CH₃ | 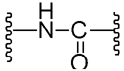 | 6 |
| G-11 | Para | C₂H₅ | 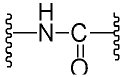 | 6 |
| G-12 | Para | C₃H₇ | 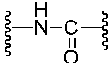 | 6 |
| G-13 | Meta | H | 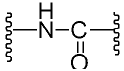 | 6 |
| G-14 | Meta | CH₃ | 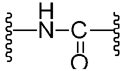 | 6 |
| G-15 | Meta | C₂H₅ | 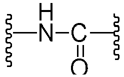 | 6 |
| G-16 | Ortho | H | 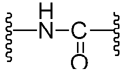 | 6 |
| G-17 | Para | CH₃ | 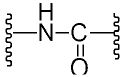 | 5 |
| G-18 | Para | CH₃ | 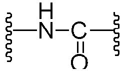 | 7 |
| G-19 | Para | CH₃ | 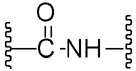 | 6 |

Depicted below are synthetic routes that were followed for synthesizing certain compounds of formula (I) listed above and shown in EXAMPLE 1 below. 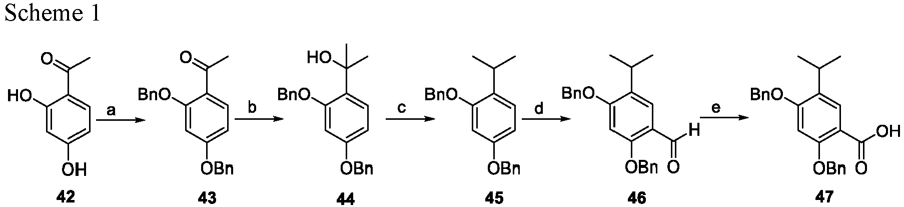 *Reagents and condition
(a) benzyl bromide, K₂CO₃, acetone, reflux, 93%
(b) methylmagnesium bromide, THF, 0°C to r.t., 77%
(c) triethylsilane, TFA, DCM, -78°C to r,t,, 92%
(d) POCl₃, DMF, 0°C to 80°C, 92%
(e) sulfamic acid, NaClO₂, H₂O, THF, DMSO, 0°C to r.t., 65% 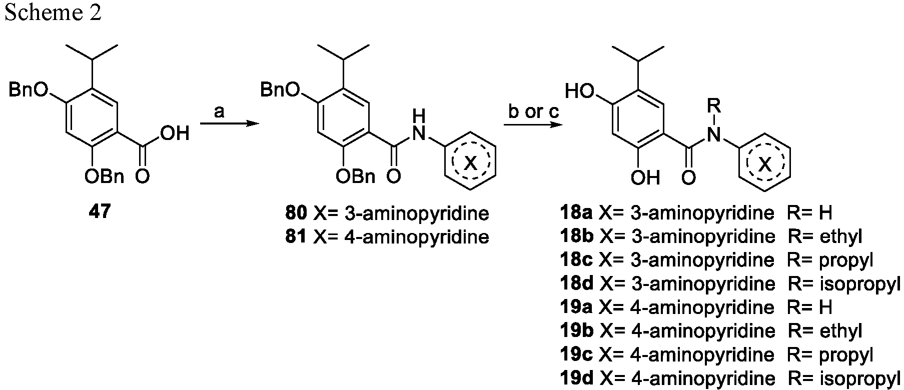
*Reagents and condition
(a) 3-or 4-aminopyridine, EDC, HOBt, NMM, DMF, r.t., for **80-81,** 71-73%
(b) 10% Pd/C, H₂, MeOH, r.t. for **18a, 19a,** 87-88%
(c) alkyl iodide, NaH, DMF, r.t. then10% Pd/C, H₂, MeOH, r.t., **18b-18d, 19a-19d,** 60-70% 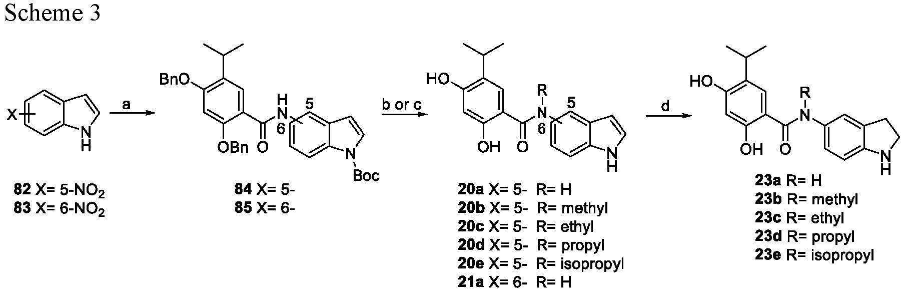
*Reagents and condition
(a) (i) Boc anhydride, DMAP, DCM, r.t. (ii) Fe powder, NH₄Cl, IPA/H₂O, reflux (iii) 47, EDC, HOBt, NMM, DMF, r.t., 79%
(b) (i) TFA, DCM, r.t. (ii) 10% Pd/C, H₂, MeOH, r.t. for **20a,** 73%
(c) (i) alkyl iodide, NaH, DMF, r.t. (ii) TFA, DCM, r.t. (iii) 10% Pd/C, H₂, MeOH, r.t., for **20b-20e, 21a,** 48-57%
(d) NaBH₃CN, acetic acid, 0°C to r.t., 70-76% 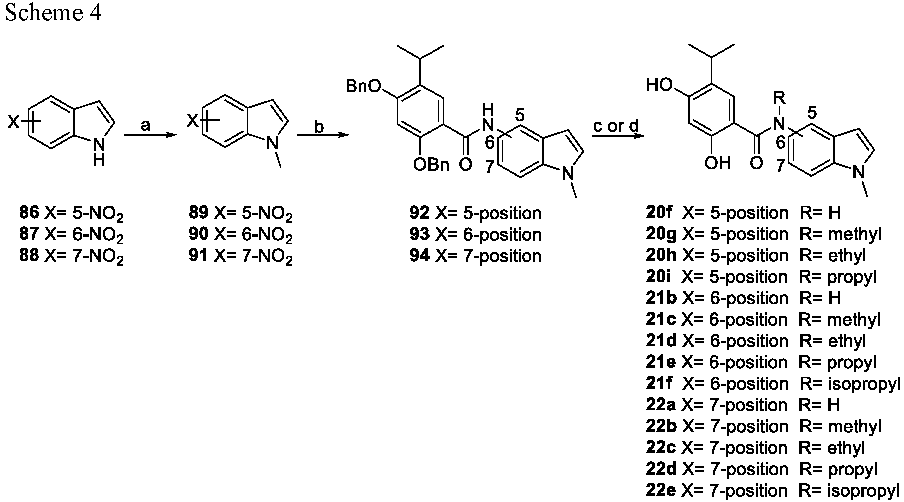
*Reagents and condition
(a) Mel, NaH, DMF, r.t., 92%
(b) (i) Fe powder, NH₄Cl, IPA/H₂O, reflux (ii) **47,** EDC, HOBt, NMM, DMF, r.t., 36%
(c) 10% Pd/C, H₂, MeOH, r.t. for **20f, 21b, 22a,** 81%
(d) alkyl iodide, NaH, DMF, r.t. then 10% Pd/C, H₂, MeOH,THF, 40psi, r.t., for **20g-20i**, 69-75%
(e) alkyl iodide, NaH, DMF, r.t. then 10% Pd/C, H₂, MeOH, for **21c-21e, 22b-22e.** r.t., 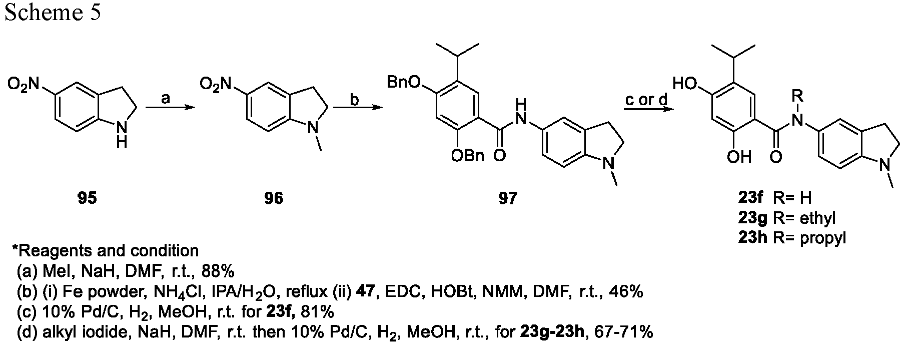 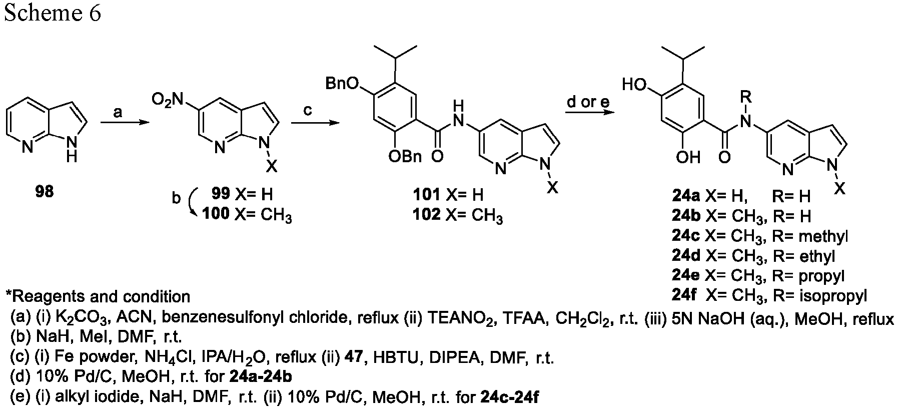 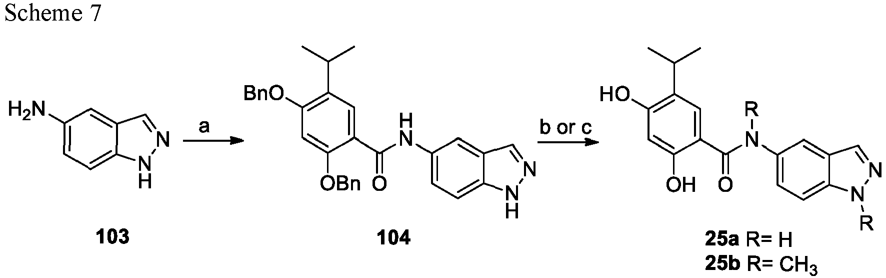
*Reagents and condition
(a) **47,** HBTU, DIPEA, DMF, r.t.
(b) 10% Pd/C, MeOH, r.t. for **25a**
(c) (i) methyl iodide, NaH, DMF, r.t. (ii) 10% Pd/C, MeOH, r.t. for **25b** 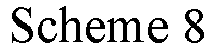 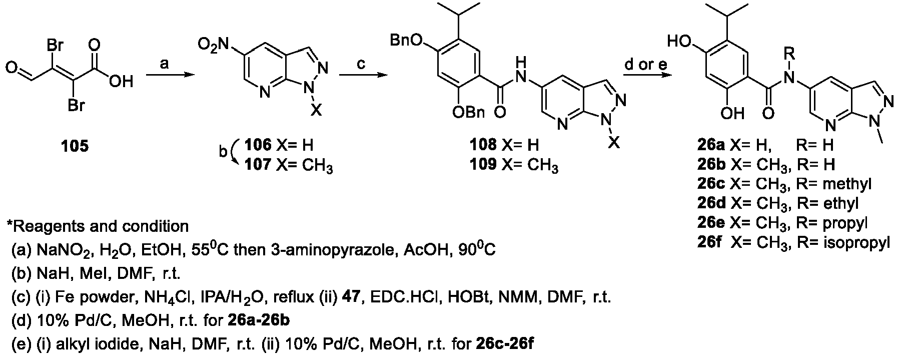 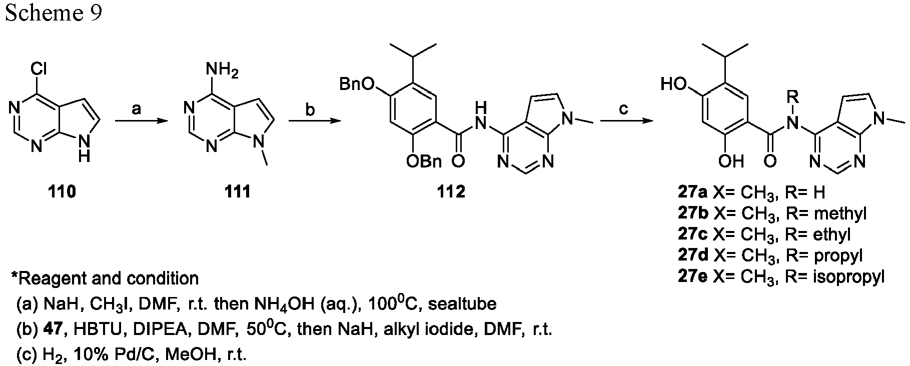 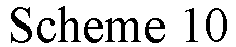 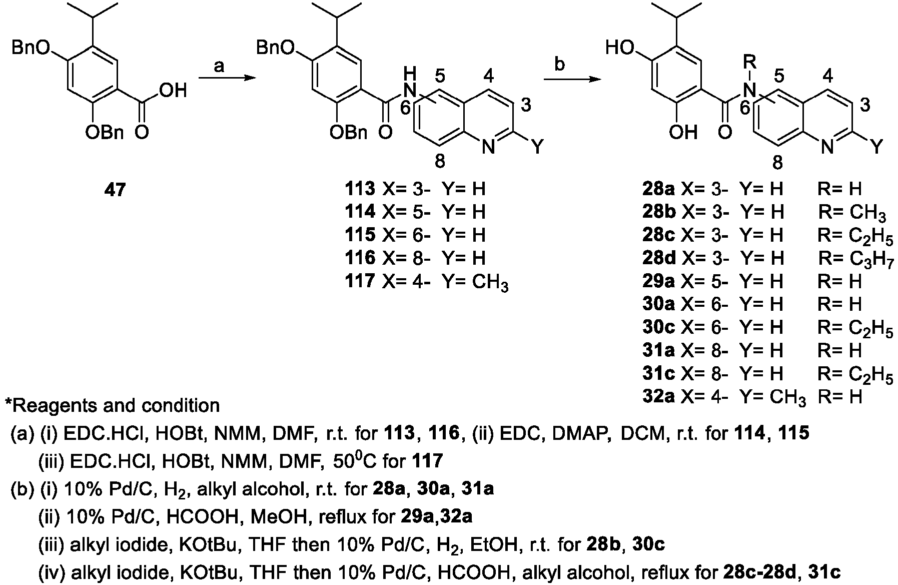 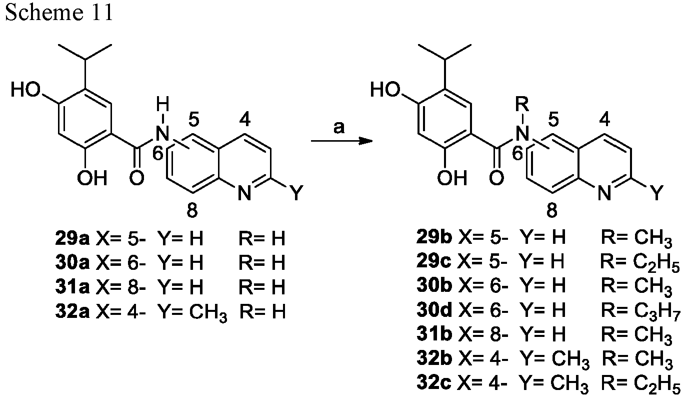
*Reagents and condition
(a) (i) TBDMSCI, DIPEA, CH₂Cl₂, r.t. (ii) KOtBu, alkyl iodide, THF, r.t. (iii) TBAF, CH₂Cl₂, r.t. 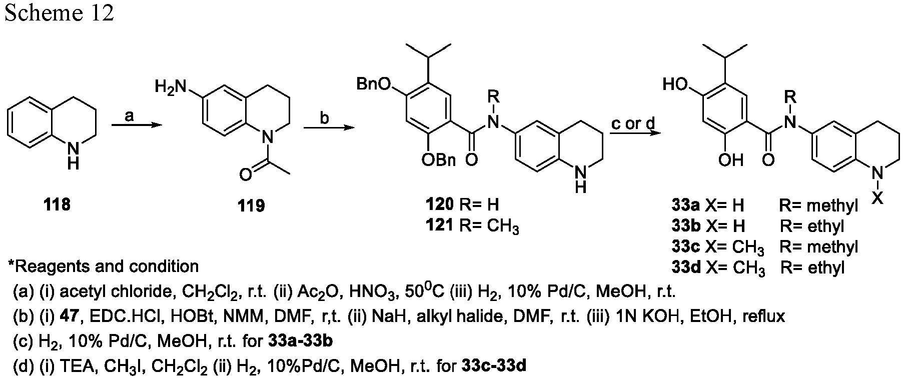 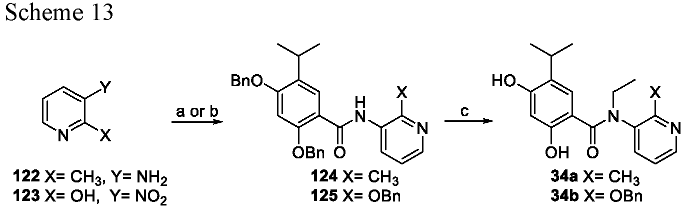
*Reagents and condition
(a) **47,** HBTU, DIPEA, DMF, 40⁰C for **124**
(b) (i) acetone, Benzyl bromide, reflux then Fe powder, NH₄Cl, IPA/H₂O, reflux (ii) **47,** HBTU, DIPEA, DMF, 40⁰C for **125**
(c) (i) KOtBu, ethyl iodide, THF, r.t. (ii) 10% Pd/C, MeOH, H₂, r.t. 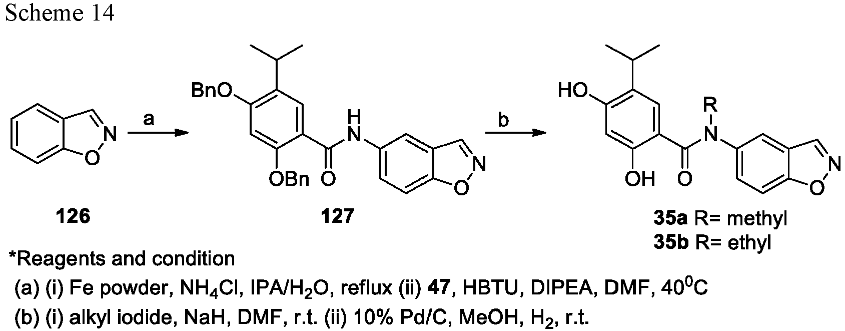 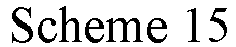 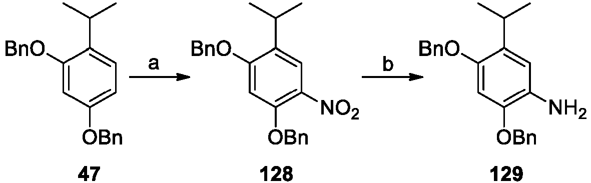
*Reagents and condition
(a) HNO₃, H₂SO₄, r.t., 54%
(b) Fe powder, NH₄Cl, IPA/H₂O, reflux, 73% 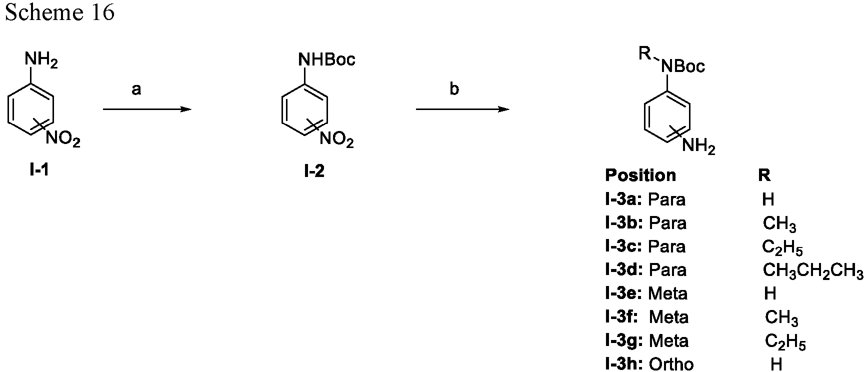
Reagents and conditions: (a) DMAP, TEA, DCM, rt; (b) (i) Different alkyl iodides, NaH, DMF, 0 °C to rt; (ii) NH₄Cl, Fe, IPA: H₂O (4:1), reflux. 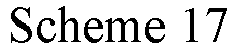 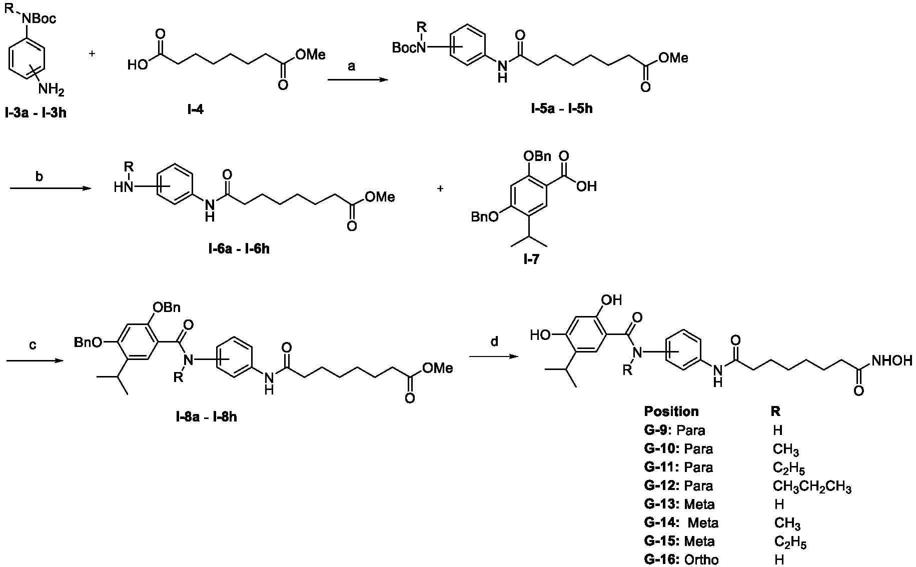 Reagents and conditions: (a) HOBt, DIPEA, DMF; (b) H2O, Dioxane, Reflux; (c) HOBt, DIPEA, DMF; (d) (i) 1M LiOH (aq), Dioxane, 40 oC; (ii) NH2OBn, EDC, HOBt, NMM, DMF, rt; (iii) Pd/C, MeOH, H2 atmosphere. 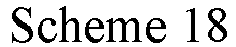 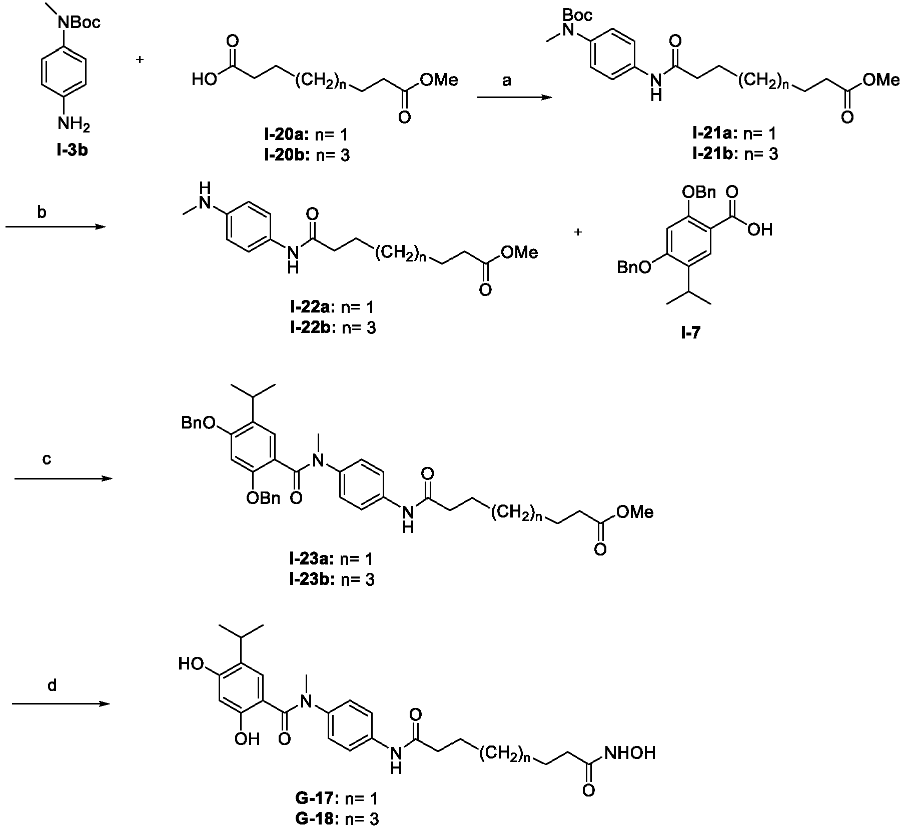 Reagents and conditions: (a) HOBt, DIPEA, DMF; (b) H₂O, Dioxane, Reflux; (c) HOBt, DIPEA, DMF; (d) (i) 1M LiOH (aq), Dioxane, 40 °C; (ii) NH₂OBn, EDC, HOBt, NMM, DMF, rt; (iii) Pd/C, MeOH, H₂ atmosphere. 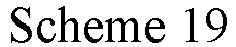 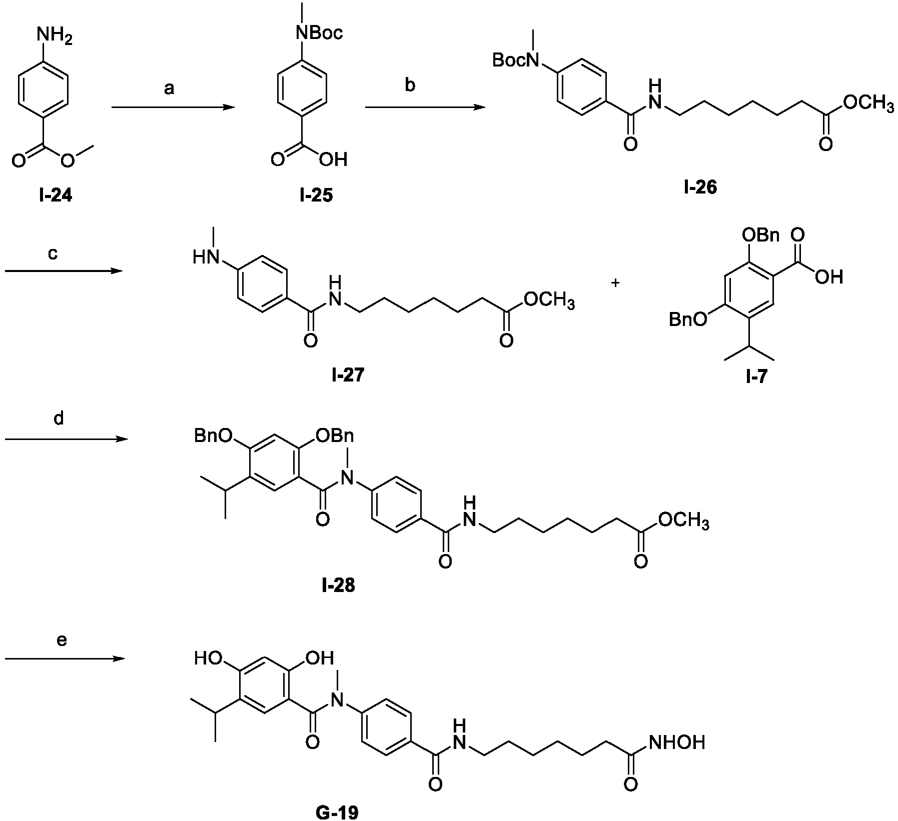 Reagents and conditions: (a) (i) DMAP, TEA, DCM; (ii) Methyl iodide, NaH, DMF, 0 °C to rt; (iii) 1M LiOH (aq), Dioxane, 40 °C; (b) HOBt, DIPEA, DMF; (c) H₂O, Dioxane, Reflux; (d) HOBt, DIPEA, DMF; (d) (i) 1M LiOH (aq), Dioxane, 40 °C; (ii) NH₂OBn, EDC, HOBt, NMM, DMF, rt; (iii) Pd/C, MeOH, H₂ atmosphere.

The compounds of formula (I) thus synthesized were purified by a method such as column chromatography, high-pressure liquid chromatography, or recrystallization.

The intermediates used in the synthesis described above were either commercially available or could be prepared by methods known in the art. The methods could also include additional steps, either before or after the steps described specifically herein, to add or remove suitable protecting groups if necessary to facilitate synthesis of the compounds. In addition, various synthetic steps could be performed in an alternate sequence or order to give the desired compounds.

Below are materials and methods used for preparing and testing the substituted aromatic compounds described above.

### Chemical materials and analysis

All chemicals and solvents were purchased from commercial suppliers and used as received. All reactions were carried out under an atmosphere of dry nitrogen. Reactions were monitored by TLC using Merck 60 F254 silica gel glass backed plates (5 × 10 cm); and zones were detected visually under ultraviolet irradiation (254 nm) or by spraying with phosphomolybdic acid reagent (Aldrich) followed by heating at 80 °C. All flash column chromatography was performed with Merck Kieselgel 60, No. 9385, 230-400 mesh ASTM silica gel as the stationary phase. Proton (¹H) nuclear magnetic resonance spectra were measured on a Varian Mercury-300 or Varian Mercury-400 spectrometer. Chemical shifts were recorded in parts per million (ppm) on the delta (δ) scale relative to the resonance of the solvent peak. The following abbreviations were used to describe coupling: s = singlet; d = doublet; t = triplet; q = quartet; quin = quintet; br = broad; and m = multiplet. LCMS data were measured on an Agilent MSD-1100 ESI-MS/MS, Agilent 1200 series LC/MSD VL, and Waters Acquity UPLC-ESI-MS/MS system.

### Cell Culture

Human cancer cells were purchased from the American Type Culture Collection (Manassas, VA, USA). These cell lines were cultured in RPMI 1640 medium or DMEM supplemented with 10% FBS (v/v) and pencillin (100 U/mL)/streptomycin (100 ug/mL)/amphotericin B (0.25 ug/mL). Cultures were maintained at 37°C in a humidified atmosphere of 5% CO2/95% air.

### Sulforhodamine B (SRB) assay

Cells were seeded in 96-well plates in medium with 5% fetal bovine serum. After 24 h, cells were fixed with 10% trichloroacetic acid to represent cell population at the time of drug addition (*T*0). After additional incubation of DMSO or drugs for 48 h, cells were fixed with 10% trichloroacetic acid and sulforhodamine B at 0.4% (w/v) in 1% acetic acid was added to stain cells. Unbound sulforhodamine B was washed out by 1% acetic acid and sulforhodamine B-bound cells were solubilized with 10 mmol/L Trizma base. The absorbance was read at a wavelength of 515 nm. Using the following absorbance measurements, such as time zero (*T*0), control growth (*C*), and cell growth in the presence of the drug *(Tx),* the percentage growth was calculated at each of the compound concentrations levels. Percentage growth inhibition was calculated as 100 - [(*Tx - T*0) /(*C - T*0)] × 100. Growth inhibition of 50% (GI50) was determined at the drug concentration that results in 50% reduction of total protein increase in control cells during the compound incubation.

### HSP90 ATP-binding assay and competition assays

To assess the inhibition of HSP90 activity within a cellular context, cells were treated with certain substituted aromatic compounds of formula (I) and the inhibition of HSP90 ATP-binding activity in cells was determined by using an ATP pull-down assay. Cells were treated with the compounds for 24 hours and then lysed in TNESV buffer (50 mM Tris, 2 mM EDTA, 100 nM NaCl, 1 mM activated sodium orthovanadate, 25 mM NaF, 1% Triton X-100 [pH 7.5]) for 30 min at 4 °C. Lysates were spun for 30 min at 12000 rpm at 4 °C. Protein (200 ug) was incubated with conditioned γ-ATP-polyacrylamide resin in incubation buffer (10 mM Tris-HCl, 50 mM KCl, 5 mM MgCl₂, 20 mM Na₂MoO₄, 0.01% Nonidet P-40) overnight at at 4 °C, rotating. The resin was then washed four times with incubation buffer. Bound proteins were isolated by boiling with SDS buffer. For the competition studies, fluorescence polarization assays were performed under the following conditions: each 96-well plate contained 5 nM fluorescent GM (geldanamycin), 30 nM HSP90 α protein , and tested inhibitor in a final volume of 100 uL. For each assay, background wells (buffer only), tracer controls (free, fluorescent GMonly), and bound GM controls (fluorescent GM in the presence of HSP90) were included on each assay plate. The fraction of tracer bound to HSP90 was correlated to the mP value and plotted against log10 values of competitor concentrations. The inhibitor concentration at which 50% of bound GM was displaced was obtained by fitting the data.

### Western blot assay

Western blot analysis was performed to detect cell apoptotic signaling activated by substituted aromatic compounds of formula (I). Cells were harvested by scraping with lysis buffer (1 mM EGTA, 1 mM EDTA, 150 mM NaCl, 1% Triton X-100, 2.5 mM sodium pyrophosphate, 1 mM PMSF, 1 mM Na3VO4, 1 ug/mL leupeptin, 1 ug/mL aprotinin, 5mM NaF in 20 mM Tris-HCl buffer, pH 7.5). Cell lysates were centrifuged at 13,000 g for 30 min. Total protein was determined and equal amounts of protein were separated by SDS-PAGE and immunoblotted with specific antibodies. Proteins were visualized by enhanced chemiluminescence (Amersham, Buckinghamshire, UK).

### EXAMPLE 1: Synthesis of compounds of formula (I)

### 1-(2,4-bis(benzyloxy)phenyl)ethanone (43)

A mixture of 1-(2,4-dihydroxyphenyl)ethanone (10g, 65.77mmol), benzyl bromide (18ml, 151.33mmol), potassium carbonate (28g, 202.60mmol) and acetone (250ml) was refluxed for overnight, the precipitant was filtered. The organic layer was collected and concentrated *in vacuo* to yield an oily product, and the oily product was added n-hexane and stirred for overnight. The residue was filtered by suction filtration to yield white product and without more purification to afford **43** (20.43g, 93.42%) as a white solid.¹H-NMR (500MHz, CDCl₃): δ 2.55 (s, 3H), 5.09 (s, 2H), 5.11 (s, 2H), 6.62 (m, 2H), 7.38 (m, 10H), 7.84 (d, *J=* 9 Hz, 1H).

### 2-(2,4-bis(benzyloxy)phenyl)propan-2-ol (44)

A mixture of **43** (20g, 60.17mmol) and THF (100ml) was added methylmagnesium bromide (3M, 33ml) at 0°C. The reaction was allowed back to room temperature and stirred for 4 h. The reaction was quenched with water at 0°C.The residue was filtered by suction filtration to yield white product and without more purification to afford **44** (16.21g, 77.30%) as a white solid.¹H-NMR (500MHz, CDCl₃): δ 1.53 (s, 3H), 1.59 (s, 3H), 3.98 (s, 1H), 5.04 (s, 2H), 5.10 (s, 2H), 6.54 (m, 1H), 6.67 (d, *J=* 2.5 Hz, 1H), 7.24 (d, 1H), 7.38 (m, 10H).

### (((4-isopropyl-1,3-phenylene)bis(oxy))bis(methylene))dibenzene (45)

A mixture of **44** (16g, 45.92mmol) and CHCl₂ (125ml) was added triethylsilane (9ml, 56.35mmol) and TFA (4.5ml, 58.77mmol) at -78°C. The reaction was allowed back to room temperature and stirred for 4 h. The reaction was quenched with 1N NaOH (aq.) and extracted with CH₂Cl₂ (30ml*3). The organic layer was collected and dried over anhydrous MgSO₄ concentrated *in vacuo* to yield colorless oil product. The residue was purified by silica gel chromatography (ethyl acetate: n-hexane = 1: 19, Rf = 0.35) to afford **45** (14.06g, 92.08%) as a white solid.¹H-NMR (500MHz, CDCl₃): δ 1.25 (d, *J=* 6.5 Hz, 6H), 3.37 (Sep, *J=* 7 Hz, 1H), 5.05 (s, 2H), 5.07 (s, 2H), 6.59 (m, 1H), 6.63 (d, *J=* 2.5 Hz, 1H), 7.16 (d, *J*= 8.5 Hz, 1H), 7.42 (m, 10H).

### 2,4-bis(benzyloxy)-5-isopropylbenzaldehyde (46)

A mixture of POCl₃ (3.3ml, 35.40mmol) and DMF (2,7ml) was stirred at 0°C for 10 min then added **45** (4g, 12.03mmol) dissolved in DMF (3ml). The reaction was heated at 80°C and stirred for 1.5 h. The reaction was quenched with 6N NaOH (aq.) and extracted with ethyl acetate (30ml*3). The organic layer was collected and dried over anhydrous MgSO₄ concentrated *in vacuo* to yield a yellow product. The residue was without more purification to afford **46** (4.01g, 92.40%) as a yellow solid.¹H-NMR (500MHz, CDCl₃): δ 1.21 (d, *J=* 7 Hz, 6H), 3.28 (Sep, *J=* 7 Hz, 1H), 5.10 (s, 2H), 5.12 (s, 2H), 6.50 (s, 1H), 7.37 (m, 10H), 7.74 (s, 1H), 10.39 (s, 1H).

### 2,4-bis(benzyloxy)-5-isopropylbenzoic acid (47)

A mixture of **46** (3g, 8.32mmol), sulfamic acid (6.5g, 66.94mmol), DMSO (2ml), THF (20ml) and water (20ml) was added NaClO₂ (6g, 66.34mmol) dissolved in water (20ml) at 0°C. The reaction was allowed back to room temperature and stirred for 2 h. The residue was extracted with ethyl acetate (40ml*3) and purified by silica gel chromatography (ethyl acetate: n-hexane = 1: 4, Rf = 0.25) to afford **47** (2.02g, 64.61%) as a yellow solid.¹H-NMR (500MHz, CDCl₃): δ 1.23 (d, *J=* 7 Hz, 6H), 2.98 (s, 1H), 3.30 (Sep, *J=* 7 Hz, 1H), 5.10 (s, 2H), 5.20 (s, 2H), 6.57 (s, 1H), 7.39 (m, 10H), 8.03 (s, 1H).

### N-benzyl-2,4-bis(benzyloxy)-5-isopropylbenzamide (48)

A mixture of **47** (2g, 5.3 1mmol), EDC.HCl (1.5g, 7.85mmol), HOBt(0.9g, 6.67mmol), NMM (1.4ml, 12.75mmol) and DMF (8ml) was stirred for 10 min then added benzylamine (0.7ml, 6.53mmol) at room temperature for overnight, the reaction was quenched with water and extracted with ethyl acetate(30ml*3).The organic layer was collected and dried over anhydrous MgSO₄ concentrated *in vacuo* to yield colorless oil product. The residue was purified by silica gel chromatography (ethyl acetate: n-hexane = 1:3, Rf= 0.6 ) to afford **48** (2.05g, 83.00%) as a white solid.¹H-NMR (500MHz, CDCl₃): δ 1.25 (d, *J=* 6.5 Hz, 6H), 3.33 (Sep, *J=* 7 Hz, 1H), 4.54 (d, *J=* 5.5 Hz, 2H), 5.03 (s, 2H), 5.10 (s, 2H), 6.54 (s, 1H), 7.34 (m, 15H), 8.16 (s, 2H).

### 2,4-bis(benzyloxy)-N-(2-fluorobenzyl)-5-isopropylbenzamide (49)

A mixture of **47** (2.52g, 6.69mmol), EDC.HCl (2g, 10.47mmol), HOBt (1.1g, 8.15mmol), NMM (2ml, 18.19mmol) and DMF (8ml) was stirred for 10 min then added 2-fluorobenzylamine (1g, 8mmol) for overnight, the reaction was quenched with water and extracted with ethyl acetate (30ml*3).The organic layer was collected and dried over anhydrous MgSO₄ concentrated *in vacuo* to yield colorless oil product. The residue was purified by silica gel chromatography (ethyl acetate: n-hexane = 1: 4, Rf= 0.3) to afford **49** (2.54g, 78.40%) as a white solid.¹H-NMR (500MHz, CDCl₃): δ 1.24 (d, *J=* 7 Hz, 6H), 3.32 (Sep, *J=* 7 Hz, 1H), 4.59 (d, *J=* 6 Hz, 2H), 5.07 (s, 2H), 5.09 (s, 2H), 6.54 (s, 1H), 7.0 (m, 2H), 7.20 (m, 11H), 8.13 (s, 1H), 8.23 (s, 1H).

### 2,4-bis(benzyloxy)-N-(3-fluorobenzyl)-5-isopropylbenzamide (50)

A mixture of **47** (2.7g, 7.17mmol), EDC.HCl (2.1g, 10.99mmol), HOBt (1.2g, 8.89mmol), NMM (2ml, 18.19mmol) and DMF (8ml) was stirred for 10 min then added 3-fluorobenzylamine (1.07g, 8.57mmol) for overnight, the reaction was quenched with water and extracted with ethyl acetate (30ml*3). The organic layer was collected and dried over anhydrous MgSO₄ concentrated *in vacuo* to yield colorless oil product. The residue was purified by silica gel chromatography (ethyl acetate: n-hexane = 1: 4, Rf= 0.5) to afford **50** (2.89g, 83.29%) as a white solid.¹H-NMR (500MHz, CDCl₃): δ 1.25 (d, *J=* 7.5 Hz, 6H), 3.34 (Sep, *J=* 7 Hz, 1H), 4.51 (d, *J=* 5.5 Hz, 2H), 5.05 (s, 2H), 5.11 (s, 2H), 6.57 (s, 1H), 6.90 (m, 2H), 6.95 (d, *J=* 8 Hz, 1H), 7.18 (m, 1H), 7.31 (m, 10H), 8.17 (s, 1H), 8.20 (t, *J*= 5.5 Hz, 1H).

### 2,4-bis(benzyloxy)-N-(3-fluorobenzyl)-5-isopropylbenzamide (51)

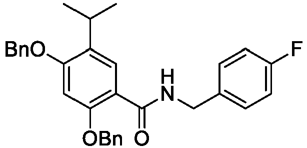

A mixture of **47** (2.6g, 6.90mmol), EDC.HCl (2g, 10.47mmol), HOBt (1.2g, 8.89mmol), NMM (2ml, 18.19mmol) and DMF (8ml) was stirred for 10 min then added 4-fluorobenzylamine (1g, 8mmol) for overnight. The reaction was quenched with water and extracted with ethyl acetate (30ml*3). The organic layer was collected and dried over anhydrous MgSO₄ concentrated *in vacuo* to yield colorless oil product. The residue was purified by silica gel chromatography (ethyl acetate: n-hexane = 1: 2, Rf= 0.5) to afford **51** (2.76g, 82.63%) as a white solid.¹H-NMR (500MHz, CDCl₃): δ 1.25 (d, *J=* 6.5 Hz, 6H), 3.33 (m, 1H), 4.48 (d, *J=* 5.5 Hz, 2H), 5.02 (s, 2H), 5.10 (s, 2H), 6.55 (s, 1H), 6.89 (m, 2H), 7.10 (m, 2H), 7.34 (m, 11H), 8.14 (s, 1H).

### N-benzyl-2,4-dihydroxy-5-isopropylbenzamide (1a)

A mixture of **48** (0.42g, 0.90mmol), 10% palladium on activated carbon (0.04g, 0.04mmol) and MeOH (8ml) was under the hydrogen gas. The reaction was stirred at room temperature for 2h. The 10% palladium on activated carbon was removed by methyl alcohol wash through celite packing. The organic layer was concentrated *in vacuo* and purified by silica gel chromatography (ethyl acetate: n-hexane = 1:2, Rf= 0.4) to afford **1a** (0.23g, 88.46%) as a white solid. m.p. 142.5- 144.8. ¹H-NMR (500MHz, MeOD): δ 1.19 (d, *J*=7 Hz, 6H), 3.17 (Sep, *J=* 7 Hz, 1H), 4.52 (s, 2H), 6.31 (s, 1H), 7.19 (t, *J*=7 Hz, 1H), 7.27 (m, 4H), 7.58 (s, 1H).

### N-benzyl-2,4-dihydroxy-5-isopropyl-N-methylbenzamide (1b)

A mixture of **48** (0.38g, 0.82mmol), NaH (0.04g, 1mmole) and DMF (4ml) was added methyl iodide(0.1ml, 1.61mmol) at room temperature for 2h. The reaction was quenched with water and extracted with ethyl acetate (30ml*3). The organic layer was collected and dried over anhydrous MgSO₄ concentrated *in vacuo* and purified by silica gel chromatography (ethyl acetate: n-hexane = 1: 3, Rf= 0.5) to get the oily product, then the oily product was dissolved in MeOH (8ml) and added 10% palladium on activated carbon (0.04g, 0.04mmol) under the hydrogen gas. The reaction was stirred at room temperature for 2h. The 10% palladium on activated carbon was removed by methyl alcohol wash through celite packing. The organic layer was concentrated *in vacuo* and purified by silica gel chromatography (ethyl acetate: n-hexane = 1: 2, Rf= 0.4) to afford **1b** (0.17g, 68%) as a white solid. m.p. 133.7- 135.4. ¹H-NMR (500MHz, MeOD): δ 1.10 (d, *J=* 7 Hz, 6H), 2.93 (s, 3H), 3.13 (Sep, *J*= 7Hz, 1H), 4.63 (s, 2H), 6.34 (s, 1H), 6.99 (s, 1H), 7.28 (m, 5H).

### N-benzyl-N-ethyl-2,4-dihydroxy-5-isopropylbenzamide (1c)

The title compound was prepared using a mixture of **48** (0.35g, 0.75mmol), NaH (0.04g, 1mmole ) and DMF (4ml) and similar process as mentioned herein afford **1c** (0.17g, 70.83%)as a white solid. m.p. 117.9- 119.8. ¹H-NMR (500MHz, MeOD): δ 1.09 (m, 9H), 3.13 (Sep, *J*= 7Hz, 1H), 3.36 (m, 2H), 4.65 (s, 2H), 6.34 (s, 1H), 6.94 (s, 1H), 7.27 (m, 5H).

### N-benzyl-2,4-dihydroxy-5-isopropyl-N-propylbenzamide (1d)

The title compound was prepared using a mixture of **48** (0.40g, 0.86mmol), NaH (0.04g, 1mmole) and DMF (4ml) and similar process as mentioned herein to afford **1d** (0.19g, 67.86%) as a white solid. m.p. 158.6- 160.6. ¹H-NMR (500MHz, MeOD): δ 0.80 (s, 3H), 1.09 (d, *J=* 7 Hz, 6H), 1.57 (6, *J*=7 Hz, 2H), 3.14 (Sep, *J=* 7 Hz, 1H), 3.26 (m, 2H), 4.64 (s, 2H), 6.35 (s, 1H), 6.94 (s, 1H), 7.27 (m, 5H).

### N-(2-fluorobenzyl)-2,4-dihydroxy-5-isopropylbenzamide (2a)

The title compound was prepared using a mixture of **49** (0.32g, 0.66mmol), 10% palladium on activated carbon (0.04g, 0.04mmol) and MeOH(8ml) and similar process as mentioned herein to afford **2a** (0.17g, 85.0%) as a colorless oil. ¹H-NMR (500MHz, MeOD): δ 1.19 (d, *J=* 7 Hz, 6H), 3.17 (Sep *J=* 7 Hz, 1H), 4.59 (s, 2H), 6.30 (s, 1H), 7.06 (m, 2H), 7.27 (m, 1H), 7.35 (m, 1H), 7.60 (s, H).

### N-(2-fluorobenzyl)-2,4-dihydroxy-5-isopropyl-N-methylbenzamide (2b)

The title compound was prepared using a mixture of **49** (0.38g, 0.79mmol), NaH (0.04g, 1mmole) and DMF (4ml) and similar process as mentioned herein to afford **2b** (0.16g, 64.0%) as a white solid. m.p. 92.3-94.3. ¹H-NMR (500MHz, MeOD): δ 1.10 (d, *J=* 7 Hz, 6H), 2.97 (s, 3H), 3.13 (Sep, *J=* 7 Hz, 1H), 4.70 (s, 2H), 6.34 (s, 1H), 6.97 (s, 1H), 7.07 (t, *J=* 9 Hz, 1H), 7.15, (t, *J=* 7 Hz, 1H), 7.28 (m, 1H), 7.36 (s, 1H).

### N-ethyl-N-(2-fluorobenzyl)-2,4-dihydroxy-5-isopropylbenzamide (2c)

The title compound was prepared using a mixture of **49** (0.30g, 0.62mmol), NaH (0.04g, 1mmole) and DMF (4ml) and similar process as mentioned herein to afford 2c (0.14g, 66.67%) as a white solid. m.p. 113.2- 114.6. ¹H-NMR (500MHz, MeOD): δ 1.1 (m, 9H), 3.13 (Sep, *J=* 7 Hz, 1H), 3.38 (d, *J*=7 Hz, 2H), 4.70 (s, 2H), 6.36 (s, 1H), 6.93 (s, 1H), 7.04 (t, *J=* 9Hz, 1H), 7.12 (t, *J=* 8 Hz, 1H), 7.26 (m, 1H), 7.39 (s, 1H).

### N-(2-fluorobenzyl)-2,4-dihydroxy-5-isopropyl-N-propylbenzamide (2d)

The title compound was prepared using a mixture of **49** (0.39g, 0.81mmol), NaH (0.04g, 1mmole) and DMF (4ml) and similar process as mentioned herein to afford **2d** (0.17g, 60.71%) as a white solid. m.p. 126.9-127.8. ¹H-NMR (500MHz, MeOD): δ 0.81 (s, 3H), 1.10 (d, *J=* 6.5 Hz, 6H), 1.58 (m, 2H), 3.14 (hept, *J=* 7 Hz, 1H), 3.30 (s, 2H), 4.69 (s, 2H), 6.34 (s, 1H), 6.92 (s, 1H), 7.05 (t, *J=* 9 Hz, 1H), 7.13 (t, *J=* 7 Hz, 1H), 7.27 (m, 1H), 7.38 (s, 1H).

### N-(3-fluorobenzyl)-2,4-dihydroxy-5-isopropylbenzamide (3a)

The title compound was prepared using **50** and similar process as mentioned herein to afford **3a** (0.18g, 94.74%) as acolorless oil.¹H-NMR (500MHz, MeOD): δ 1.20 (d, *J=* 6.5 Hz, 6H), 3.18 (Sep, *J=* 7 Hz, 1H), 4.54 (s, 2H), 6.28 (s, 1H), 6.95 (m, 1H), 7.05 (m, 1H), 7.14 (d, *J=* 7.5 Hz, 1H), 7.31 (m, 1H), 7.56 (s, 1H).

### N-(3-fluorobenzyl)-2,4-dihydroxy-5-isopropyl-N-methylbenzamide (3b)

The title compound was prepared using a mixture of **50** (0.39g, 0.81mmol ), NaH (0.04g, 1mmole) and DMF (4ml) and similar process as mentioned herein to afford **3b** (1.8g, 69.23%) as a pink solid. m.p. 149.8- 150.7.¹H-NMR (500MHz, MeOD): δ 1.11 (d, *J=* 7 Hz, 6H), 2.95 (s, 3H), 3.13 (t, *J=* 7 Hz, 1H), 4.66 (s, 2H), 6.33 (s, 1H), 6.99 (m, 2H), 7.10 (m, 2H), 7.34 (m, 1H).

### N-ethyl-N-(3-fluorobenzyl)-2,4-dihydroxy-5-isopropylbenzamide (3c)

The title compound was prepared using a mixture of **50** (0.33g, 0.68mmol), NaH (0.04g, 1mmole) and DMF (4ml) and similar process as mentioned herein to afford **3c** (0.16g, 69.57%)as a white solid. m.p. 118.1- 118.9. ¹H-NMR (500MHz, CDCl₃): δ 0.94 (d, *J=* 6.5 Hz, 6H), 1.27 (t, *J=* 7 Hz, 3H), 2.38 (s, 1H), 3.04 (hept, *J=* 7 Hz, 1H), 3.49 (q, *J=* 7 Hz, 2H), 4.71 (s, 2H), 6.38 (s, 1H), 7.00 (m, 5H), 7.34 (m, 1H), 10.16 (s, 1H).

### N-(3-fluorobenzyl)-2,4-dihydroxy-5-isopropyl-N-propylbenzamide (3d)

The title compound was prepared using a mixture of **50** (0.38g, 0.79mmol), NaH (0.04g, 1mmole) and DMF (4ml) and similar process as mentioned herein to afford 3d (0.17g, 62.96%) as a colorless oil. ¹H-NMR (500MHz, CDCl₃): δ 0.91 (t, *J=* 7.5 Hz, 3H), 0.97 (d, *J=* 7 Hz, 6H), 1.75 (m, 2H), 3.03 (qui, *J*= 7 Hz, 1H), 3.38 (m, 2H), 4.73 (s, 2H), 5.38 (s, 1H), 6.37 (s, 1H), 7.01 (m, 2H), 7.08 (m, 2H), 7.37 (m, 1H), 10.35 (s, 1H).

### N-(4-fluorobenzyl)-2,4-dihydroxy-5-isopropylbenzamide (4a)

The title compound was prepared using a mixture of **51** (0.3g, 0.62mmol), 10% palladium on activated carbon (0.04g, 0.04mmol) and MeOH (7ml) and similar process as mentioned herein to afford **4a** (0.17g, 89.47%) as a colorless oil. ¹H-NMR (500MHz, MeOD): δ 1.18 (d, *J=* 7 Hz, 6H), 3.16 (Sep, *J=* 7 Hz, 1H), 4.49 (s, 2H), 6.29 (s, 1H), 6.99 (t, *J=* 9 Hz, 2H), 7.31 (m, 2H), 7.56 (s, 1H).

### N-(4-fluorobenzyl)-2,4-dihydroxy-5-isopropyl-N-methylbenzamide (4b)

The title compound was prepared using a mixture of **51** (0.33g, 0.68mmol), NaH (0.04g, 1mmol) and DMF (4ml) and similar process as mentioned herein to afford **4b** (0.15g, 68.18%) as a colorless oil. ¹H-NMR (500MHz, MeOD): δ 1.12 (d, *J=* 7 Hz, 6H), 2.92 (s, 3H), 3.14 (hept, *J=* 7 Hz, 1H), 4.62 (s, 2H), 6.33 (s, 1H), 6.97 (s, 1H), 7.05 (t, *J=* 9 Hz, 2H), 7.31 (m, 2H).

### N-ethyl-N-(4-fluorobenzyl)-2,4-dihydroxy-5-isopropylbenzamide (4c)

The title compound was prepared using a mixture of **51** (0.37g, 0.77mmol), NaH (0.04g, 1mmol) and DMF (4ml) and similar process as mentioned herein to afford **4c** (0.17g, 65.38%) as a colorless oil.¹H-NMR (500MHz, MeOD): δ 1.09 (m, 9H), 3.14 (qui, *J*= 7 Hz, 1H), 3.25 (m, 2H), 4.63 (s, 2H), 6.35 (s, 1H), 6.93 (s, 1H), 7.02 (m, 2H), 7.31 (m, 2H).

### N-(4-fluorobenzyl)-2,4-dihydroxy-5-isopropyl-N-propylbenzamide (4d)

The title compound was prepared using a mixture of **51** (0.40g, 0.83mmol), NaH (0.04g, 1mmol) and DMF (4ml) and similar process as mentioned herein to afford **4d** (0.18g, 62.07%) as a white solid. m.p.103.2-103.9. ¹H-NMR (500MHz, MeOD): δ 0.79 (s, 3H), 1.10 (d, *J=* 7 Hz, 6H), 1.56 (6, *J=* 7 Hz, 2H), 3.14 (Sep, *J=* 7 Hz, 1H), 3.26 (m, 2H), 4.62 (s, 2H), 6.35 (s, 1H), 6.92 (s, 1H), 7.03 (t, *J=* 9 Hz, 2H), 7.30 (s, 2H).

### N-benzylpropan-2-amine (55)

The title compound was prepared using a mixture of benzylamine (3g, 28.00mmol), acetone (2.3ml, 31.32mmol), MeOH (15ml) and acetic acid (cat.) and similar process as mentioned herein to afford 55 (1.8g, 43.06%) as a colorless oil. ¹H-NMR (500MHz, CDCl₃): δ 1.10 (d, *J=* 6.5 Hz, 6H), 2.92 (Sep, *J=* 6.5 Hz, 1H), 3.75 (s, 2H), 7.22 (m, 5H).

### N-(3-fluorobenzyl)propan-2-amine (56)

The title compound was prepared using a mixture of 3-fluorobenzylamine (2g, 11.96mmol), acetone (1ml, 13.62mmol), MeOH (14ml) and acetic acid (cat.) and similar process as mentioned herein to afford **56** (0.87g, 43.50%) as a oily product. ¹H-NMR (500MHz, CDCl₃):δ 1.08 (d, *J=* 6.5 Hz, 6H), 2.83 (Sep, *J=* 6.5 Hz, 1H), 3.76 (s, 2H), 6.90 (m, 1H), 7.04 (m, 2H), 7.24 (m, 1H).

### N-(4-fluorobenzyl)propan-2-amine (57)

The titled compound was prepared using a mixture of 4-fluorobenylamine (2g, 11.96mmol), acetone (1ml, 13.62mmol), MeOH (15ml) and acetic acid (cat.) and similar process as mentioned herein to afford 57 (0.81g, 40.50%) as a oily product.¹H-NMR (500MHz, CDCl₃):δ 1.08 (d, *J=* 6.5 Hz, 6H), 2.83 (Sep, *J=* 6.5), 3.72 (s, 2H) 6.98 (m, 2H), 7.26 (m, 2H).

### N-benzyl-2,4-dihydroxy-N,5-diisopropylbenzamide (1e)

The title compound was prepared using a mixture of **47** (0.6g, 1.59mmol), EDC.HCl (0.45g, 2.36mmol), HOBt (0.3g, 2.22mmol), NMM (0.5ml, 4.5mmol) and DMF (3ml) and similar process as mentioned herein to afford **1e** (0.37g, 71.15%) as a white solid. m.p.176.1- 178.0. ¹H-NMR (500MHz, MeOD): δ 1.12 (m, 12H), 3.14 (qui, *J*= 7 Hz, 1H), 4.26 (m, 1H), 4.64 (s, 2H), 6.34 (s, 1H), 6.92 (s, 1H), 7.18 (t, *J=* 7 Hz, 1H), 7.27 (m, 2H), 7.34 (d, *J*=7,5 Hz, 2H).

### N-(3-fluorobenzyl)-2,4-dihydroxy-N,5-diisopropylbenzamide (3e)

The title compound was prepared using a mixture of **47** (0.5g, 1.33mmol), EDC.HCl (0,4g, 2.09mmol), HOBt (0.25g, 1.85mmol), NMM (0.5ml, 4.5mmol) and DMF (3ml) and similar process as mentioned herein to afford **3e** (0.32g, 73.91%) as a white solid. m.p. 164.3-165.1. ¹H-NMR (500MHz, MeOD): δ 1.14 (d, *J=* 7 Hz, 12H), 3.16 (Sep, *J=* 7 Hz, 1H), 4.25 (s, 1H), 4.66 (s, 2H), 6.35 (s, 1H), 6.91 (m, 2H), 7.17 (m, 2H), 7.29 (m, 1H).

### N-(4-fluorobenzyl)-2,4-dihydroxy-N,5-diisopropylbenzamide (4e)

The title compound was prepared using a mixture of **47** (0.5g, 1.33mmol), EDC.HCl (0,4g, 2.09mmol), HOBt (0.25g, 1.85mmol), NMM (0.5ml, 4.5mmol) and DMF (3ml) and similar process as mentioned herein to afford **4e** (0.31g, 67.39%) as a white solid. m.p. 195.9-197.1. ¹H-NMR (500MHz, MeOD): δ 1.12 (d, *J=* 7 Hz, 12H), 3.14 (Sep, *J=* 7 Hz, 1H), 4.24 (s, 1H), 4.61 (s, 2H), 6.34 (s, 1H), 6.90 (s, 1H), 6.99 (m, 2H), 7.36 (m, 2H).

### 2,4-bis(benzyloxy)-5-isopropyl-N-phenylbenzamide (58)

The title compound was prepared using a mixture of **47** (2.8g, 7.44mmol), EDC.HCl (2.3g, 12.04mmol), HOBt (1.5g, 11.11mmol), NMM (2ml, 18.19mmol) and DMF (7ml) and similar process as mentioned herein to afford **58** (2.71g, 80.65%) as acolorless oil product. ¹H-NMR (500MHz, CDCl₃):δ 1.27 (d, *J=* 6.5 Hz, 6H), 3.35 (qui, *J*= 7 Hz, 1H), 5.15 (s, 2H), 5.16 (s, 2H), 6.62 (s, 1H), 7.15 (t, *J=* 7.5 Hz, 1H), 7.39 (m, 14H), 8.20 (s, 1H), 9.85 (s, 1H).

### 2,4-bis(benzyloxy)-N-(2-fluorophenyl)-5-isopropylbenzamide (59)

The title compound was prepared using a mixture of **47** (2.6g, 6.91mmol), EDC.HCl (2g, 10.47mmol), HOBt (1.3g, 9.63mmol), NMM (2ml, 18.19nnol) and DMF and similar process as mentioned herein to afford **59** (2.40g, 74.07%) as a white solid. ¹H-NMR (500MHz, CDCl₃): δ 1.25 (d, *J=* 7 Hz, 6H), 3.32 (qui, *J*= 7 Hz, 1H), 5.07 (s, 2H), 5.22 (s, 2H), 6.56 (s, 1H), 7.28 (m, 13H), 8.17 (s, 1H), 8.55 (t, *J=* 8 Hz, 1H), 10.19 (s, 1H).

### 2,4-bis(benzyloxy)-N-(3-fluorophenyl)-5-isopropylbenzamide (60)

The title compound was prepared using a mixture of **47** (2g, 5.31mmol), EDC.HCl (1.5g, 7.85mmol), HOBt (1g, 7.41mmol), NMM (1.5ml, 13.64mmol) and DMF (6ml) and similar process as mentioned herein to afford **60** (2.03g, 81.53%) as a white solid.¹H-NMR (500MHz, CDCl₃): δ 1.26 (d, *J=* 6.5 Hz, 6H), 3.35 (qui, *J*= 7 Hz, 1H), 5.14 (s, 2H), 5.17 (s, 2H), 6.62 (s, 1H), 6.68 (m, 1H), 6.78 (m, 1H), 7.08 (m, H), 7.21 (m, 1H), 7.47 (m, 10H), 8.18 (s, 1H), 9.93 (s, 1H).

### 2,4-bis(benzyloxy)-N-(4-fluorophenyl)-5-isopropylbenzamide (61)

The title compound was prepared using a mixture of **47** (2g, 5.31mmol), EDC.HCl (1.5g, 7.85mmol), HOBt (0.9g, 6.67mmol), NMM (1.5ml, 13.64mmol) and DMF (8ml) and similar process as mentioned herein to afford **61** (1.89g, 75.90%) as a white solid. ¹H-NMR (500MHz, CDCl₃): δ 1.26 (d, *J=* 7 Hz, 6H), 3.35 (Sep, *J=* 7 Hz, 1H), 5.14 (s, 2H), 5.16 (s, 2H), 6.62 (s, 1H), 6.88 (t, *J=* 8.5 Hz, 2H), 7.20 (m, 2H), 7.44 (m, 10H), 8.18 (s, 1H), 9.84 (s, 1H).

### 2,4-bis(benzyloxy)-5-isopropyl-N-(3,4,5-trimethoxyphenyl)benzamide (62)

The title compound was prepared using a mixture of **47** (3g, 7.97mmol), EDC.HCl (2.4g, 12.57mmol), HOBt (1.4g, 10.37mmol), NMM (2.5ml, 22.74mmol) and DMF (10ml) and similar process as mentioned herein to afford **62** (3.09g, 71.53%) as a white solid. ¹H-NMR (500MHz, CDCl₃): δ 1.27 (d, *J=* 6.5 Hz, 6H), 3.37 (qui, *J*= 7 Hz, 1H) 3.69 (s, 6H), 3.77 (s, 3H), 5.13 (s, 2H), 5.19 (s, 2H), 6.63 (s, 2H), 6.66 (s, 1H), 7.43 (m, 10H), 8.21 (s, 1H), 9.82 (s, 1H).

### 2,4-bis(benzyloxy)-5-isopropyl-N-(4-morpholinophenyl)benzamide (63)

The title compound was prepared using a mixture of **47** (2.2g, 5.84mmol), EDC.HCl (1.7g, 8.9mmol), HOBt (1g, 7.41mmol), NMM (1.5ml, 13.64mmol) and DMF (7ml) and similar process as mentioned herein to afford 63 (2.52g, 80.51%) as a white solid. ¹H-NMR (500MHz, CDCl₃): δ 1.26 (d, *J=* 7 Hz, 6H), 3.08 (t, *J=* 5 Hz, 4H), 3.35 (qui, *J*= 7 Hz, 1H), 3.84 (t, *J=* 5 Hz, 4H), 5.14 (s, 2H), 5.15 (s, 2H), 6.61 (s, 1H), 6.77 (d, *J=* 9 Hz, 2H), 7.20 (d, *J=* 9 Hz, 2H), 7.45 (m, 10H), 8.20 (s, 1H), 9,74 (s, 1H).

### 2,4-dihydroxy-5-isopropyl-N-phenylbenzamide (5a)

The title compound was prepared using a mixture of **58** (0.3g, 0.66mmol), 10% palladium on activated carbon (0.04g, 0.04mmol) and MeOH (7ml) and similar process as mentioned herein to afford **5a** (0.16g, 88.89%) as a white solid. 197.1- 198.9. ¹H-NMR (500MHz, MeOD): δ 1.24 (d, *J=* 7 Hz, 6H), 3.20 (Sep, *J=* 7 Hz, 1H), 6.34 (s, 1H), 7.13 (m, 1H), 7.33 (m, 2H), 7.60 (m, 2H), 7.75 (s, 1H).

### 2,4-dihydroxy-5-isopropyl-N-methyl-N-phenylbenzamide (5b)

The title compound was prepared using a mixture of **58** (0.33g, 0.73mmol), NaH (0.04g, 1mmole) and DMF (3ml) and similar process as mentioned herein to afford **5b** (0.15g, 71.42%) as a white solid. m.p. 126.8- 128.0. ¹H-NMR (500MHz, MeOD): δ 0.76 (d, *J=* 7 Hz, 6H), 2.89 (Sep, *J=* 7 Hz, 1H), 3.40 (s, 1H), 6.19 (s, 1H), 6.58 (s, 1H) 7.16 (m, 2H), 7.21 (m, 1H), 7.32 (m, 2H).

### N-ethyl-2,4-dihydroxy-5-isopropyl-N-phenylbenzamide (5c)

The title compound was prepared using a mixture of **58** (0.36g, 0.80mmol), NaH (0.04g, 1mmole) and DMF (3ml) and similar process as mentioned herein to afford **5c** (0.17g, 70.83%) as a white solid. m.p. 143.2- 144.3. ¹H-NMR (500MHz, MeOD): δ 0.75 (d, *J=* 7 Hz, 6H), 1.17 (t, *J=* 7 Hz, 3H), 2.88 (Sep, *J=* 7 Hz, 1H), 3.90 (q, *J=* 7 Hz, 2H), 6.19 (s, 1H), 6.57 (s, 1H), 7.14 (m, 2H), 7.22 (m, 1H), 7.32 (m, 2H).

### 2,4-dihydroxy-5-isopropyl-N-phenyl-N-propylbenzamide (5d)

The title compound was prepared using a mixture of **58** (0.34g, 0.75mmol), NaH (0.04g, 1mmole) and DMF (3ml) and similar process as mentioned herein to afford **5d** (0.15g, 62.50%) as a white solid. m.p. 119.8-120.6. ¹H-NMR (500MHz, MeOD): δ 0.76 (d, *J=* 6.5 Hz, 6H), 0,91 (t, *J=* 7.5 Hz, 3H), 1.62 (m, 2H), 2.88 (hept, *J=* 7 Hz, 1H), 3.82 (m, 2H), 6.19 (s, 1H), 6.57 (s, 1H), 7.15 (m, 2H), 7.22 (m, 1H), 7.31 (m, 2H).

### 2,4-dihydroxy-N,5-diisopropyl-N-phenylbenzamide (5e)

The title compound was prepared using a mixture of **58** (0.34g, 0.75mmol), NaH (0.04g, 1mmole) and DMF (3ml) and similar process as mentioned herein to afford **5e** (0.14g, 58.33%) as a white solid. m.p. 135.8-137.1. ¹H-NMR (500MHz, MeOD): δ 0.82 (d, *J=* 7 Hz, 6H), 1.17 (d, *J=* 7 Hz, 6H), 2.90 (Sep, *J=* 7 Hz, 1H), 4.95 (Sep, *J=* 7 Hz, 1H), 6.16 (s, 1H), 6.59 (s, 1H), 7.13 (m, 2H), 7.25 (m, 1H), 7.31 (m, 2H).

### N-(2-fluorophenyl)-2,4-dihydroxy-5-isopropylbenzamide (6a)

The title compound was prepared using a mixture of **59** (0.34g, 0.72mmol), 10% Palladium on activated carbon (0.04g, 0.04mmol) and MeOH(7ml) and similar process as mentioned herein to afford **66a** (0.18g, 85.71%) as a yellow solid. m.p. 67.3-68.2¹H-NMR (500MHz, MeOD):δ 1.22 (d, *J=* 7 Hz, 6H), 3.20 (Sex, *J*= 7 Hz, 1H), 6.39 (s, 1H), 7.13 (m, 3H), 7.81 (s, 1H), 8.14 (m, 1H).

### N-(2-fluorophenyl)-2,4-dihydroxy-5-isopropyl-N-methylbenzamide (6b)

The title compound was prepared using a mixture of **59** (0.31g, 0.66mmol), NaH (0.04g, 1mmole) and DMF (3ml) and similar process as mentioned herein to afford **6b** (0.14g, 70.0%) as a white solid. m.p. 146.7-147.6. ¹H-NMR (500MHz, MeOD): δ 0.81 (d, *J=* 7 Hz, 6H), 2.92 (Sep, *J=* 7 Hz, 1H), 3.34 (s, 3H), 6.19 (s, 1H), 6.66 (s, 1H), 7.11 (m, 2H), 7.25 (m, 2H).

### N-ethyl-N-(2-fluorophenyl)-2,4-dihydroxy-5-isopropylbenzamide (6c)

The title compound was prepared using a mixture of **59** (0.35g, 0.75), NaH (0.04g, 1mmole) and DMF (3ml) and similar process as mentioned herein to afford **6c** (0.16g, 66.67%)as a white solid. m.p. 153.1-154.2. ¹H-NMR (500MHz, MeOD): δ 0.80 (d, *J=* 7 Hz, 6H), 1.16 (t, *J=* 7 Hz, 3H), 2.91 (Sep, *J=* 7 Hz, 1H), 3.85 (q, *J=* 7 Hz, 2H), 6.17 (s, 1H), 6.66 (s, 1H), 7,13 (m, 2H), 7.28 (m, 2H).

### N-(2-fluorophenyl)-2,4-dihydroxy-5-isopropyl-N-propylbenzamide (6d)

The title compound was prepared using a mixture of **59** (0.32g, 0.68mmol), NaH (0.04g, 1mmole) and DMF (3ml) was added 1-iodopropane (0.1ml, 1.03mmol) at room temperature for 2h. The reaction was quenched with water and extracted with ethyl acetate (30ml*3). The organic layer was collected and dried over anhydrous MgSO₄ concentrated *in vacuo* and purified by silica gel chromatography (ethyl acetate: n-hexane = 1: 2, Rf= 0.4) to get the oily product, then the oily product was dissolved in MeOH (6ml) and added 10% palladium on activated carbon (0.04g, 0.04mmol) under the hydrogen gas. The reaction was stirred at room temperature for 2h. The 10% palladium on activated carbon was removed by methyl alcohol wash through celite packing. The organic layer was concentrated *in vacuo*and purified by silica gel chromatography (ethyl acetate: n-hexane = 1: 2, Rf= 0.2) to afford **6d** (0.14g, 60.87%) as a white solid. m.p. 132.1-132.4. ¹H-NMR (500MHz, MeOD): δ 0.82 (d, *J=* 7 Hz, 6H), 0.92 (t, *J=* 7 Hz, 3H), 1.60 (Sex, *J*= 7.5 Hz, 2H), 2.91 (Sep, *J=* 7, 1H), 3.76 (m, 2H), 6.17 (s, 1H), 6.66 (s, 1H), 7.09 (m, 1H), 7.16 (m, 1H), 7.28 (m, 2H).

### N-(2-fluorophenyl)-2,4-dihydroxy-N,5-diisopropylbenzamide (6e)

The title compound was prepared using a mixture of **59** (0.38g, 0.81mmol), NaH (0.04g, 1mmole) and DMF (3ml) and similar process as mentioned herein to afford **6e** (0.15g, 55.56%) as a pink solid. m.p. 125.1-126.7. ¹H-NMR (500MHz, CDCl₃):δ 0.78 (d, *J=* 6.5 Hz, 6H), 1.21 (s, 6H), 2.84 (Sep, *J=* 7 Hz, 1H), 4.97 (qui, *J*= 7 Hz, 1H), 5.98 (s, 1H), 6.28 (s, 1H), 6.61 (s, 1H). 7.05 (m, 1H), 7.14 (m, 1H), 7.21 (m, 1H), 7.27 (m, 1H).

### N-(3-fluorophenyl)-2,4-dihydroxy-5-isopropylbenzamide (7a)

The title compound was prepared using a mixture of **60** (0.34g, 0.72mmol), 10% palladium on activated carbon (0.04g, 0.04mmol) and MeOH (7ml) and similar process as mentioned herein to afford **7a** (0.18g, 85.71%) as a colorless oil. ¹H-NMR (500MHz, CDCl₃): δ 1.21 (d, *J=* 7 Hz, 6H), 3.14 (Sep, *J=* 7 Hz, 1H), 6.37 (s, 1H), 6.82 (m, 1H), 7.02 (s, 1H), 7.21 (m,1H), 7.28 (m, 1H), 7.43 (m, 1H), 8.28 (s, 1H), 11.96 (s, 1H).

### N-(3-fluorophenyl)-2,4-dihydroxy-5-isopropyl-N-methylbenzamide (7b)

The title compound was prepared using a mixture of **60** (0.34g, 0.72mmol), NaH (0.04g, 1mmole) and DMF (3ml) and similar process as mentioned herein to afford **7b** (0.16g, 72.72%) as a white solid. m.p. 113.4-114.7. ¹H-NMR (500MHz, MeOD): δ 0.82 (d, *J=* 7 Hz, 6H), 2.94 (Sep, *J=* 7 Hz, 1H), 3.34 (s, 3H), 6.21 (s, 1H), 6.64 (s, 1H), 6.96 (m, 3H), 7.30 (m, 1H).

### N-ethyl-N-(3-fluorophenyl)-2,4-dihydroxy-5-isopropylbenzamide (7c)

The title compound was prepared using a mixture of **60** (0.36g, 0.76mmol), NaH (0.04g, 1mmole) and DMF (3ml) and similar process as mentioned herein to afford 7c (0.17g, 70.83%) as a colorless oil. ¹H-NMR (500MHz, MeOD): δ 0.81 (d, *J=* 7 Hz, 6H), 1.17 (t, *J=* 7 Hz, 3H), 2.93 (Sep, *J=* 7 Hz, 1H), 3.91 (q, *J=* 7 Hz, 2H), 6.20 (s, 1H), 6.62 (s, 1H), 6.97 (m, 3H), 7.31 (m, 1H).

### N-(3-fluorophenyl)-2,4-dihydroxy-5-isopropyl-N-propylbenzamide (7d)

The title compound was prepared using a mixture of **60** (0.39g, 0.83mmol), NaH (0.04g, 1mmole) and DMF (3ml) and similar process as mentioned herein to afford **7d** (0.17g, 60.71%) as a colorless oil. ¹H-NMR (500MHz, MeOD): δ 0.83 (d, *J=* 7 Hz, 6H), 0.92 (t, *J=* 7 Hz, 3H), 1.62 (m, 2H), 2.93 (qui, *J*= 7 Hz, 1H), 3.83 (m, 2H), 6.19 (s, 1H), 6.62 (s, 1H), 6.95 (m, 3H), 17.30 (m, 1H).

### N-(3-fluorophenyl)-2,4-dihydroxy-N,5-diisopropylbenzamide (7e)

The title compound was prepared using a mixture of **60** (0.39g, 0.83mmol), NaH (0.04g, 1mmole) and DMF (3ml) and similar process as mentioned herein to afford **7e** (0.17g, 60.70%) as a white solid. m.p. 138.7-139.5. ¹H-NMR (500MHz, MeOD): δ 0.88 (d, *J=* 6.5 Hz, 6H), 1.19 (d, *J=* 7 Hz, 6H), 2.95 (Sep, *J=* 7 Hz, 1H), 4.93 (Sep, *J=* 7 Hz, 1H), 6.17 (s, 1H), 6.64 (s, 1H), 6.94(m, 2H), 7.01 (m, 1H), 7.30 (m, 1H).

### N-(4-fluorophenyl)-2,4-dihydroxy-5-isopropylbenzamide (8a)

The title compound was prepared using a mixture of **61** (0.31g, 0.66mmol), 10% palladium on activated carbon (0.04g, 0.04mmol) and MeOH (6ml) and similar process as mentioned herein to afford **8a** (0.17g, 89.47%) as a white solid. m.p. 165.3-166.4. ¹H-NMR (500MHz, MeOD): δ 1.23 (d, *J=* 7 Hz, 6H), 3.19 (Sep, *J=* 7 Hz, 1H), 6.33 (s, 1H), 7.07 (m, 2H), 7.60 (m, 2H), 7.73 (s, 1H).

### N-(4-fluorophenyl)-2,4-dihydroxy-5-isopropyl-N-methylbenzamide (8b)

The title compound was prepared using a mixture of **61** (0.35g, 0.75mmol), NaH (0.04g, 1mmole) and DMF (3ml) and similar process as mentioned herein to afford **8b** (0.17g, 73.91%) as a white solid. m.p. 120.1-121.3. ¹H-NMR (500MHz, MeOD): δ 0.82 (d, *J=* 7 Hz, 6H), 2.94 (Sep, *J=* 7 Hz, 1H), 3.37 (s, 3H), 6.20 (s, 1H), 6.60 (s, 1H), 7.04 (m, 2H), 7.17 (m, 2H).

### N-ethyl-N-(4-fluorophenyl)-2,4-dihydroxy-5-isopropylbenzamide (8c)

The title compound was prepared using a mixture of **61** (0.34g, 0.72mmol), NaH (0.04g, 1mmole) and DMF (3ml) and similar process as mentioned herein to afford **8c** (0.17g, 73.91%) as a white solid. m.p. 121.6-122.3. ¹H-NMR (500MHz, MeOD): δ 0.81 (d, *J=* 7 Hz, 6H), 1.16 (t, *J=* 7 Hz, 3H), 2.93 (Sep, *J=* 7 Hz, 1H), 3.87 (q, *J=* 7 Hz, 2H), 6.20 (s, 1H), 6.58 (s, 1H), 7.04 (m, 1H), 7.16 (m, 1H).

### N-(4-fluorophenyl)-2,4-dihydroxy-5-isopropyl-N-propylbenzamide (8d)

The title compound was prepared using a mixture of **61** (0.30g, 0.64mmol), NaH (0.04g, 1mmole) and DMF (3ml) and similar process as mentioned herein to afford **8d** (0.14g, 66.67%) as a white solid. m.p. 137.6-138.6. ¹H-NMR (500MHz, MeOD): δ 0.84 (d, *J=* 7Hz, 6H), 0.92 (t, *J=* 7.5 Hz, 3H), 1.61 (Sex, *J*= 7.5 Hz, 2H), 2.93 (Sep, *J=* 7 Hz, 1H), 3.80 (m, 2H), 6.17 (s, 1H), 6.58 (s, 1H), 7.05 (m, 2H), 7.17 (m, 2H).

### N-(4-fluorophenyl)-2,4-dihydroxy-N,5-diisopropylbenzamide (8e)

The title compound was prepared using a mixture of **61** (0.33g, 0.70mmol), NaH (0.04g, 1mmole) and DMF (3ml) and similar process as mentioned herein to afford **8e** (0.14g, 60.87%) as a colorless oil. ¹H-NMR (500MHz, CDCl₃): δ 0.78 (d, *J=* 7 Hz, 6H), 1.17 (d, *J=* 6.5 Hz, 6H), 2.86 (Sep, *J=* 7 Hz, 1H), 5.05 (Sep, *J=* 6.5 Hz, 1H), 6.29 (d, *J=* 9.5 Hz, 2H), 6.48 (s, 1H), 7.06 (m, 4H), 11.41 (s, 1H).

### 2,4-dihydroxy-5-isopropyl-N-(3,4,5-trimethoxyphenyl)benzamide (9a)

The title compound was prepared using a mixture of **62** (0.37g, 0.68mmol), 10% palladium on activated carbon (0.04g, 0.04mmol) and MeOH (10ml) and similar process as mentioned herein to afford **9a** (0.21g, 84.0%) as a white solid. m.p. 113.9-115.8. ¹H-NMR (500MHz, MeOD): δ 1.24 (d, *J=* 7 Hz, 6H), 3.20 (Sep, *J=* 7 Hz, 1H), 3.74 (s, 3H), 3.83 (s, 6H), 6.34 (s, 1H), 7.02 (s, 2H), 7.73 (s, 1H).

### 2,4-dihydroxy-5-isopropyl-N-methyl-N-(3,4,5-trimethoxyphenyl)benzamide (9b)

The title compound was prepared using a mixture of **62** (0.38g, 0.70mmol), NaH (0.04g, 1mmole) and DMF (3ml) and similar process as mentioned herein to afford **9b** (0.17g, 65.38%) as a white solid. m.p. 163.0-164.8. ¹H-NMR (500MHz, MeOD): δ 0.85 (d, *J=* 7 Hz, 6H), 2.96 (Sep, *J=* 7 Hz, 1H), 3.41 (s, 3H), 3.72 (s, 9H), 6.20 (s, 1H), 6.51 (s, 2H), 6.70 (s, 1H).

### N-ethyl-2,4-dihydroxy-5-isopropyl-N-(3,4,5-trimethoxyphenyl)benzamide (9c)

The title compound was prepared using a mixture of **62** (0.34g, 0.63mmol), NaH(0.04g, 1mmole) and DMF (3ml) and similar process as mentioned herein to afford **9c** (0.16g, 64.0%) as a white solid. m.p. 130.2-131.9. ¹H-NMR (500MHz, MeOD): δ 0.84 (d, *J=* 7 Hz, 6H), 1.20 (t, *J=* 7 Hz, 3H), 2.95 (Sep, *J=* 7 Hz, 1H), 3.72 (d, *J=* 3 Hz, 9H), 3.91 (q, *J=* 7 Hz, 2H), 6.19 (s, 2H), 6.68 (s, 1H).

### 2,4-dihydroxy-5-isopropyl-N-propyl-N-(3,4,5-trimethoxyphenyl)benzamide (9d)

The title compound was prepared using a mixture of **62** (0.37g, 0.68mmol), NaH(0.04g, 1mmole) and DMF (3ml) and similar process as mentioned herein to afford **9d** (0.17g, 62.96%) as a white solid. m.p.168.9- 171.2. ¹H-NMR (500MHz, MeOD): δ 0.84 (d, *J=* 7 Hz, 6H), 0.94 (t, *J=* 7.5 Hz, 3H), 1.65 (m, 2H), 2.95 (Sep, *J=* 7 Hz, 1H), 3.71 (d, *J=* 4 Hz, 9H), 3.82 (m, 2H), 6.19 (s, 1H), 6.47 (s, 2H), 6.67 (s, 1H).

### 2,4-dihydroxy-N,5-diisopropyl-N-(3,4,5-trimethoxyphenyl)benzamide (9e)

The title compound was prepared using a mixture of **62** (0.39g, 0.72mmol), NaH (0.04g, 1mmole) and DMF (3ml) and similar process as mentioned herein to afford **9e** (0.17g, 58.62%) as a white solid. m.p. 140.5-142.6. ¹H-NMR (500MHz, MeOD): δ 0.89 (d, *J=* 7 Hz, 6H), 1.23 (d, *J=* 6.5 Hz, 6H), 2.96 (Sep, *J=* 7 Hz, 1H), 3.72 (s, 3H), 3.74 (s, 6H), 4.95 (qui, *J=* 7 Hz, 1H), 6.17 (s, 1H), 6.45 (s, 2H), 6.70 (s,1H).

### 2,4-dihydroxy-5-isopropyl-N-(4-morpholinophenyl)benzamide (10a)

The title compound was prepared using a mixture of **63** (0.31g, 0.58mmol), 10% palladium on activated carbon (0.04g, 0.04mmol) and MeOH (6ml) and similar process as mentioned herein to afford **10a** (0.18g, 85.71%) as a white solid. m.p. 202.1- 203.6. ¹H-NMR (500MHz, MeOD): δ 1.22 (d, *J=* 6.5 Hz, 6H), 3.10 (t, *J=* 5Hz, 4H), 3.18 (Sep, *J=* 7 Hz, 1H), 3.82 (t, *J=* 5 Hz, 4H), 6.32 (s, 1H) 6.93 (m, 2H), 7.46 (m, 2H), 7.71 (s, 1H).

### 2,4-dihydroxy-5-isopropyl-N-methyl-N-(4-morpholinophenyl)benzamide (10b)

The title compound was prepared using a mixture of **63** (0.31g, 0.58mmol), NaH (0.04g, 1mmole) and DMF (3ml) and similar process as mentioned herein to afford **10b** (0.15g, 71.43%) as a white solid. m.p. 171.1-172.8. ¹H-NMR (500MHz, CDCl₃): δ 0.73 (d, *J=* 7 Hz, 6H), 2.87 (Sep, *J=* 7 Hz, 1H), 3.11 (t, *J=* 5 Hz, 4H), 3.40 (s, 3H), 3.85 (t, *J=* 5 Hz, 4H), 6.27 (s, 1H), 6.54 (s, 1H), 6.83 (m, 3H), 7.04 (m, 2H), 11.63 (s, 1H).

### N-ethyl-2,4-dihydroxy-5-isopropyl-N-(4-morpholinophenyl)benzamide (10c)

The title compound was prepared using a mixture of **63** (0.34g, 0.63mmol), NaH (0.04g, 1mmole) and DMF (3ml) and similar process as mentioned herein to afford **10c** (0.17g, 70.83%) as a white solid. m.p. 145.4-146.5. ¹H-NMR (500MHz, CDCl₃): δ 0.74 (d, *J=* 7 Hz, 6H), 1.20 (t, *J=* 7 Hz, 3H), 2.85 (Sep, *J=* 7 Hz, 1H), 3.12 (t, *J=* 5 Hz, 4H), 3.87 (m, 6H), 6.12 (s, 1H), 6.26 (s, 1H), 6.54 (s, 1H), 6.85 (d, *J=* 8.5 Hz, 2H), 7.01 (m, 2H), 11.75 (s, 1H).

### 2,4-dihydroxy-5-isopropyl-N-(4-morpholinophenyl)-N-propylbenzamide (10d)

The title compound was prepared using a mixture of **63** (0.36g, 0.67mmol), NaH (0.04g, 1mmole) and DMF (3ml) and similar process as mentioned herein to afford **10d** (0.18g, 66.67%) as a white solid. m.p. 191.2-193.1. ¹H-NMR (500MHz, MeOD): δ 0.75 (d, *J=* 7 Hz, 6H), 0.9 (t, *J=* 7.5 Hz, 3H), 1.61 (Sex, *J=* 7.5 Hz, 2H), 2.88 (Sep, *J=* 7.5 Hz, 1H), 3.10 (t, *J=* 5 Hz, 4H), 3.78 (m, 6H), 6.19 (s, 1H), 6.54 (s, 1H), 6.88 (d, *J=* 9 Hz, 2H), 7.01 (d, *J=* 9 Hz, 2H).

### 2,4-dihydroxy-N,5-diisopropyl-N-(4-morpholinophenyl)benzamide (10e)

The title compound was prepared using a mixture of **63** (0.39g, 0.73mmol), NaH (0.04g, 1mmole) and DMF (3ml) and similar process as mentioned herein to afford **10e** (0.18g, 62.07%) as a white solid. m.p. 144.1-145.6. ¹H-NMR (500MHz, CDCl₃): δ 0.75 (d, *J=* 7 Hz, 6H), 1.16 (d, *J=* 7 Hz, 6H), 2.86 (Sep, *J=* 7 Hz, 1H), 3.13 (t, *J=* 5 Hz, 4H), 3.85 (t, *J=* 5 Hz, 4H), 5.05 (Sep, *J=* 7 Hz, 1H), 6.24 (s, 1H), 6.53 (s, 1H), 6.84 (d, *J=* 9 Hz, 2H), 6.98 (d, *J=* 9 Hz, 2H), 11.78 (s, 1H).

### 4-(2-nitrophenyl)morpholine (65)

The title compound was prepared using a mixture of 1-Bromo-2-nitroaniline (2g, 9.90mmol) and morpholine (1.3g, 14.92mmol) and similar process as mentioned herein to afford **65** (1.99g, 96.60%) as a red oily product. ¹H-NMR (500MHz, CDCl₃): δ 3.03 (t, *J=* 4.5 Hz, 4H), 3.81 (t, *J=* 4.5 Hz, 4H), 7.05 (m, 1H), 7.13 (d, *J=* 8 Hz, 1H), 7.48 (m, 1H), 7.74 (m, 1H).

### 2,4-bis(benzyloxy)-5-isopropyl-N-(2-morpholinophenyl)benzamide (66)

The title compound was prepared using a mixture of **65** (1.99g, 9.56mmol), 10% Palladium on activated carbon (0.4g, 0.38mmol) and MeOH(15ml) and similar process as mentioned herein to afford **66** (2.70g, 63.08%) as a white solid. ¹H-NMR (500MHz, CDCl₃): δ 1.26 (d, *J=* 7 Hz, 6H), 2.70 (t, *J=* 4.5 Hz, 4H), 3.33 (qui, *J*= 7 Hz, 1H), 3.50 (t, *J=* 4.5 Hz, 4H), 5.06 (s, 2H), 5.26 (s, 2H), 6.58 (s, 1H), 7.25 (m, 12H), 8.15 (s, 1H), 8.50 (d, *J=* 8 Hz, 1H), 10.36 (s, 1H).

### 2,4-dihydroxy-5-isopropyl-N-(2-morpholinophenyl)benzamide (11a)

The title compound was prepared using a mixture of **66** (0.31, 0.58mmol), 10% palladium on activated carbon (0.04g, 0.04mmol) and MeOH (8ml) and similar process as mentioned herein to afford **11a** (0.19g, 90.19%) as a white solid. m.p. 264.8- 266.9. ¹H-NMR (500MHz, MeOD): δ 1.23 (d, *J=* 7 Hz, 6H), 2.88 (t, *J=* 4.5 Hz, 4H), 3.2 (qui, *J*= 7 Hz, 1H), 3.89 (t, *J=* 4.5 Hz, 4H), 6.44 (s, 1H), 7.10 (m, 2H), 7.23 (d, *J=* 7 Hz, 1H), 7.80 (s, 1H), 8.38 (d, *J=* 7.5 Hz, 1H).

### 2,4-dihydroxy-5-isopropyl-N-methyl-N-(2-morpholinophenyl)benzamide (11b)

The title compound was prepared using a mixture of **66** (0.33g, 0.61mmol), NaH (0.04g, 1mmole) and DMF (3ml) and similar process as mentioned herein to afford **11b** (0.17g, 73.91%) as a white solid. m.p.166.4-168.3. ¹H-NMR (500MHz, MeOD): δ 0.61 (d, *J=* 6.5 Hz, 3H), 0.75 (d, *J=* 6.5 Hz, 3H), 2.26 (s, 2H), 2.76 (m, 2H), 2.84 (qui, *J*= 7 Hz, 1H), 3.38 (s, 3H), 3.61 (d, *J=* 2.5 Hz, 4H), 6.22 (s, 1H), 6.59 (s, 1H), 7.01 (m, 1H), 7.21 (m, 2H), 7.38 (m, 1H).

### N-ethyl-2,4-dihydroxy-5-isopropyl-N-(2-morpholinophenyl)benzamide (11c)

The title compound was prepared using a mixture of **66** (0.35g, 0.65mmol), NaH (0.04g, 1mmole) and DMF (3ml) and similar process as mentioned herein to afford **11c** (0.18g, 72.0%) as a white solid. m.p.72.4-73.2. ¹H-NMR (500MHz, MeOD): δ 0.70 (m, 6H), 1.29 (t, *J=* 7 Hz, 3H), 2.38 (s, 2H), 2.78 (s, 2H), 2.85 (qui, *J*= 7 Hz, 1H), 3.47 (q, *J=* 6 Hz, 1H), 3.64 (t, *J=* 4 Hz, 4H), 4.29 (d, *J=* 6 Hz, 1H), 6.22 (s, 1H), 6.60 (s, 1H), 7.05 (d, *J=* 8 Hz, 1H), 7.16 (m, 1H), 7.23 (m, 1H), 7.32 (d, *J=* 8 Hz, 1H).

### 2,4-dihydroxy-5-isopropyl-N-(2-morpholinophenyl)-N-propylbenzamide (11d)

The title compound was prepared using a mixture of **66** (0.38g, 0.71mmol), NaH (0.04g, 1mmole) and DMF (3ml) and similar process as mentioned herein to afford **11d** (0.19g, 67.86%) as a white solid. m.p.70.7-71.6. ¹H-NMR (500MHz, MeOD): δ 0.71 (m, 6H), 0.97 (t, *J=* 7.5 Hz, 3H), 1.78 (q, *J=* 7.5 Hz, 2H), 2.33 (s, 2H), 2.80 (s, 2H), 2.85 (qui, *J*= 7 Hz, 1H), 3.30 (m, 1H), 3.66 (m, 4H), 4.25 (m, 1H), 6.20 (s, 1H), 6.62 (s, 1H), 7.08 (m, 1H), 7.24 (m, 2H), 7.38 (m, 1H).

### 2,4-bis(benzyloxy)-5-isopropyl-N-(2-(morpholine-4-carbonyl)phenyl)benzamide (71)

The title compound was prepared using a mixture of 2-nitrobenzoyl chloride (3g, 16.17mmol), TEA (7ml, 49.81mmol) and DCM (30ml) and similar process as mentioned herein to afford **71** (2.94g, 65.33%) as a white solid. ¹H-NMR (500MHz, CDCl₃): δ 1.26 (d, *J=* 7 Hz, 6H), 3.35 (Sep, *J=* 7 Hz, 1H), 3.68 (m, 8H), 5.13 (s, 2H), 5.18 (s, 2H), 6.62 (s, 1H), 7.04 (m, 1H), 7.16 (m, 1H), 7.23 (m, 1H), 7.45 (m, 11H), 8.17 (s, 1H), 9.96 (s, 1H).

### 2,4-bis(benzyloxy)-5-isopropyl-N-(4-(morpholine-4-carbonyl)phenyl)benzamide (72)

The title compound was prepared using a mixture of 4-nitrobenzoyl chloride (3g, 16.17mmol), TEA (7ml, 49.81mmol) and DCM (30ml) and similar process as mentioned herein to afford 72 (2.26g, 50.22%) as a white solid. ¹H-NMR (500MHz, CDCl₃): δ 1.26 (d, *J=* 7 Hz, 6H), 3.35 (Sep, *J=* 7 Hz, 1H), 3.66 (s, 8H), 5.13 (s, 2H), 5.17 (s, 2H), 6.64 (s, 1H), 7.41 (m, 14H), 8.18 (s, 1H), 10.20 (s, 1H).

### 2,4-bis(benzyloxy)-5-isopropyl-N-(2-(morpholinomethyl)phenyl)benzamide (73)

The title compound was prepared using a mixture of 1-(chloromethyl)-2-nitrobenzene (3.00g, 17.48mmol), TEA (7ml, 49.81mmol) and DCM (25ml) and similar process as mentioned herein to afford 73 (2.27g, 51.82%) as a white solid. ¹H-NMR (500MHz, CDCl₃): δ 1.26 (d, *J=* 7 Hz, 6H), 2.23 (s, 4H), 3.30 (s, 2H), 3.34 (qui, *J*= 7 Hz, 1H), 3.42 (s, 4H), 5.08 (s, 2H), 5.11 (s, 2H), 6.57 (s, 1H), 7.29 (m, 13H), 7.86 (s, 1H), 8.36 (d, *J*= 7.5 Hz, 1H), 10.59 (s, 1H).

### 2,4-bis(benzyloxy)-5-isopropyl-N-(4-(morpholinomethyl)phenyl)benzamide .(74)

The title compound was prepared using a mixture of 1-(chloromethyl)-4-nitrobenzene (3.00g, 17.48mmol), TEA (7ml, 49.81mmol) and DCM (25ml) and similar process as mentioned herein to afford **74** (2.27g, 51.82%) as a white solid. ¹H-NMR (500MHz, CDCl3): δ 1.26 (d, *J=* 7 Hz, 6H), 2.41 (s, 4H), 3.35 (qui, *J*= 7 Hz, 1H), 3.41 (s, 2H), 3.69 (s, 4H), 5.14 (s, 2H), 5.16 (s, 2H), 6.62 (s, 1H), 7.41 (m, 14H), 8.19 (s, 1H), 9.87 (s, 1H).

### 2,4-dihydroxy-5-isopropyl-N-(2-(morpholine-4-carbonyl)phenyl)benzamide (12a)

The title compound was prepared using a mixture of **71** (0.35g, 0.62mmol), 10% palladium on activated carbon (0.04g, 0.04mmol) and MeOH (8ml) and similar process as mentioned herein to afford **12a** (0.22g, 91.67%) as a white solid. m.p. 191.7- 193.0. ¹H-NMR (500MHz, CDCl₃): δ 1.27 (d, *J=* 6.5 Hz, 6H), 3.15 (Sep, *J=* 7 Hz, 1H), 3.70 (s, 8H), 6.35 (s, 1H), 7.13 (m, 1H), 7.26 (m, 1H), 7.40 (s, 1H), 7.46 (m, 1H), 8.27 (d, *J=* 8 Hz, 1H), 10.16 (s, 1H), 12.18 (s, 1H).

### 2,4-dihydroxy-5-isopropyl-N-methyl-N-(2-(morpholine-4-carbonyl)phenyl)benzamide (12b)

The title compound was prepared using a mixture of **71** (0.33g, 0.58mol), NaH (0.04g, 1mmole) and DMF (3ml) and similar process as mentioned herein to afford **12b** (0.17g, 73.91%) as a white solid. m.p. 247.5-24.9. ¹H-NMR (500MHz, DMSO): δ 0.57 (m, 6H), 3.30 (m, 12H), 6.26 (s, 1H), 7.10 (m, 5H), 9.80 (m, 1H).

### N-ethyl-2,4-dihydroxy-5-isopropyl-N-(2-(morpholine-4-carbonyl)phenyl)benzamide (12c)

The title compound was prepared using a mixture of **71** (0.37g, 0.66mmol), NaH (0.04g, 1mmole) and DMF (3ml) and similar process as mentioned herein to afford **12c** (0.19g, 70.37%) as a white solid. m.p. 211.3- 213.0. ¹H-NMR (500MHz, DMSO): δ 0.75 (m, 6H), 1.32 (s, 3H), 1.62 (m, 1H), 3.45 (m, 9H), 4.30 (m, 1H), 6.32 (m, 1H), 6.69 (m, 2H), 7.30 (m, 3H).

### 2,4-dihydroxy-5-isopropyl-N-(2-(morpholine-4-carbonyl)phenyl)-N-propylbenzamide (12d)

The title compound was prepared using a mixture of **71** (0.38g, 0.67mmol), NaH (0.04g, 1mmole) and DMF (3ml) and similar process as mentioned herein to afford **12d** (0.2g, 68.97%) as a white solid. m.p. 190.1-192.3. ¹H-NMR (500MHz, DMSO): δ 0.88 (m, 9H), 1.75 (m, 4H), 3.41 (m, 8H), 4.32 (m, 1H), 6.30 (s, 1H), 6.69 (s, 1H), 7.30 (m, 3H).

### 2,4-dihydroxy-5-isopropyl-N-(4-(morpholine-4-carbonyl)phenyl)benzamide (14a)

The title compound was prepared using a mixture of **72** (0.41g, 0.73mmol), 10% palladium on activated carbon (0.04g, 0.04mmol) and MeOH(8ml) and similar process as mentioned herein to afford **14a** (0.26g, 92.86%) as a white solid. m.p. 284.5- 288.1. ¹H-NMR (500MHz, MeOD): δ 1.23 (d, *J=* 7 Hz, 6H), 3.19 (Sep, *J=* 7 Hz, 1H), 3.70 (m, 8H), 6.35 (s, 1H), 7.43 (t, *J=* 7 Hz, 2H), 7.74 (m, 3H).

### 2,4-dihydroxy-5-isopropyl-N-methyl-N-(4-(morpholine-4-carbonyl)phenyl)benzamide (14b)

The title compound was prepared using a mixture of **72** (0.33g, 0.58mmol), NaH (0.04g, 1mmole) and DMF (3ml) and similar process as mentioned herein to afford **14b** (0.15g, 65.22%) as a white solid. m.p. 187.9-189.3. ¹H-NMR (500MHz, MeOD): δ 0.85 (d, *J=* 7 Hz, 6H), 2.93 (Sep, *J=* 7 Hz, 1H), 3.42 (s, 3H), 3.68 (m, 8H), 6.18 (s, 1H), 6.69 (s, 1H), 7.26 (d, *J=* 8 Hz, 2H), 7.39 (d, *J=* 8 Hz, 2H).

### N-ethyl-2,4-dihydroxy-5-isopropyl-N-(4-(morpholine-4-carbonyl)phenyl)benzamide (14c)

The title compound was prepared using a mixture of **72** (0.35g, 0.62mmol), NaH (0.04g, 1mmole) and DMF (3ml) and similar process as mentioned herein to afford **14c** (0.18g, 69.23%) as a white solid. m.p. 216.2- 217.8. ¹H-NMR (500MHz, MeOD): δ 0.80 (d, *J=* 7 Hz, 6H), 1.18 (t, *J=* 7 Hz, 3H), 2.90 (Sep, *J*= 7Hz, 1H), 3.48 (m, 8H), 3.93 (q, *J=* 7 Hz, 2H), 6.19 (s, 1H), 6.63 (s, 1H), 7.22 (d, *J=* 8.5 Hz, 2H), 7.39 (m, 2H).

### 2,4-dihydroxy-5-isopropyl-N-(4-(morpholine-4-carbonyl)phenyl)-N-propylbenzamide (14d)

The title compound was prepared using a mixture of **72** (0.37g, 0.66mmol), NaH (0.04g, 1mmole) and DMF (3ml) and similar process as mentioned herein to afford **14d** (0.19g, 67.86%) as a white solid. m.p. 206.3- 207.7. ¹H-NMR (500MHz, MeOD): δ 0.78 (d, *J=* 6.5 Hz, 6H), 0.91 (t, *J=* 7.5 Hz, 3H), 1.62 (Sex, *J*= 7.5 Hz, 2H), 2.90 (Sep, *J=* 7 Hz, 1H), 3.55 (m, 8H), 3.82 (t, *J=* 7.5 Hz, 2H), 6.20 (s, 1H), 6.60 (s, 1H), 7.21 (d, *J=* 8.5 Hz, 2H), 7.37 (d, *J=* 8.5 Hz, 2H).

### 2,4-dihydroxy-N,5-diisopropyl-N-(4-(morpholine-4-carbonyl)phenyl)benzamide (14e)

The title compound was prepared using a mixture of **72** (0.39g, 0.69mmol), NaH (0.04g, 1mmole) and DMF (3ml) and similar process as mentioned herein to afford **14e** (0.19g, 65.51%) as a white solid. m.p.176.8- 177.8. ¹H-NMR (500MHz, MeOD): δ 0.92 (d, *J=* 7 Hz, 6H), 1.21 (d, *J=* 7 Hz, 6H), 2.94 (Sep, *J=* 7 Hz, 1H), 3.65 (m, 8H), 4.93 (Sep, *J=* 7 Hz, 1H), 6.11 (s, 1H), 6.67 (s, 1H), 7.25 (m, 2H), 7.36 (m, 2H).

### 2,4-dihydroxy-5-isopropyl-N-(2-(morpholinomethyl)phenyl)benzamide (15a)

The title compound was prepared using a mixture of **73** (0.31g, 0.56mmol), 10% palladium on activated carbon (0.04g, 0.04mmol) and MeOH (8ml) and similar process as mentioned herein to afford **15a** (0.11g, 52.38%) as a white solid. m.p. 191.0-192.3. ¹H-NMR (500MHz, MeOD): δ 1.30 (d, *J=* 7 Hz, 6H), 2.52 (s, 4H), 3.10 (Sex, *J*= 7 Hz, 1H), 3.65 (s, 2H), 3.79 (t, *J=* 4.5 Hz, 4H), 6.36 (s, 1H), 7.08 (m, 1H), 7.17 (d, *J=* 6.5 Hz, 1H), 7.31 (s, 1H), 7.36 (m, 1H), 8.10 (d, *J=* 8 Hz, 1H).

### N-ethyl-2,4-dihydroxy-5-isopropyl-N-(2-(morpholinomethyl)phenyl)benzamide (15b)

The title compound was prepared using a mixture of **73** (0.35, 0.64mmol), NaH (0.04g, 1mmole) and DMF (3ml) and similar process as mentioned herein to afford **15b** (0.15g, 57.69%) as a colorless oil. ¹H-NMR (500MHz, MeOD): δ 0.64 (s, 3H), 0.76 (s, 3H), 1.25 (s, 3H), 2.26 (d, *J=* 5.5 Hz, 4H), 2.85 (s, 1H), 3.05 (m, 1H), 3.61 (m, 6H), 4.14 (s, 1H), 6.20 (s, 1H), 6.50 (s, 1H), 7.31 (m, 1H), 7.39 (d, *J=* 4 Hz, 2H), 7.51 (d, *J=* 7 Hz, 1H).

### 2,4-dihydroxy-5-isopropyl-N-(2-(morpholinomethyl)phenyl)-N-propylbenzamide (15c)

The title compound was prepared using a mixture of **73** (0.36g, 0.65mmol), NaH (0.04g, 1mmole) and DMF (3ml) and similar process as mentioned herein to afford **15c** (0.13g, 48.15%) as a yellow solid. m.p. 137.2-139.4. ¹H-NMR (500MHz, MeOD): δ 0.64 (d, *J*= 4 Hz, 3H), 0.77 (d, *J=* 4 Hz, 3H), 0.95 (s, 3H), 1.72 (s, 2H), 2.25 (s, 4H), 2.84 (s, 1H), 3.04 (m, 1H), 3.47 (m, 6H), 4.09 (q, *J=* 7 Hz, 2H), 6.19 (s, 1H), 6.51 (s, 1H), 7.31 (m, 1H), 7.38 (m, 2H), 7.49 (d, *J=* 7 Hz, 1H).

### 2,4-dihydroxy-5-isopropyl-N-(4-(morpholinomethyl)phenyl)benzamide (17a)

The title compound was prepared using a mixture of **74** (0.31g, 0.56mmol), 10% palladium on activated carbon (0.04g, 0.04mmol) and MeOH (6ml) and similar process as mentioned herein to afford **17a** (0.1g, 47.62%) as a pink solid. m.p. 111.2-113.1. ¹H-NMR (500MHz, MeOD): δ 1.23 (d, *J=* 7 Hz, 6H), 2.45 (m, 4H), 3.19 (Sex, *J*= 7 Hz, 1H), 3.47 (s, 2H), 3.66 (t, *J=* 4.5 Hz, 4H), 6.33 (s, 1H), 7.29 (d, *J=* 8.5 Hz, 2H), 7.57 (d, *J=* 8.5 Hz, 2H), 7.74 (s, 1H).

### N-ethyl-2,4-dihydroxy-5-isopropyl-N-(4-(morpholinomethyl)phenyl)benzamide (17b)

The title compound was prepared using a mixture of **74** (0.38g, 0.69mmol), NaH (0.04g, 1mmole) and DMF (3ml) and similar process as mentioned herein to afford **17b** (0.14g, 51.85%) as a white solid. m.p. 153.0-154.7. ¹H-NMR (500MHz, MeOD): δ 0.77 (d, *J=* 7 Hz, 6H), 1.17 (t, *J=* 7 Hz, 3H), 2.42 (t, *J=* 4.5 Hz, 4H), 2.87 (sep, *J=* 7 Hz, 1H), 3.45 (s, 2H), 3.65 (t, *J=* 4.5 Hz, 4H), 3.89 (q, *J=* 7 Hz, 2H), 6.18 (s, 1H), 6.60 (s, 1H), 7.11 (d, *J*= 8.5 Hz, 2H), 7.30 (d, *J=* 8.5 Hz, 2H).

### 2,4-dihydroxy-5-isopropyl-N-(4-(morpholinomethyl)phenyl)-N-propylbenzamide (17c)

The title compound was prepared using a mixture of **74** (0.34g, 0.62mmol), NaH (0.04g, 1mmole) and DMF (3ml) and similar process as mentioned herein to afford **17c** (0.14g, 53.84%) as a white solid. m.p. 144.3- 145.0. ¹H-NMR (500MHz, MeOD): δ 0.78 (d, *J=* 6.5 Hz, 6H), 0.91 (t, *J=* 7.5 Hz, 3H), 1.61 (Sex, *J*= 7.5 Hz, 2H), 2.42 (m, 4H), 2.87 (Sep, *J=* 7 Hz, 1H), 3.45 (s, 2H), 3.65 (t, *J=* 4.5 Hz, 4H), 3.81 (m, 2H), 6.17 (s, 1H), 6.60 (s, 1H), 7.11 (d, *J=* 8.5 Hz, 2H), 7.29 (d, *J=* 8.5 Hz, 2H).

### Morpholino(3-nitrophenyl)methanone (76)

The title compound was prepared using a mixture of 3-nitrobenzaldehyde (3.00g, 19.85mmol), sulfamic acid (10.15g, 104.53mmol), DMSO (2ml), THF (20ml) and water (20ml) and similar process as mentioned herein to afford **76** (2.89g, 61.62%) as a yellow solid.¹H-NMR (500MHz, CDCl₃): δ 3.61 (m, 8H), 7.59 (t, *J=* 8 Hz, 1H), 7.70 (d, *J=* 8 Hz, 1H), 8.22 (m, 2H).

### 4-(3-nitrobenzyl)morpholine (77)

The title compound was prepared using a mixture of 3-nitrobenzaldehyde (3.00g, 19.85mmol), morpholine (2.5ml, 28.90mmol), MeOH (15ml) and acetic acid (cat.) and similar process as mentioned herein to afford **77** (1.83g, 41.50%) as a oily product. ¹H-NMR (500MHz, CDCl₃): δ 2.45 (t, *J=* 4.5 Hz, 4H), 3.58 (s, 2H), 3.71 (t, *J=* 4.5 Hz, 4H), 7.48 (t *J*= 7.5 Hz, 1H), 7.67 (d, *J=* 7.5 Hz, 1H), 8.11 (m, 1H), 8.21 (s, 1H).

### 2,4-bis(benzyloxy)-5-isopropyl-N-(3-(morpholine-4-carbonyl)phenyl)benzamide (78)

The title compound was prepared using a mixture of **76** (2.5g, 10.58mmol), 10% palladium on activated carbon (0.25g, 0.23mmol) and MeOH (15ml) and similar process as mentioned herein to afford **78** (3.52g, 57.70%) as a white solid.¹H-NMR (500MHz, CDCl₃): δ 1.26 (d, *J=* 7 Hz, 6H), 3.35 (Sep, *J=* 7 Hz, 1H), 3.68 (m, 8H), 5.13 (s, 2H), 5.18 (s, 2H), 6.62 (s, 1H), 7.04 (m, 1H), 7.16 (m, 1H), 7.23 (m, 1H), 7.45 (m, 11H), 8.17 (s, 1H), 9.96 (s, 1H).

### 2,4-bis(benzyloxy)-5-isopropyl-N-(3-(morpholinomethyl)phenyl)benzamide (79)

The title compound was prepared using a mixture of **77** (1.80g, 8.10mmol), Fe powder (2.26g, 40.47mmol), NH₄Cl (1,70g, 31.78mmol), IPA (64ml) and H₂O (16ml) and similar process as mentioned herein to afford **79** (1.5g, 51.36%) as a white solid. ¹H-NMR (500MHz, CDCl₃): δ 1.27 (d, *J=* 7 Hz, 6H), 2.41 (s, 4H), 3.35 (qui, *J*= 7 Hz, 3H), 3.69 (t, *J=* 4.5 Hz, 4H), 5.15 (s, 2H), 5.16 (s, 2H), 6.63 (s, 1H), 7.40 (m, 14H), 8.21 (s, 1H), 9.89 (s, 1H).

### 2,4-dihydroxy-5-isopropyl-N-(3-(morpholine-4-carbonyl)phenyl)benzamide (13a)

The title compound was prepared using a mixture of **78** (0.40g, 0.71mmol), 10% palladium on activated carbon (0.04g, 0.04mmol) and MeOH (8ml) and similar process as mentioned herein to afford **13a** (0.25g, 92.59%) as a white solid. m.p. 246.0- 247.4. ¹H-NMR (500MHz, MeOD): δ 1.24 (d, *J=* 7 Hz, 6H), 3.19 (Sep, *J=* 7 Hz, 1H), 3.65 (m, 8H), 6.34 (s, 1H), 7.17 (d, *J=* 7.5 Hz, 1H), 7.43 (t, *J=* 8 Hz, 1H), 7.68 (m, 1H) 7.55 (s, 1H), 7.79 (d, *J=* 1.5 Hz, 1H).

### 2,4-dihydroxy-5-isopropyl-N-methyl-N-(3-(morpholine-4-carbonyl)phenyl)benzamide (13b)

The title compound was prepared using a mixture of **78** (0.35g, 0.62mmol), NaH (0.04g, 1mmole) and DMF (3ml) and similar process as mentioned herein to afford **13b** (0.17g, 68.0%) as a white solid. m.p. 216.3-217.6. ¹H-NMR (500MHz, MeOD): δ 0.84 (d, *J=* 7 Hz, 6H), 2.96 (Sex, *J*= 7 Hz, 3H), 3.35 (s, 2H), 3.44 (s, 3H), 3.64 (s, 4H), 6.19 (s, 1H), 6.65 (s, 1H), 6.96 (s, 1H), 7.28 (m, 1H), 7.52 (m, 2H).

### 2,4-dihydroxy-5-isopropyl-N-(3-(morpholine-4-carbonyl)phenyl)-N-propylbenzamide (13c)

The title compound was prepared using a mixture of **78** (0.33g, 0.58mmol), NaH (0.04g, 1mmole) and DMF (3ml) and similar process as mentioned herein to afford **13c** (0.17g, 70.83%) as a white solid.¹H-NMR (500MHz, MeOD): δ 0.83 (d, *J=* 7 Hz, 6H), 1.19 (t, *J=* 7 Hz, 3H), 2.89 (Sex, *J*= 7 Hz, 3H), 3.35 (s, 2H), 3.64 (s, 4H), 3.96 (q, *J=* 7 Hz, 2H), 6.19 (s, 1H), 6.64 (s, 1H), 6.92 (s, 1H), 7.30 (m, 1H), 7.53 (m, 2H).

### 2,4-dihydroxy-5-isopropyl-N-(3-(morpholine-4-carbonyl)phenyl)-N-propylbenzamide (13d)

The title compound was prepared using a mixture of **78** (0.36g, 0.63mmol), NaH (0.04g, 1mmole) and DMF (3ml) and similar process as mentioned herein to afford **13d** (0.19g, 70.37%) as a white solid. m.p. 219.1-220.8. ¹H-NMR (500MHz, MeOD): δ 0.78 (d, *J=* 7 Hz, 6H), 0.92 (t, *J=* 7.5 Hz, 3H), 1.62 (Sep, *J=* 7.5 Hz, 2H), 2.87 (m, 3H), 3.33 (s, 2H), 3.64 (s, 4H), 3.85 (t, *J=* 7.5 Hz, 2H), 6.21 (s, 1H), 6.58 (s, 1H), 6.87 (d, *J=* 2 Hz, 1H), 7.29 (m, 1H), 7.51 (m, 2H).

### 2,4-dihydroxy-N,5-diisopropyl-N-(3-(morpholine-4-carbonyl)phenyl)benzamide (13e)

The title compound was prepared using a mixture of **78** (0.36g, 0.63mmol), NaH (0.04g, 1mmole) and DMF (3ml) and similar process as mentioned herein to afford **13e** (0.18g, 66.67%) as a white solid. m.p. 224.8-227.0. ¹H-NMR (500MHz, MeOD): δ 0.80 (d, *J*= 5.5 Hz, 6H), 1.22 (m, 6H), 2.76 (s, 2H), 2.87 (Sep, *J=* 7 Hz, 1H), 3.32 (s, 2H), 3.65 (s, 4H), 4.96 (Sep, *J=* 7 Hz, 1H), 6.17 (s, 1H), 6.53 (s, 1H), 6.82 (s, 1H), 7.32 (m, 1H), 7.48 (m, 2H).

### 2,4-dihydroxy-5-isopropyl-N-(3-(morpholinomethyl)phenyl)benzamide (16a)

The title compound was prepared using a mixture of **79** (0.5g, 0.91mmol), 10% palladium on activated carbon (0.04g, 0.04mmol) and MeOH (8ml) and similar process as mentioned herein to afford **16a** (0.19g, 55.88%) as a white solid. m.p. 90.0-90.7. ¹H-NMR (500MHz, CDCl₃): δ 1.24 (d, *J=* 7 Hz, 6H), 2.48 (s, 4H), 3.20 (qui, *J*= 7 Hz, 1H), 3.53 (s, 2H), 3.69 (t, *J=* 4.5 Hz, 4H), 6.34 (s, 1H), 7.11 (d, *J=* 7.5 Hz, 1H), 7.31 (t, *J=* 7.5 Hz, 1H), 7.55 (m, 1H), 7.60 (s 1H), 7.45 (s, 1H).

### N-ethyl-2,4-dihydroxy-5-isopropyl-N-(3-(morpholinomethyl)phenyl)benzamide (16b)

The title compound was prepared using a mixture of **79** (0.40g, 0.73mmol), NaH (0.04g, 1mmole) and DMF (3ml) and similar process as mentioned herein to afford **16b** (0.18g, 62.07%) as a white solid. m.p. 201.5-203.2. ¹H-NMR (500MHz, MeOD): δ 0.79 (d, *J=* 7 Hz, 6H), 1.20 (t, *J=* 7 Hz, 3H), 2.19 (t, *J=* 4.5 Hz, 4H), 2.87 (Sep, *J=* 7 Hz, 1H), 3.41 (s, 2H), 3.57 (t, *J=* 4.5 Hz, 4H), 3.95 (q, *J=* 7 Hz, 2H), 6.20 (s, 1H), 6.59 (s, 1H), 6.94 (s, 1H), 7.18 (d, *J=* 7.5 Hz, 1H), 7.27 (d, *J=* 9 Hz, 1H), 7.38 (t, *J=* 7.5 Hz, 1H).

### 2,4-dihydroxy-5-isopropyl-N-(3-(morpholinomethyl)phenyl)-N-propylbenzamide (16c)

The title compound was prepared using a mixture of **79** (0.35g, 0.64mmol), NaH (0.04g, 1mmole) and DMF (3ml) and similar process as mentioned herein to afford **16c** (0.15g, 57.69%) as a white solid. m.p. 224.8-227.0. ¹H-NMR (500MHz, CDCl₃): δ 0.62 (d, *J=* 7 Hz, 6H), 0.86 (t, *J=* 7.5 Hz, 3H), 1.58 (Sex, *J*= 7.5 Hz, 2H), 2.13 (d, *J=* 4.5 Hz, 4H), 2.76 (Sep, *J=* 7 Hz, 1H), 3.32 (s, 2H), 3.51 (t, *J=* 4.5 Hz, 4H), 3.75 (t, *J=* 7.5 Hz, 2H), 6.16 (s, 1H), 6.41 (s, 1H), 6.86 (s,1 H), 7.06 (d, *J=* 7.5 Hz, 1H), 7.27 (t, *J=* 8 Hz, 1H).

### 2,4-bis(benzyloxy)-5-isopropyl-N-(pyridin-3-yl)benzamide (80)

The title compound was prepared using a mixture of **47** (2g, 5.31mmol), EDC.HCl (1.5g, 7.85mmol), HOBt (0.9g, 6.67mmol), NMM (1.4ml, 12.75mmol) and DMF (8ml) and similar process as mentioned herein to afford **80** (1.76g, 73.33%) as a white solid. ¹H-NMR (500MHz, CDCl₃): δ 1.27 (d, *J=* 7 Hz, 6H), 3.36 (Sep, *J=* 7 Hz, 1H), 5.15 (s, 2H), 5.18 (s, 2H), 6.64 (s, 1H), 7.18 (m, 1H), 7.45 (m, 10H), 7.95 (m, 1H), 8.16 (m, 1H), 8.20 (s, 1H), 8.28 (m, 1H), 9.93 (s, 1H).

### 2,4-bis(benzyloxy)-5-isopropyl-N-(pyridin-4-yl)benzamide (81)

The title compound was prepared using a mixture of **47** (2g, 5.31mmol), EDC.HCl (1.5g, 7.85mmol), HOBt (0.9g, 6.67mmol), NMM (1.4ml, 12.75mmol) and DMF (8ml) and similar process as mentioned herein to afford **81** (1.7g, 70.83%) as a white solid. ¹H-NMR (500MHz, CDCl₃): δ 1.27 (d, *J=* 7 Hz, 6H), 3.35 (qui, *J*= 7 Hz, 1H), 5.14 (s, 2H), 5.18 (s, 2H), 6.64 (s, 1H), 7.07 (m, 2H), 7.41 (m, 10H), 8.16 (s, 1H), 8.34 (m, 2H), 10.01 (s, 1H).

### 2,4-dihydroxy-5-isopropyl-N-(pyridin-3-yl)benzamide (18a)

The title compound was prepared using a mixture of **80** (0.42g, 0.93mmol), 10% palladium on activated carbon (0.04g, 0.04mmol) and MeOH (8ml) and similar process as mentioned herein to afford **18a** (0.22g, 88.0%) as a white solid. m.p. 269.1-270.3. ¹H-NMR (500MHz, MeOD): δ 1.24 (d, *J=* 7 Hz, 6H), 3.20 (Sep, *J=* 7 Hz, 1H), 6.35 (s, 1H), 7.43 (m, 1H), 7.75 (s, 1H), 8.19 (m, 1H), 8.27 (m, 1H), 8.82 (d, *J=* 2.5 Hz, 1H).

### N-ethyl-2,4-dihydroxy-5-isopropyl-N-(pyridin-3-yl)benzamide (18b)

The title compound was prepared using a mixture of **80** (0.35g, 0.77mmol), NaH (0.04g, 1mmole ) and DMF (4ml) and similar process as mentioned herein to afford **18b** (0.16g, 69.57%) as a yellow solid. m.p. 179.6-181.5. ¹H-NMR (500MHz, MeOD): δ 0.90 (d, *J=* 7 Hz, 6H), 1.20 (t, *J=* 7 Hz 3H), 2.96 (Sep, *J*= 7 Hz, 1H), 3.95 (q, *J=* 7 Hz, 2H), 6.15 (s, 1H), 6.66 (s, 1H), 7.39 (m, 1H), 7.69 (m, 1H), 8.28 (d, *J=* 2 Hz, 1H), 8.34 (m, 1H).

### 2,4-dihydroxy-5-isopropyl-N-propyl-N-(pyridin-3-yl)benzamide (18c)

The title compound was prepared using a mixture of **80** (0.40g, 0.88mmol), NaH (0.04g, 1mmole) and DMF(4ml) and similar process as mentioned herein to afford **18c** (0.19g, 67.85%) as a white solid. m.p. 196.3- 198.2. ¹H-NMR (500MHz, MeOD): δ 0.92 (m, 9H), 1.63 (6, *J=* 7.5 Hz, 2H), 2.97 (Sep, *J=* 7 Hz, 1H), 3.87 (t, *J=* 7.5 Hz, 2H), 6.14 (s, 1H), 6.66 (s, 1H), 7.38 (m, 1H), 7.70 (m, 1H), 8.29 (m, 2H).

### 2,4-dihydroxy-N,5-diisopropyl-N-(pyridin-3-yl)benzamide (18d)

The title compound was prepared using a mixture of **80** (0.34g, 0.75mmol), NaH (0.04g, 1mmole) and DMF (3ml) and similar process as mentioned herein to afford **18d** (0.15g, 60.25%) as a yellow solid. m.p. 172.4-174.3. ¹H-NMR (500MHz, MeOD): δ 0.96 (d, *J=* 6.5 Hz, 6H), 1.19 (d, *J=* 7 Hz, 6H), 2.98 (Sep, *J=* 7 Hz, 1H), 4.96 (Sep, *J=* 7 Hz, 1H), 6.11 (s, 1H), 6.66 (s, 1H), 7.37 (m, 1H), 7.68 (m, 1H), 8.29 (d, *J=* 2.5 Hz, 1H), 8.37 (m, 1H).

### 2,4-dihydroxy-5-isopropyl-N-(pyridin-4-yl)benzamide (19a)

The title compound was prepared using a mixture of **81** (0.38g, 0.84mmol), 10% palladium on activated carbon (0.04g, 0.04mmol) and MeOH (8ml) and similar process as mentioned herein to afford **19a** (0.20g, 86.95%) as a yellow solid. m.p. 235.9-238.3. ¹H-NMR (500MHz, MeOD): δ 1.23 (d, *J=* 7 Hz, 6H), 3.19 (Sep, *J=* 7 Hz, 1H), 6.36 (s, 1H), 7.77 (m, 3H), 8.40 (d, *J=* 6.5 Hz, 2H).

### N-ethyl-2,4-dihydroxy-5-isopropyl-N-(pyridin-4-yl)benzamide (19b)

The title compound was prepared using a mixture of **81** (0.33g, 0.73mmol), NaH (0.04g, 1mmole ) and DMF (4ml) and similar process as mentioned herein to afford **19b** (0.14g, 63.64%) as a white solid. m.p. 185.2-186.1. ¹H-NMR (500MHz, MeOD): δ 0.86 (d, *J=* 7 Hz, 6H), 1.21 (t, *J=* 7 Hz, 3H), 2.97 (Sep, *J=* 7 Hz, 1H) 4.01 (q, *J=* 7 Hz, 2H), 6.20 (s, 1H), 6.68 (s, 1H), 7.17 (m, 2H), 8.40 (m, 2H).

### 2,4-dihydroxy-5-isopropyl-N-propyl-N-(pyridin-4-yl)benzamide (19c)

The title compound was prepared using a mixture of **81** (0.32g, 0.71mmol), NaH (0.04g, 1mmole ) and DMF (4ml) and similar process as mentioned herein to afford **19c** (0.14g, 63.64%) as a white solid. m.p. 196.3- 198.2. ¹H-NMR (500MHz, MeOD): δ 0.86 (d, *J=* 7 Hz, 6H), 0.94 (t, *J=* 7.5 Hz, 3H), 1.65 (Sex, *J*= 7.5 Hz, 2H), 2.97 (Sep, *J=* 7 Hz, 1H), 3.92 (t, *J=* 7.5 Hz, 2H), 6.20 (s, 1H), 6.68 (s, 1H), 7.17 (m, 2H), 8.39 (m, 2H).

### 2,4-dihydroxy-N,5-diisopropyl-N-(pyridin-4-yl)benzamide (19d)

The title compound was prepared using a mixture of **81** (0.34g, 0.75mmol), NaH (0.04g, 1mmole) and DMF (3ml) and similar process as mentioned herein to afford **19d** (0.15g, 60.25%) as a white solid. m.p. 165.4-166.6. ¹H-NMR (500MHz, MeOD): δ 0.89 (d, *J=* 7 Hz, 6H), 1.27 (d, *J=* 6.5 Hz, 6H), 2.96 (Sep, *J=* 7 Hz, 1H), 4.89 (Sep, *J=* 7 Hz, 1H), 6.15 (s, 1H), 6.63 (s, 1H), 7.21 (d, *J=* 6 Hz, 2H), 8.44 (m, 2H).

### tert-butyl 5-(2,4-bis(benzyloxy)-5-isopropylbenzamido)-1H-indole-1-carboxylate (84)

The title compound was prepared using a mixture of 5-nitroindole (2g, 12.33mmol) was dissolved in DCM (15ml) and similar process as mentioned herein to afford **84** (2.46g, 78.59%) as a white solid. ¹H-NMR (500MHz, CDCl₃): δ 1.28 (d, *J=* 7 Hz, 6H), 1.16 (s, 9H), 3.36 (qui, *J*= 7 Hz, 1H), 5.15 (s, 4H), 6.45 (s, 1H), 6.46 (s, 1H), 6.80 (s, 1H), 7.44 (m, 11H), 7.91 (s, 2H), 8.24 (s, 1H), 9.95 (s, 1H).

### tert-butyl 6-(2,4-bis(benzyloxy)-5-isopropylbenzamido)-1H-indole-1-carboxylate (85)

The title compound was prepared using a mixture of 6-nitroindole (0.2g, 1.23mmol) dissolved in DCM (10ml), DMAP (0.23g, 1.85mmol) and Boc anhydride (0.4g, 1.85mmol) and similar process as mentioned herein to afford **85** (0.26g, 82.45%) as a white solid. ¹H-NMR (300 MHz, CDCl₃): δ 1.28 (d, *J=* 6.9 Hz, 6H), 1.68 (s, 9H), 3.35 (m, 1H), 5.15 (s, 2H), 5.18 (s, 2H), 6.47 (d, *J=* 3.6 Hz, 1H), 6.62 (s, 1H), 6.86 (d, *J=* 8.1 Hz, 1H), 7.33 (d, *J*= 8.1 Hz, 1H), 7.36-7.52 (m, 11H), 8.23 (s, 1H), 8.66 (s, 1H), 9.92 (s, 1H).

### 2,4-dihydroxy-N-(1H-indol-5-yl)-5-isopropylbenzamide (20a)

The title compound was prepared using a mixture of **84** (0.5g, 0.85mmol) dissolved in DCM (5ml) and TFA (1ml) and similar process as mentioned herein to afford **20a** (0.19g, 73.07%) as a green solid. m.p. 221.1-223.7. ¹H-NMR (500MHz, MeOD): δ 1.22 (d, *J=* 6.5 Hz, 6H), 3.19 (Sep, *J=* 7 Hz, 1H), 6.35 (s, 1H), 6.41 (d, *J=* 7 Hz, 1H), 7.15 (d, *J=* 2 Hz, 1H), 7.20 (m, 1H), 7.32 (d, *J=* 8.5 Hz, 1H), 7.24 (m, 2H).

### 2,4-dihydroxy-N-(1H-indol-5-yl)-5-isopropyl-N-methylbenzamide (20b)

The title compound was prepared using a mixture of **84** (0.8g, 1.35mmol), NaH (0.07g, 1.75mmole ) and DMF (5ml) and similar process as mentioned herein to afford **20b** (0.25g, 56.82%)as a white solid. m.p. 217.7-219.5. ¹H-NMR (500MHz, MeOD): δ 0.49 (d, *J=* 7 Hz, 6H), 2.74 (qui, *J*= 7 Hz, 1H), 3.46 (s, 3H), 6.16 (s, 1H), 6.41 (d, *J=* 3 Hz, 1H), 6.48 (s, 1H), 6.94 (m, 1H), 7.26 (d, *J=* 3 Hz, 1H), 7.33 (s, 1H), 7.37 (d, *J=* 8.5 Hz, 1H).

### N-ethyl-2,4-dihydroxy-N-(1H-indol-5-yl)-5-isopropylbenzamide (20c)

The title compound was prepared using a mixture of **84** (0.75g, 1.27mmol), NaH (0.07g, 1.75mmole ) and DMF (6ml) and similar process as mentioned herein to afford **20c** (0.22g, 51.16%) as a white solid. m.p. 234.4-236.7. ¹H-NMR (500MHz, MeOD): δ 0.46 (d, *J=* 6.5 Hz, 6H), 1.21 (t, *J=* 7 Hz, 3H), 2.72 (Sex, *J*= 7 Hz, 1H), 3.94 (q, *J=* 7 Hz, 2H), 6.17 (s. 1H), 6.39 (d, *J=* 3 Hz, 1H), 6.45 (s, 1H), 6.91 (m, 1H), 7.24 (d, *J=* 3 Hz, 1H), 7.30 (d, *J=* 2 Hz, 1H), 7.37 (d, *J=* 8.5 Hz, 1H).

### 2,4-dihydroxy-N-(1H-indol-5-yl)-5-isopropyl-N-propylbenzamide (20d)

The title compound was prepared using a mixture of **84** (0.78g, 1.32mmol), NaH (0.07g, 1.75mmole ) and DMF (6ml) and similar process as mentioned herein to afford **20d** (0.24g, 51.06%) as a white solid. m.p. 206.0-207.8. ¹H-NMR (500MHz, MeOD): δ 0.48 (d, *J=* 6.5 Hz, 6H), 0.93 (t, *J=* 7.5 Hz, 3H), 1.66 (Sex, *J*= 7.5 Hz, 2H), 2.73 (Sex, *J*= 6.5 HZ, 1H), 3.86 (t, *J=* 7.5 Hz, 2H), 6.16 (s, 1H), 6.40 (d, *J=* 3 Hz, 1H), 6.46 (s, 1H), 6.91 (s, 1H), 7.25 (d, *J=* 3.5 Hz, 1H), 7.31 (d, *J=* 1.5 Hz, 1H), 7.34 (d, *J=* 8.5 Hz, 1H).

### 2,4-dihydroxy-N-(1H-indol-5-yl)-N,5-diisopropylbenzamide (20e)

The title compound was prepared using a mixture of **84** (0.81g, 1.37mmol), NaH (0.07g, 1.75mmole ) and DMF(7ml) and similar process as mentioned herein to afford **20e** (0.23g, 47.91%) as a white solid. m.p. 226.1-227.9. ¹H-NMR (500MHz, MeOD): δ 0.56 (d, *J*= 5.5 Hz, 6H), 1.19 (d, *J=* 7 Hz, 6H), 2.76 (qui, *J*= 7 Hz, 1H), 5.04 (t, *J=* 5.5 Hz, 1H), 6.13 (s, 1H), 6.42 (d, *J=* 3 Hz, 1H), 6.49 (s, 1H), 6.86 (m, 1H), 7.24 (d, *J=* 3 Hz, 1H), 7.31 (d, *J*= 1.5 Hz, 1H), 7.33 (d, *J=* 9 Hz, 1H).

### 2,4-dihydroxy-N-(1H-indol-6-yl)-5-isopropylbenzamide (21a)

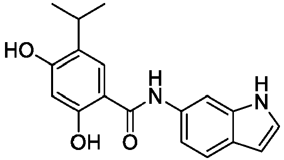

### 2,4-dihydroxy-N-(indolin-5-yl)-5-isopropylbenzamide (23a)

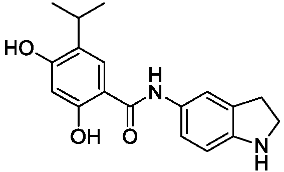

The title compound was prepared using a mixture of **20a** (0.25g, 0.81mmol) dissolved in acetic acid (5ml) and NaBH₃CN (0.2g, 3.18mmol) and similar process as mentioned herein to afford **23a** (0.19g, 76.0%) as a green solid. m.p. 104.9-107.5. ¹H-NMR (500MHz, MeOD): δ 1.23 (d, *J=* 7 Hz, 6H), 3.00 (t, *J=* 8.5 Hz, 2H), 3.19 (hept, *J=* 7 Hz, 1H), 3.48 (t, *J=* 8.5 Hz, 2H), 6.31 (s, 1H), 6.66 (d, *J=* 8.5 Hz, 1H), 7.12 (m, 1H), 7.32 (s, 1H), 7.70 (s, 1H).

### 2,4-dihydroxy-N-(indolin-5-yl)-5-isopropyl-N-methylbenzamide (23b)

The title compound was prepared using a mixture of **20b** (0.27g, 0.83mmol) dissolved in acetic acid (6ml) and NaBH₃CN (0.2g, 3.18mmol) and similar process as mentioned hereinto afford **23b** (0.2g, 74.07%) as a pink solid. m.p. 176.7-178.3. ¹H-NMR (500MHz, MeOD): δ 0.82 (d, *J=* 7 Hz, 6H), 2.92 (m, 3H), 3.34 (s, 3H), 3.46 (t, *J=* 8.5 Hz, 2H), 6.17 (s, 1H), 6.56 (d, *J=* 8 Hz, 1H), 6.68 (s, 1H), 6.76 (m, 1H), 6.90 (s, 1H).

### N-ethyl-2,4-dihydroxy-N-(indolin-5-yl)-5-isopropylbenzamide (23c)

The title compound was prepared using a mixture of **20c** (0.2g, 0.59mmol) dissolved in acetic acid (5ml) and NaBH₃CN (0.2g, 3.18mmol) and similar process as mentioned hereinto afford **23c** (0.14g, 70. 0%) as a white solid. 213.3- 215.6. ¹H-NMR (500MHz, MeOD): δ 0.82 (d, *J=* 6.5 Hz, 6H), 1.17 (t, J= 7 Hz, 3H), 2.91 (m, 3H), 3.47 (t, *J=* 8 Hz, 2H), 3.83 (q, *J=* 7 Hz, 2H), 6.17 (s, 1H), 6.56 (d, *J=* 8 Hz, 1H), 6.67 (s, 1H), 6.72 (m, 1H), 6.87 (d, *J=* 1.5 Hz, 1H).

### 2,4-dihydroxy-N-(indolin-5-yl)-5-isopropyl-N-propylbenzamide (23d)

The title compound was prepared using a mixture of **20d** (0.24g, 0.68mmol) dissolved in acetic acid (5ml) and NaBH₃CN (0.2g, 3.18mmol) and similar process as mentioned herein to afford **23d** (0.17g, 70.83%) as a brown solid. m.p. 150.1-152.2. ¹H-NMR (500MHz, MeOD): δ 0.82 (d, *J=* 7 Hz, 6H), 0.92 (t, *J=* 7.5 Hz, 3H), 1.62 (Sex, *J*= 7.5 Hz, 2H), 2.91 (m, 3H), 3.46 (t, *J=* 8.5 Hz, 2H), 3.74 (m, 2H), 6.16 (s, 1H), 6.56 (t, *J=* 8.5 Hz, 1H), 6.66 (s, 1H), 6.72 (m, 1H), 6.88 (d, *J=* 1.5 Hz, 1H).

### 2,4-dihydroxy-N-(indolin-5-yl)-N,5-diisopropylbenzamide (23e)

The title compound was prepared using a mixture of **20e** (0.2g, 0.57mmol) dissolved in acetic acid (4ml) and NaBH₃CN (0.2g, 3.18mmol) and similar process as mentioned herein to afford **23e** (0.14g, 70.0 %) as a pink solid. m.p. 226.3-228.1. ¹H-NMR (500MHz, MeOD): δ 0.87 (d, *J=* 7 Hz, 6H), 1.15 (d, *J=* 7 Hz, 6H), 2.94 (m, 3H), 3.47 (t, *J=* 8.5 Hz, 2H), 4.94 (t, *J=* 6.5 Hz, 1H), 6.13 (s, 1H), 6.54 (d, *J=* 8 Hz, 1H), 6.68 (m, 2H), 6.86 (s, 1H).

### 1-methyl-5-nitro-1H-indole (89)

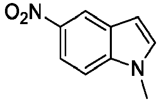

A mixture of 5-nitroindole (2g, 12.33mmol), NaH (0.6g, 15mmole ) and DMF (10ml) was added methyl iodide (1.5ml, 24.09mmol) at room temperature for 2h. The reaction was quenched with water and extracted with ethyl acetate (30ml *3). The organic layer was collected and dried over anhydrous MgSO₄ concentrated *in vacuo* and purified by silica gel chromatography (ethyl acetate: n-hexane = 1: 4, Rf= 0.2) to afford **89** (2g, 91.71 %) as a yellow solid. ¹H-NMR (500MHz, CDCl₃): δ 3.86 (s 3H), 6.67 (d, *J=* 3Hz, 1H), 7.20 (d, *J=* 3Hz, 1H), 7.33 (d, *J=* 9 Hz, 1H), 8.13 (m, 1H), 8.58 (d, *J=* 2 Hz, 1H).

### 1-methyl-6-nitro-1H-indole (90)

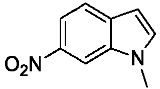

### 1-methyl-7-nitro-1H-indole (91)

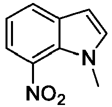

### 2,4-bis(benzyloxy)-5-isopropyl-N-(1-methyl-1H-indol-5-yl)benzamide (92)

The title compound was prepared using a mixture of **89** (1.50g, 8.51mmol) dissolved in IPA (68ml) and H₂O (17ml) and similar process as mentioned herein to afford **92** (0.96g, 35.82%) as a white solid. ¹H-NMR (500MHz, CDCl₃): δ 1.28 (d, *J=* 7 Hz, 6H), 3.36 (qui, *J*= 7 Hz, 1H), 3.73 (s, 3H), 5.15 (s, 2H), 5.16 (s, 2H), 6.36 (d, *J=* 3Hz, 1H), 6.62 (s, 1H), 7.02 (m, 2H), 7.11 (d, *J=* 8.5 Hz, 1H), 7.35 (t, *J=* 7 Hz, 1H), 7.46 (m, 10H), 7.70 (d, *J=* 1.5 Hz, 1H), 8.24 (s, 1H), 9.86 (d, 1H).

### 2,4-bis(benzyloxy)-5-isopropyl-N-(1-methyl-1H-indol-6-yl)benzamide (93)

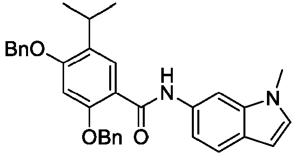

### 2,4-bis(benzyloxy)-5-isopropyl-N-(1-methyl-1H-indol-7-yl)benzamide (93)

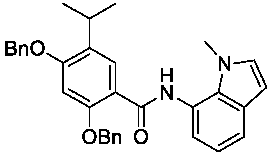

### 2,4-dihydroxy-5-isopropyl-N-(1-methyl-1H-indol-5-yl)benzamide (20f)

A mixture of **92** (0.40g, 0.79mmol), 10% palladium on activated carbon (0.04g, 0.04mmol) and MeOH (8ml) was under the hydrogen gas. The reaction was stirred at room temperature for 2h. The 10% palladium on activated carbon was removed by methyl alcohol wash through celite packing. The organic layer was concentrated *in vacuo* and purified by silica gel chromatography (ethyl acetate: n-hexane = 1: 1, Rf= 0.3) to afford **20f** (0.21g, 80.77%) as a pink solid. m.p. 221.2- 223.8. ¹H-NMR (500MHz, MeOD): δ 1.25 (d, *J=* 7 Hz, 6H), 3.21 (Sep, *J=* 7 Hz, 1H), 3.75 (s, 1H), 6.35 (s, 1H), 6.39 (d, *J=* 3 Hz, 1H), 7.11 (d, *J=* 3 Hz, 1H), 7.27 (m, 1H), 7.31 (d, *J=* 8.5 Hz, 1H), 7.74 (d, *J=* 1.5 Hz, 1H), 7.77 (s, 1H).

### 2,4-dihydroxy-5-isopropyl-N-methyl-N-(1-methyl-1H-indol-5-yl)benzamide (20g)

The title compound was prepared using a mixture of **92** (0.36g, 0.71mmol), NaH (0.04g, 1mmole) and DMF (4ml) and similar process as mentioned herein to afford **20g** (0.18g, 75.0%) as a white solid. m.p. 157.2-158.4. ¹H-NMR (500MHz, MeOD): δ 0.49 (d, *J=* 6.5 Hz, 6H), 2.74 (qui, *J*= 7 Hz, 1H), 3.46 (s, 3H), 3.78 (s, 3H), 6.15 (s, 1H), 6.39 (d, *J=* 8 Hz, 1H), 6.45 (s, 1H), 7.00 (m, 1H), 7.18 (d, *J=* 3 Hz, 1H), 7.35 (d, *J=* 5 Hz, 1H), 7.37 (s, 1H).

### N-ethyl-2,4-dihydroxy-5-isopropyl-N-(1-methyl-1H-indol-5-yl)benzamide (20h)

The title compound was prepared using a mixture of **92** (0.35g, 0.69mmol), NaH (0.04g, 1mmole ) and DMF (4ml) and similar process as mentioned herein to afford **20h** (0.17g, 70.83%) as a white solid. m.p. 167.6-169.8. ¹H-NMR (500MHz, MeOD): δ 0.47 (d, *J=* 6.5 Hz, 6H), 1.19 (t, *J=* 7 Hz, 3H), 2.73 (Sex, *J*= 7 Hz, 1H), 3.74 (s, 3H), 3.93 (q, *J=* 7 Hz, 2H), 6.17 (s, 1H), 6.37 (d, *J=* 3 Hz, 1H), 6.44 (s, 1H), 6.95 (m, 1H), 7.15 (d, *J=* 3 Hz, 1H), 7.31 (m 2H).

### 2,4-dihydroxy-5-isopropyl-N-(1-methyl-1H-indol-5-yl)-N-propylbenzamide (20i)

The title compound was prepared using a mixture of **92** (0.40g, 0.79mmol), NaH (0.04g, 1mmole) and DMF (4ml) and similar process as mentioned herein to afford **20i** (0.2g, 68.96%) as a white solid. m.p. 179.9-182.4. ¹H-NMR (500MHz, MeOD): δ 0.49 (d, *J=* 6.5 Hz, 6H), 0.93 (t, *J=* 7 Hz, 3H), 1.66 (Sex, *J*= 7.5 Hz, 2H), 2.73 (Sex, *J*= 7 Hz, 1H), 3.77 (s, 1H), 3.87 (t, *J=* 7.5 Hz, 2H), 6.15 (s, 1H), 6.38 (d, *J=* 2.5 Hz, 1H), 6.43 (s, 1H), 6.97 (m, 1H), 7.17 (d, *J=* 3 Hz, 1H), 7.33 (m, 2H).

### 2,4-dihydroxy-5-isopropyl-N-(1-methyl-1H-indol-6-yl)benzamide (21b)

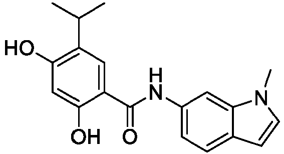

### 2,4-dihydroxy-5-isopropyl-N-methyl-N-(1-methyl-1H-indol-6-yl)benzamide (21c)

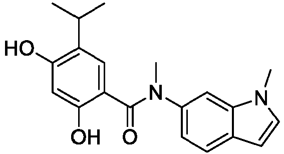

### N-ethyl-2,4-dihydroxy-5-isopropyl-N-(1-methyl-1H-indol-6-yl)benzamide (21d)

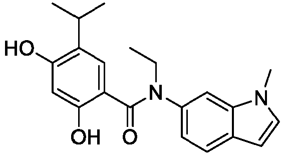

### 2,4-dihydroxy-5-isopropyl-N-(1-methyl-1H-indol-6-yl)-N-propylbenzamide (21e)

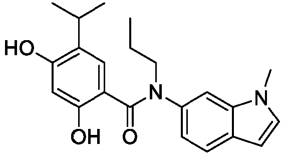

### 2,4-dihydroxy-N,5-diisopropyl-N-(1-methyl-1H-indol-6-yl)benzamide (21f)

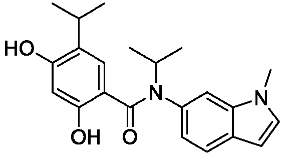

### 2,4-dihydroxy-5-isopropyl-N-(1-methyl-1H-indol-7-yl)benzamide (22a)

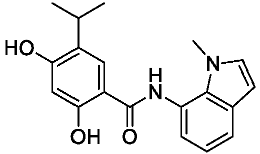

### 2,4-dihydroxy-5-isopropyl-N-methyl-N-(1-methyl-1H-indol-7-yl)benzamide (22b)

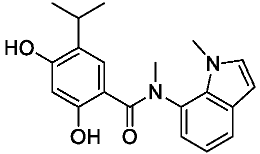

### N-ethyl-2,4-dihydroxy-5-isopropyl-N-(1-methyl-1H-indol-7-yl)benzamide (22c)

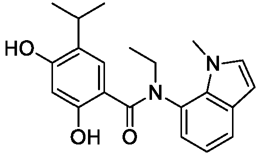

### 2,4-dihydroxy-5-isopropyl-N-(1-methyl-1H-indol-7-yl)-N-propylbenzamide (22d)

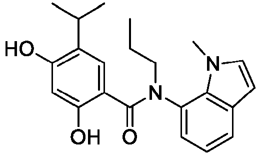

### 2,4-dihydroxy-N,5-diisopropyl-N-(1-methyl-1H-indol-7-yl)benzamide (22e)

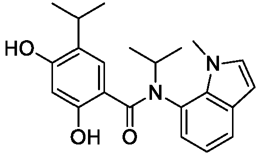

### 1-methyl-5-nitroindoline (96)

A mixture of 5-nitroindoline (2g, 12.33mmol), NaH (0.6g, 15mmole ) and DMF (10ml) was added methyl iodide (1.5ml, 24.09mmol) at room temperature for 2h. The reaction was quenched with water and extracted with ethyl acetate (30ml*3). The organic layer was collected and dried over anhydrous MgSO₄ concentrated *in vacuo* and purified by silica gel chromatography (ethyl acetate: n-hexane = 1: 2, Rf= 0.5) to afford **96** (1.93g, 87.72%) as a orange solid.¹H-NMR (500MHz, CDCl₃): δ 2.90 (s, 3H), 3.05 (t, *J=* 8.5 Hz, 2H), 3.62 (t, *J*= 8.5 Hz, 2H), 6.25 (d, *J=* 9 Hz, 1H), 7.86 (s, 1H), 8.05 (m, 1H).

### 2,4-bis(benzyloxy)-5-isopropyl-N-(1-methylindolin-5-yl)benzamide (97)

A mixture of **96** (1.50g, 8.51mmol) was dissolved in IPA (68ml) and H₂O (17ml) and added iron powder (2.5g, 45.45mmol) and NH₄Cl (2g, 37.04mmol). The reaction was stirred and reflux for 1h. The iron powder was removed by methyl alcohol wash through celite packing. The organic layer was concentrated *in vacuo* and without more purification. To the mixture of **46** (2g, 5.31mmol), EDC.HCl (1.5g, 7.85mmol), HOBt (0.9g, 6.67mmol), NMM (1.4ml, 12.75mmol) and DMF (8ml) was stirred for 10 min then added crude product at room temperature for overnight, the reaction was quenched with water and extracted with ethyl acetate (30ml*3). The organic layer was collected and dried over anhydrous MgSO₄ concentrated *in vacuo* to yield colorless oil product. The residue was purified by silica gel chromatography (ethyl acetate: n-hexane = 1: 2, Rf= 0.6) to afford **97** (1.24g, 45.93%) as a yellow solid. ¹H-NMR (500MHz, CDCl₃): δ 1.26 (d, *J=* 7 Hz, 6H), 2.71 (s, 3H), 2.86 (t, *J=* 7 Hz, 2H), 3.24 (s, 2H), 3.34 (Sep, *J=* 7 Hz, 1H), 5.13 (s, 2H), 5.14 (s, 2H), 6.32 (d, *J=* 8 Hz, 1H), 6.60 (s, 1H), 6.89 (d, *J=* 7.5 Hz, 1H), 7.16 (s, 1H), 7.44 (m, 10H), 8.20 (s, 1H), 9.68 (s, 1H).

### 2,4-dihydroxy-5-isopropyl-N-(1-methylindolin-5-yl)benzamide (23f)

A mixture of **97** (0.32g, 0.63mmol), 10% palladium on activated carbon (0.04g, 0.04mmol) and MeOH (8ml) was under the hydrogen gas. The reaction was stirred at room temperature for 2h. The 10% palladium on activated carbon was removed by methyl alcohol wash through celite packing. The organic layer was concentrated *in vacuo* and purified by silica gel chromatography (ethyl acetate: n-hexane = 1: 2, Rf= 0.3) to afford **23f** (0.17g, 80.95%) as a yellow oil. ¹H-NMR (500MHz, CDCl₃): δ 1.25 (d, *J=* 7 Hz, 6H), 2.75 (s, 3H), 2.96 (d, *J=* 5 Hz, 2H), 3.16 (qui, *J*= 7 Hz, 1H), 3.31 (s, 2H), 6.34 (s, 1H), 6.45 (d, *J=* 8 Hz, 1H), 7.10 (d, *J=* 8 Hz, 1H), 7.21 (s, 1H), 7.26 (m, 1H), 7.67 (s, 1H).

### N-ethyl-2,4-dihydroxy-5-isopropyl-N-(1-methylindolin-5-yl)benzamide (23g)

The title compound was prepared using a mixture of **97** (0.35g, 0.69mmol), NaH (0.04g, 1mmole ) and DMF (4ml) and similar process as mentioned herein to afford **23g** (0.17g, 70.83%) as a white solid. m.p. 136.5-138.3. ¹H-NMR (500MHz, MeOD): δ 0.78 (d, *J=* 6.5 Hz, 6H), 1.16 (t, *J=* 7 Hz, 3H), 2.70 (s, 3H), 2.85 (t, *J=* 8 Hz, 2H), 2.91 (qui, *J*= 7 Hz, 1H), 3.25 (t, *J=* 8 Hz, 2H), 3.82 (q, *J=* 7 Hz, 2H), 6.18 (s, 1H), 6.42 (d, *J=* 8.5 Hz, 1H), 6.63 (s, 1H), 6.77 (m, 1H), 6.84 (s, 1H).

### 2,4-dihydroxy-5-isopropyl-N-(1-methylindolin-5-yl)-N-propylbenzamide (23h)

The title compound was prepared using a mixture of **97** (0.33g, 0.65mmol), NaH (0.04g, 1mmole) and DMF (4ml) and similar process as mentioned herein to afford **23h** (0.16g, 66.67%) as a white solid. m.p. 132.0-133.9. ¹H-NMR (500MHz, MeOD): δ 0.79 (d, *J=* 7 Hz, 6H), 0.91 (t, *J=* 7.5 Hz, 3H), 1.62 (Sex, *J*= 7.5 Hz, 2H), 2.71 (s, 3H), 2.85 (t, *J=* 8 Hz, 2H), 2.91 (qui, *J*= 7 Hz, 1H), 3.26 (t, *J=* 8 Hz, 2H), 3.74 (m, 2H), 6.17 (s, 1H), 6.42 (d, *J=* 8.5 Hz, 1H), 6.62 (s, 1H), 6.78 (m, 1H), 6.85 (s, 1H).

### 5-nitro-1H-pyrrolo[2,3-b]pyridine (99)

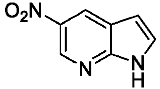

### 1-methyl-5-nitro-1H-pyrrolo[2,3-b]pyridine (100)

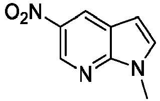

### 2,4-bis(benzyloxy)-5-isopropyl-N-(1H-pyrrolo[2,3-b]pyridin-5-yl)benzamide (101)

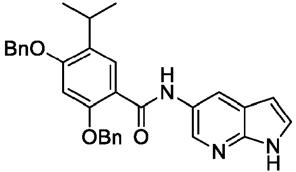

### 2,4-bis(benzyloxy)-5-isopropyl-N-(1-methyl-1H-pyrrolo[2,3-b]pyridin-5-yl)benzamide (102)

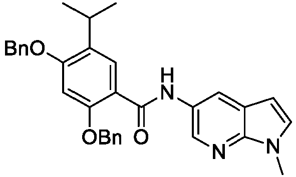

### 2,4-dihydroxy-5-isopropyl-N-(1H-pyrrolo[2,3-b]pyridin-5-yl)benzamide (24a)

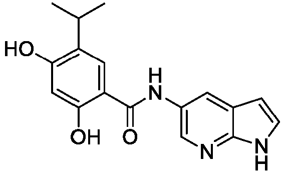

### 2,4-dihydroxy-5-isopropyl-N-(1-methyl-1H-pyrrolo[2,3-b]pyridin-5-yl)benzamide (24b)

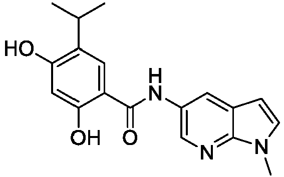

### 2,4-dihydroxy-5-isopropyl-N-methyl-N-(1-methyl-1H-pyrrolo[2,3-b]pyridin-5-yl)benzamide (24c)

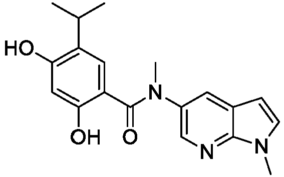

### N-ethyl-2,4-dihydroxy-5-isopropyl-N-(1-methyl-1H-pyrrolo[2,3-b]pyridin-5-yl)benzamide (24d)

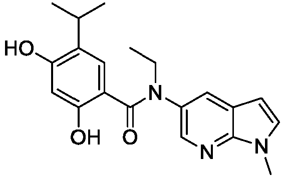

### 2,4-dihydroxy-5-isopropyl-N-(1-methyl-1H-pyrrolo[2,3-b]pyridin-5-yl)-N-propylbenzamide (24e)

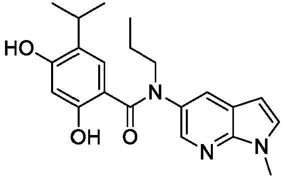

### 2,4-dihydroxy-N,5-diisopropyl-N-(1-methyl-1H-pyrrolo[2,3-b]pyridin-5-yl)benzamide (24f)

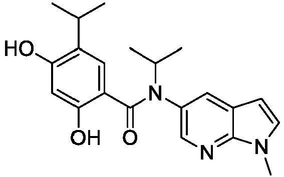

### 2,4-bis(benzyloxy)-N-(1H-indazol-5-yl)-5-isopropylbenzamide (104)

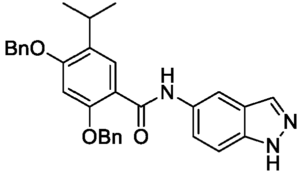

### 2,4-dihydroxy-N-(1H-indazol-5-yl)-5-isopropylbenzamide (25a)

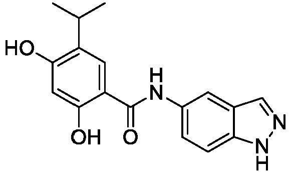

### 2,4-dihydroxy-5-isopropyl-N-methyl-N-(1-methyl-1H-indazol-5-yl)benzamide (25b)

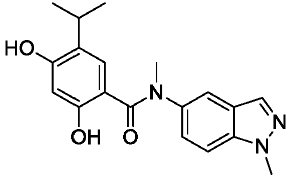

### 5-nitro-1H-pyrazolo[3,4-b]pyridine (106)

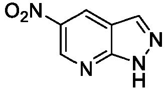

### 1-methyl-5-nitro-1H-pyrazolo[3,4-b]pyridine (107)

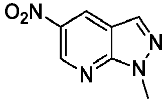

### 2,4-bis(benzyloxy)-5-isopropyl-N-(1H-pyrazolo[3,4-b]pyridin-5-yl)benzamide (108)

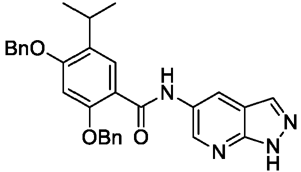

### 2,4-bis(benzyloxy)-5-isopropyl-N-(1-methyl-1H-pyrazolo[3,4-b]pyridin-5-yl)benzamide (109)

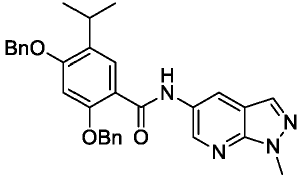

### 2,4-dihydroxy-5-isopropyl-N-(1H-pyrazolo[3,4-b]pyridin-5-yl)benzamide (26a)

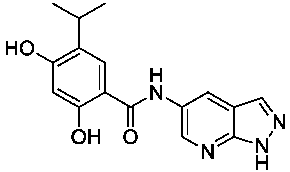

### 2,4-dihydroxy-5-isopropyl-N-(1-methyl-1H-pyrazolo[3,4-b]pyridin-5-yl)benzamide (26b)

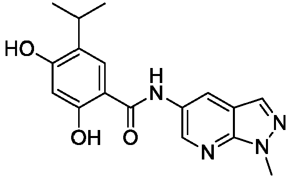

### 2,4-dihydroxy-5-isopropyl-N-methyl-N-(1-methyl-1H-pyrazolo[3,4-b]pyridin-5-yl)benzamide (26c)

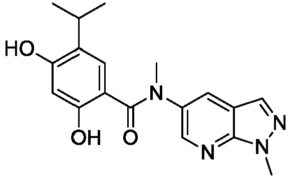

### N-ethyl-2,4-dihydroxy-5-isopropyl-N-(1-methyl-1H-pyrazolo[3,4-b]pyridin-5-yl)benzamide (26d)

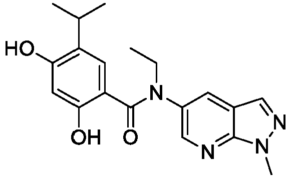

### 2,4-dihydroxy-5-isopropyl-N-(1-methyl-1H-pyrazolo[3,4-b]pyridin-5-yl)-N-propylbenzamide (26e)

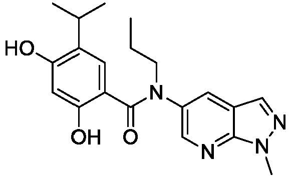

### 2,4-dihydroxy-N,5-diisopropyl-N-(1-methyl-1H-pyrazolo[3,4-b]pyridin-5-yl)benzamide (26f)

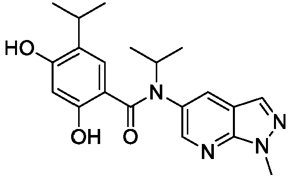

### 7-methyl-7H-pyrrolo[2,3-d]pyrimidin-4-amine (111)

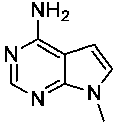

### 2,4-bis(benzyloxy)-5-isopropyl-N-(7-methyl-7H-pyrrolo[2,3-d]pyrimidin-4-yl)benzamide (112)

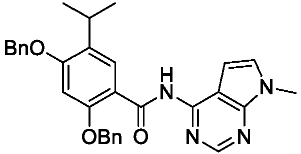

### 2,4-dihydroxy-5-isopropyl-N-(7-methyl-7H-pyrrolo[2,3-d]pyrimidin-4-yl)benzamide (27a)

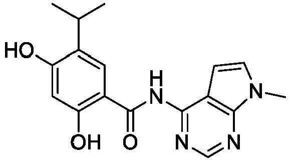

### 2,4-dihydroxy-5-isopropyl-N-methyl-N-(7-methyl-7H-pyrrolo[2,3-d]pyrimidin-4-yl)benzamide (27b)

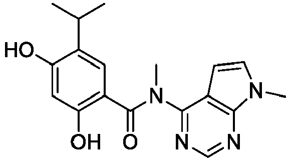

### N-ethyl-2,4-dihydroxy-5-isopropyl-N-(7-methyl-7H-pyrrolo[2,3-d]pyrimidin-4-yl)benzamide (27c)

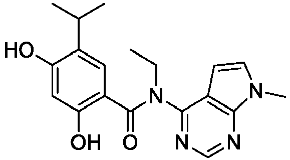

### 2,4-dihydroxy-5-isopropyl-N-(7-methyl-7H-pyrrolo[2,3-d]pyrimidin-4-yl)-N-propylbenzamide (27d)

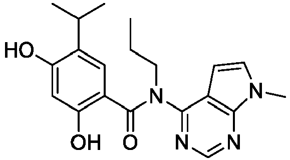

### 2,4-dihydroxy-N,5-diisopropyl-N-(7-methyl-7H-pyrrolo[2,3-d]pyrimidin-4-yl)benzamide (27e)

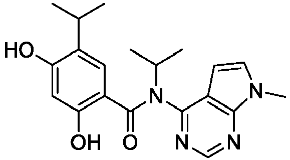

### 2,4-bis(benzyloxy)-5-isopropyl-N-(quinolin-3-yl)benzamide (113)

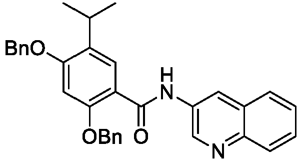

The title compound was prepared using a mixture of **47** (1.28g, 3.40mmol), EDC.HCl (0.97g, 5.06mmol), HOBt (0.55g, 4.07mmol), NMM (0.9ml, 8.19mmol) and DMF (5ml) and similar process as mentioned herein to afford **113** (1.03g, 61%). ¹H-NMR (300MHz, CDCl₃): δ 1.29 (d, *J=* 6.9 Hz, 6H), 3.37 (qui, *J*= 6.9 Hz, 1H), 5.19 (s, 2H), 5.20 (s, 2H), 7.35-7.64 (m, 13H), 7.74-7.78 (m, 1H), 8.00-8.15 (m, 2H), 8.22 (s, 1H), 8.83 (d, J =2.4 Hz, 1H), 10.22 (s, 1H).

### 2,4-bis(benzyloxy)-5-isopropyl-N-(quinolin-5-yl)benzamide (114)

A mixture of **47** (1.28g, 3.40mmol), EDC.HCl (0.97g, 5.06mmol), 4-dimethyl-aminopyridine (0.42 g, 3.40 mmol) and DCM (5ml) was stirred for 10 min then added 6-aminoquinoline (0.49 g, 3.40 mmol) for overnight, the reaction was quenched with water and extracted with DCM (30ml*3). The organic layer was collected and dried over anhydrous MgSO₄ concentrated *in vacuo* to yield crude product. The residue was purified by silica gel chromatography (ethyl acetate: n-hexane = 1: 2) to afford **114** (0.93g, 54%). ¹H-NMR (300 MHz, CDCl₃): δ 1.29 (d, *J*=6.6 Hz, 6H), 3.38 (qui, *J*= 6.9 Hz, 1H), 5.20 (s, 2H), 5.22 (s, 2H), 6.72 (s, 1H), 7.00-7.07 (m, 1H), 7.34-7.50 (m, 11H), 7.69-7.90 (m, 2H), 8.09 (d, *J=* 8.4 Hz, 1H), 8.25 (s, 1H), 8.34 (d, *J=* 7.8 Hz, 1H), 8.8 (d, *J=* 4.2 Hz, 1H).

### 2,4-bis(benzyloxy)-5-isopropyl-N-(quinolin-6-yl)benzamide (115)

The title compound was prepared using a mixture of **47** (1.28g, 3.40mmol), EDC.HCl (0.97g, 5.06mmol), 4-dimethyl-aminopyridine (0.42 g, 3.40 mmol) and DCM (5ml) and similar process as mentioned herein to afford **115** (0.91g, 53%). ¹H-NMR (300 MHz, CDCl₃): δ 1.31 (d, *J=* 6.9 Hz, 6H), 3.38 (qui, *J*=6.9 Hz, 1H), 5.16 (s, 4H), 6.63 (s, 1H), 6.96 (dd, *J=* 6.6, 9 Hz, 1H), 7.24-7.60 (m, 12H), 7.86 (d, *J=* 9 Hz, 1H), 8.01 (d, *J=* 8.1 Hz, 1H), 8.25-8.30 (m, 2H), 8.75 (dd, *J=* 2.4, 3.9 Hz, 1H), 10.14 (s, 1H).

### 2,4-bis(benzyloxy)-5-isopropyl-N-(quinolin-8-yl)benzamide (116)

The title compound was prepared using a mixture of **47** (1.28g, 7.97mmol), EDC.HCl (0.97g, 5.06mmol), HOBt (0.55g, 4.07mmol), NMM (0.9ml, 8.19mmol) and DMF (5ml) and similar process as mentioned herein to afford **116** (0.78g, 45.64%) as a white solid. ¹H-NMR (300 MHz, CDCl₃): δ 1.20 (d, *J=* 6.6 Hz, 6H), 3.25 (qui, *J*= 6.9 Hz, 1H), 4.90 (s, 2H), 5.30 (s, 2H), 6.44 (s, 1H), 7.13-8.19 (m, 11H), 8.33 (m, 1H), 8.34 (s, 1H), 8.99 (d, *J*= 0.9 Hz, 1H), 9.02 (d, *J=* 0.9 Hz, 1H), 12.25 (s,1H).

### 2,4-bis(benzyloxy)-5-isopropyl-N-(2-methylquinolin-4-yl)benzamide (117)

The title compound was prepared using a mixture of **47** (1.42g, 3.79mmol), EDC.HCl (1.09g, 5.69mmol), HOBt (0.55g, 4.07mmol), NMM (1.0ml, 9.10mmol) and DMF (5ml) and similar process as mentioned herein to afford **117** (0.97g, 49%). ¹H-NMR (300MHz, CDCl₃): δ 1.27 (d, *J=* 6.9 Hz, 6H), 2.68 (s, 3H), 3.34-3.39 (m, 1H), 5.27 (s, 2H), 5.42 (s, 2H), 7.00-7.10 (m, 2H), 7.26-7.62 (m, 13H), 7.86 (d, *J=* 8.4 Hz, 1H), 8.11 (s, 1H), 8.39 (s, 1H).

### 2,4-dihydroxy-5-isopropyl-N-(quinolin-3-yl)benzamide (28a)

A mixture of **113** (0.1 g, 0.19 mmol) in ethanol (10 mL) was added 10% palladium on activated carbon (cat.). The mixture was stirred under H₂ for 6 hr. The reaction was filtered through celite packing and the organic layer was concentrated *in vacuo* and purified by silica gel chromatography (EtOAc: n-hexane= 1: 1) to afford **28a** (0.10g, 31%). m.p. 232.0-234.5. ¹H-NMR (300MHz, DMSO): δ 1.20 (d, *J=* 6.9 Hz, 6H), 3.13 (qui, *J*= 6.9 Hz, 1H), 6.43 (s, 1H), 7.55-7.72 (m, 2H), 7.82 (s, 1H), 7.93-8.01 (m, 2H), 8.70 (d, *J=* 2.4 Hz, 1H), 9.06 (d, *J*= 2.7 Hz, 1H), 10.22 (s, 1H), 10.58 (s, 1H).

### 2,4-dihydroxy-5-isopropyl-N-methyl-N-(quinolin-3-yl)benzamide (28b)

A mixture of **113** (1.00g, 1.98 mmol) dissolved in acetonitrile (5mL) was added cesium carbonate (0.65g, 1.98 mmol). The mixture was heated at 60~70⁰C and stirred for 30 min. The reaction was quenched with water and extracted with ethyl acetate (30ml*3). The organic layer was collected and dried over anhydrous MgSO₄ concentrated *in vacuo* to yield colorless oil product. The residue was purified by silica gel chromatography (ethyl acetate: n-hexane = 1: 4) to afford product. To a solution of this product was dissolved in ethanol (10 mL) and stirred under H₂ at room temperature for 3 hr. The reaction was filtered through celite packing and the organic layer was concentrated *in vacuo* and purified by silica gel chromatography (EtOAc: n-hexane= 1: 4) to afford **28b** (0.04g, 79%). m.p. 236.9-238.6. ¹H-NMR (300MHz, d-Acetone): δ 1.27 (d, *J=* 7.2 Hz, 10H), 3.26 (qui, *J*= 7.2 Hz, 1H), 3.84 (s, 3H), 6.53 (s, 1H), 7.29-7.37 (m, 1H), 7.55-7.59 (m, 2H), 7.70-7.77 (m, 2H), 8.82 (s, 1H), 9.20 (s, 1H), 10.20 (s, 1H), 11.35 (s, 1H).

### N-ethyl-2,4-dihydroxy-5-isopropyl-N-(quinolin-3-yl)benzamide (28c)

The title compound was prepared using a mixture of **113** (0.5 g, 1 mmol), NaH (0.05g, 2mmole) and DMF (5ml) and similar process as mentioned herein to afford **28c** (0.10g, 73 %). m.p. 207.2-209.8. ¹H-NMR (500MHz, MeOD): δ 0.70 (d, *J=* 6.9 Hz, 6H), 1.26 (t, *J=* 7.2 Hz, 3H), 2.80-2.94 (m, 1H), 4.04-4.14 (m, 2H), 6.11 (s, 1H), 6.67(s, 1H), 7.57-7.65 (m, 1H), 7.70-7.79 (m, 1H), 7.88 (d, *J=* 8.4 Hz, 1H), 7.96 (d, *J*= 8.7Hz, 1H), 8.21 (d, *J=* 2.4 Hz, 1H), 8.55 (d, *J=* 2.4 Hz, 1H).

### 2,4-dihydroxy-5-isopropyl-N-propyl-N-(quinolin-3-yl)benzamide (28d)

The title compound was prepared using a mixture of compound **113** (0.35 g, 0.90 mmol) in tetrahydrofuran (5 mL) and potassium tert-butoxide (0.15g, 1.35mmol) and similar process as mentioned herein to afford **28d** (0.04 g, 13%). m.p. 197.8-199.5. ¹H-NMR (300MHz, CDCl₃): δ 0.42 (d, *J=* 6.9 Hz, 6H), 0.96 (t, *J=* 7.2 Hz, 3H), 1.65-1.79 (m, 2H), 2.75 (qui, *J*= 6.9 Hz, 1H), 3.93-4.01 (m, 2H), 6.29 (s, 1H), 6.37 (s, 1H), 7.53-7.63 (m, 1H), 7.67-7.81 (m, 2H), 7.96 (d, *J=* 2.4 Hz, 1H), 8.08 (d, *J=* 8.4 Hz, 1H), 8.58 (d, *J=* 2.7 Hz, 1H), 10.93 (s, 1H).

### 2,4-dihydroxy-5-isopropyl-N-(quinolin-5-yl)benzamide (29a)

A mixture of **114** (0.2 g, 0.40 mmol) in methanol (15 mL) was added 10% palladium on activated carbon (cat.) and formic acid (0.9 mL, 23.85mmol). The mixture was stirred under H₂ for 12 hr. The reaction was filtered through celite packing and the organic layer was concentrated *in vacuo* and purified by silica gel chromatography (EtOAc: n-hexane= 1: 1) to afford **29a** (0.06g, 45%). m.p. 222.0-224.5. ¹H-NMR (300MHz, MeOD): δ 1.26 (d, *J=* 7.2 Hz, 7H), 3.18-3.24 (m, 1H), 6.4 (s, 1H), 7.56-7.63 (m, 1H), 7.78-7.99 (m, 4H), 8.48 (d, *J*= 9 Hz, 1H), 8.88 (d, *J=* 3.9 Hz, 1H).

### 2,4-dihydroxy-5-isopropyl-N-methyl-N-(quinolin-5-yl)benzamide (29b)

The title compound was prepared using a mixture of **29a** (0.10 g, 0.31 mmol) in DCM (5 mL), tert-butyldimethylsilyl chloride (0.09 g, 0.62mmol) and DIPEA (0.08 g, 0.62 mmol) and similar process as mentioned hereinto afford **29b** (0.02 g, 71%). ¹H-NMR (300MHz, MeOD): δ 1.22-1.30 (m, 9H), 3.20-3.28 (m, 1H), 4.09 (s, 3H), 6.63 (s, 1H), 7.63 (q, *J*= 4.5 Hz, 1H), 7.84 (m, 1H), 7.95 (m, 2H), 8.06 (d, *J=* 7.5 Hz, 1H), 8.48 (dd, *J=* 1.2, 8.4Hz, 1H), 8.87-8.91 (m, 1H).

### N-ethyl-2,4-dihydroxy-5-isopropyl-N-(quinolin-5-yl)benzamide (29c)

The title compound was prepared using a mixture of **29a** (0.10 g, 0.31 mmol) in DCM (5 mL), tert-butyldimethylsilyl chloride (0.09 g, 0.62mmol) and DIPEA (0.08 g, 0.62 mmol) and similar process as mentioned herein to afford **29c** (0.02 g, 33%). m.p. 152.3-154.9. ¹H-NMR (300MHz, MeOD): δ 1.24 (d, *J=* 6.9 Hz, 6H), 1.57 (t, *J=* 6.9 Hz, 3H), 3.16-3.28 (m, 1H), 4.33 (q, *J=* 6.9 Hz, 2H), 6.61 (s, 1H), 7.74-7.81 (m, 1H), 7.89-8.01 (m, 3H), 8.15 (d, *J=* 6.9 Hz, 1H), 8.76 (d, *J=* 8.4 Hz, 1H), 9.00 (s, 1H).

### 2,4-dihydroxy-5-isopropyl-N-(quinolin-6-yl)benzamide (30a)

A mixture of **115** (0.1 g, 0.19 mmol) in methanol (10 mL) was added 10% palladium on activated carbon (cat.). The mixture was stirred under H₂ for 12 hr. The reaction was filtered through celite packing and the organic layer was concentrated *in vacuo* and purified by silica gel chromatography (EtOAc: n-hexane= 1: 1) to afford **30a** (0.04g, 63%). m.p. 162.1-164.2. ¹H-NMR (300MHz, DMSO): δ 1.20 (d, *J=* 6.9 Hz, 6H), 3.14 (qui, *J*= 6.9 Hz, 1H), 6.43 (s, 1H), 7.50 (q, *J =* 4.2 Hz, 1H), 7.82 (s, 1H), 7.92-8.05 (m, 2H), 8.28-8.42 (m, 2H), 8.78-8.84 (m, 1H), 10.18 (s, 1H), 10.46 (s, 1H).

### 2,4-dihydroxy-5-isopropyl-N-methyl-N-(quinolin-6-yl)benzamide (30b)

The title compound was prepared using a mixture of **30a** (0.61 g, 1.89 mmol) in DCM (15 mL), tert-butyldimethylsilyl chloride (0.57 g, 3.78mmol) and DIPEA (0.49 g, 3.78 mmol) and similar process as mentioned herein to afford **30b** (0.1 g, 40%). m.p. 277.5-279.8. ¹H-NMR (300MHz, DMSO): δ 1.17 (d, *J=* 6.9 Hz, 6H), 3.10-3.22 (m, 1H), 3.93 (s, 3H), 6.60 (s, 1H), 7.46-7.53 (m, 1H), 7.69 (s, 1H), 7.93-7.99 (m, 2H), 8.28 (dd, *J=* 1.5, 8.4 Hz, 1H), 8.51 (d, *J=* 1.5 Hz, 1H), 8.76-8.82 (m, 1H), 10.13 (s, 1H).

### N-ethyl-2,4-dihydroxy-5-isopropyl-N-(quinolin-6-yl)benzamide (30c)

The title compound was prepared using a mixture of **115** (0.40 g, 0.80 mmol) dissolved in tetrahydrofuran (5 ml), potassium tert-butoxide (0.13 g, 1.20 mmol) and ethyl iodide (3.30 mL) and similar process as mentioned herein to afford **30c** (0.09g, 38%). m.p. 220.8-221.2. ¹H-NMR (300MHz, DMSO): δ 0.57 (d, *J=* 6.6 Hz, 6H), 1.14 (t, *J=* 6.9 Hz, 3H), 2.71-2.77 (m, 1H), 3.93 (q, *J=* 6.9 Hz, 2H), 6.12 (s, 1H), 6.53 (s, 1H), 7.49 (q, *J=* 4.2 Hz, 1H), 7.58 (dd, *J=* 2.4, 9 Hz, 1H), 7.76 (d, *J=* 2.4 Hz, 1H), 7.94 (d, *J=* 9 Hz, 1H), 8.23-8.28 (m, 1H), 8.83-8.87 (m, 1H).

### 2,4-dihydroxy-5-isopropyl-N-propyl-N-(quinolin-6-yl)benzamide (30d)

A mixture of **30a** (0.61 g, 1.89 mmol) in DCM (15 mL) was added tert-butyldimethylsilyl chloride (0.57 g, 3.78mmol) and DIPEA (0.49 g, 3.78 mmol). The mixture was stirred at room temperature for 12 hr. The reaction was quenched with water and extracted with DCM (30ml*3). The organic layer was collected and dried over anhydrous MgSO₄ concentrated *in vacuo* and purified by silica gel chromatography (ethyl acetate: n-hexane = 1: 2) to afford product. Then this product was dissolved in tetrahydrofuran (5 mL) and added potassium tert-butoxide (0.06 g, 0.51 mmol) and 1-iodopropane (1.65 mL) to the reaction solution and stirred at room temperature for 12 hours. The reaction was quenched with water and extracted with DCM (30ml*3). The organic layer was collected and dried over anhydrous MgSO₄ concentrated *in vacuo* and purified by silica gel chromatography (ethyl acetate: n-hexane = 1: 1) to afford product. Then this product was dissolved in DCM (3 ml) and added tetra-n-butylammonium fluoride (0.15 ml). The reaction solution was stirred at room temperature for 2hr. The reaction was quenched with water and extracted with DCM (30ml*3). The organic layer was collected and dried over anhydrous MgSO₄ concentrated *in vacuo* and purified by silica gel chromatography (ethyl acetate: n-hexane = 1: 1) to afford **30d** (0.01 g, 13%). ¹H-NMR (300MHz, DMSO): δ 1.03 (t, *J=* 7.5 Hz, 3H), 1.17 (d, *J=* 6.9 Hz, 7H), 1.87-1.99 (m, 2H), 3.15 (t, *J=* 6.9 Hz, 1H), 4.07 (t, *J=* 6.6 Hz, 2H), 6.59 (s, 1H), 7.49 (q, *J=* 4.2 Hz, 1H), 7.70 (s, 1H), 7.80 (dd, *J=* 2.4, 9 Hz, 1H), 8.00 (d, *J=* 9 Hz, 1H), 8.29 (d, *J=* 6.6 Hz, 1H), 8.52 (d, *J=* 2.4 Hz, 1H), 8.76-8.81 (m, 1H).

### 2,4-dihydroxy-5-isopropyl-N-(quinolin-8-yl)benzamide (31a)

A mixture of **116** (0.21g, 0.42mmol), 10% palladium on activated carbon (cat.) and MeOH (10ml) was under the hydrogen gas. The reaction was stirred at room temperature for 12h. The 10% palladium on activated carbon was removed by methyl alcohol wash through celite packing. The organic layer was concentrated *in vacuo* and purified by silica gel chromatography (ethyl acetate: n-hexane = 1:2, Rf= 0.4) to afford **31a** (0.07g, 51.70%) as a yellow solid. m.p. 172.5-174.6. ¹H-NMR (300MHz, MeOD): δ 1.27 (d, *J=* 6.9 Hz, 6H), 3.23 (qui, *J*= 6.9 Hz, 1H), 6.41 (s, 1H), 7.49-7.60 (m, 3H), 7.81 (s, 1H), 8.26 (dd, *J=* 1.8, 8.4 Hz, 1H), 8.80 (dd, *J=* 2.1, 6.9 Hz, 1H), 8.85 (dd, *J*=1.5, 4.2 Hz, 1H).

### 2,4-dihydroxy-5-isopropyl-N-methyl-N-(quinolin-8-yl)benzamide (31b)

A mixture of **31a** (0.21 g, 0.65 mmol) in DCM (10 mL) was added tert-butyldimethylsilyl chloride (0.20 g, 1.30mmol) and DIPEA (0.18 g, 1.30 mmol). The mixture was stirred at room temperature for 5 hr. The reaction was quenched with water and extracted with DCM (30ml*3). The organic layer was collected and dried over anhydrous MgSO₄ concentrated *in vacuo* and purified by silica gel chromatography (ethyl acetate: n-hexane = 1: 3) to afford product. Then this product was dissolved in tetrahydrofuran (5 ml) and added potassium tert-butoxide (0.03 g, 0.25 mmol) and methyl iodide (0.51 ml) to the reaction solution and stirred at room temperature for 4 hours. The reaction was quenched with water and extracted with DCM (30ml*3). The organic layer was collected and dried over anhydrous MgSO₄ concentrated *in vacuo* and purified by silica gel chromatography (ethyl acetate: n-hexane = 1: 8) to afford product. Then this product was dissolved in DCM (3 ml) and added tetra-n-butylammonium fluoride (0.11 ml). The reaction solution was stirred at room temperature for 3hr. The reaction was quenched with water and extracted with DCM (30ml*3). The organic layer was collected and dried over anhydrous MgSO₄ concentrated *in vacuo* and purified by silica gel chromatography (ethyl acetate: n-hexane = 1: 1) to afford **31b** (0.02 g, 9%). m.p. 229.5-231.5. ¹H-NMR (300MHz, d-Acetone): δ 1.27 (d, *J=* 6.9 Hz, 6H), 3.03 (qui, *J*= 6.9 Hz, 1H), 4.17 (s, 3H), 6.73 (s, 1H), 7.56-7.62 (m, 3H), 8.15 (s, 1H), 8.34 (dd, *J*= 1.5, 8.1 Hz, 1H), 8.96-9.07 (m, 2H), 12.33 (s, 1H).

### N-ethyl-2,4-dihydroxy-5-isopropyl-N-(quinolin-8-yl)benzamide (31c)

A mixture of compound **116** (0.35 g, 0.69 mmol) in tetrahydrofuran (5 mL) was added potassium tert-butoxide (0.12 g, 1.03 mmol). The mixture was stirred for 10min and added ethyl iodide (2.85 mL) and stirred at room temperature for 6 hours. The reaction was quenched with water and extracted with ethyl acetate (30ml*3). The organic layer was collected and dried over anhydrous MgSO₄ concentrated *in vacuo* and purified by silica gel chromatography (ethyl acetate: n-hexane = 1: 3) to afford product. A mixture of above product in ethanol was added formic acid (0.68 mL), 10% palladium on activated carbon (cat.). The resulting mixture was refluxed for 12hr. After cooling to room temperature, The organic layer was concentrated *in vacuo* and purified by silica gel chromatography (ethyl acetate: n-hexane = 1:2) to afford **31c** (0.07 g, 66%). m.p. 82.5-85.9. ¹H-NMR (300MHz, CDCl₃): δ 1.21 (qui, *J*= 7.2 Hz, 6H), 2.03 (s, 3H), 2.66 (t, *J=* 6.9 Hz, 1H), 4.10 (q, *J=* 7.2 Hz, 1H), 6.20 (s, 1H), 6.40 (s, 1H), 6.46 (s, 1H), 7.25-7.50 (m, 3H), 7.75 (dd, *J=* 1.8, 7.8 Hz, 1H), 8.19 (dd, *J*= 1.8, 8.4 Hz, 1H), 8.96 (dd, *J*= 1.8, 4.2 Hz, 1H), 11.23 (s, 1H).

### 2,4-dihydroxy-5-isopropyl-N-(2-methylquinolin-4-yl)benzamide (32a)

A mixture of **117** (0.73g, 1.41mmol), 10% palladium on activated carbon (cat.) and MeOH (105ml) was added formic acid (3.2 ml, 84.81mmol). The reaction was stirred at room temperature for 12h. The 10% palladium on activated carbon was removed by methyl alcohol wash through celite packing. The organic layer was concentrated *in vacuo* and purified by silica gel chromatography (ethyl acetate: n-hexane = 2:1) to afford **32a** (0.15g, 31%). m.p. 216.3-218.6. ¹H-NMR (300MHz, MeOD): δ 1.23-1.28 (m, 6H), 2.70 (s, 3H), 3.21 (qui, *J*= 6.9 Hz, 1H), 6.45 (d, *J=* 1.5 Hz, 1H), 7.54-7.62 (m, 1H), 7.71-7.79 (m, 1H), 7.94 (d, *J=* 9 Hz, 2H), 8.06 (d, *J=* 8.4 Hz, 1H), 8.42-8.47 (m, 1H).

### 2,4-dihydroxy-5-isopropyl-N-methyl-N-(2-methylquinolin-4-yl)benzamide (32b)

A mixture of **32a** (0.15 g, 0.44 mmol) in DCM (5 mL) was added tert-butyldimethylsilyl chloride (0.13 g, 0.89mmol) and DIPEA (0.12 g, 0.89 mmol). The mixture was stirred at room temperature for 6 hr. The reaction was quenched with water and extracted with DCM (30ml*3). The organic layer was collected and dried over anhydrous MgSO₄ concentrated *in vacuo* and purified by silica gel chromatography (ethyl acetate: n-hexane = 1: 1) to afford product. Then this product was dissolved in tetrahydrofuran (5 ml) and added potassium tert-butoxide (0.04 g, 0.36 mmol) and methyl iodide (0.77 ml) to the reaction solution and stirred at room temperature for 5 hours. The reaction was quenched with water and extracted with DCM (30ml*3). The organic layer was collected and dried over anhydrous MgSO₄ concentrated *in vacuo* and purified by silica gel chromatography (ethyl acetate: n-hexane = 1: 1) to afford product. Then this product was dissolved in DCM (3 ml) and added tetra-n-butylammonium fluoride (0.09 ml). The reaction solution was stirred at room temperature for 3hr. The reaction was quenched with water and extracted with DCM (30ml*3). The organic layer was collected and dried over anhydrous MgSO₄ concentrated *in vacuo* and purified by silica gel chromatography (ethyl acetate: n-hexane = 1: 1) to afford **32b** (0.02 g, 27%). ¹H-NMR (300MHz, MeOD): δ 1.25 (d, *J=* 6.9 Hz, 7H), 2.67 (s, 3H), 3.22-3.26 (m, 1H), 4.13 (s, 3H), 6.60 (s, 1H), 7.58-7.63 (m, 1H), 7.71-7.76 (m, 1H), 7.87-7.92 (m, 2H), 8.01 (s, 1H), 8.40 (s, 1H).

### N-ethyl-2,4-dihydroxy-5-isopropyl-N-(2-methylquinolin-4-yl)benzamide (32c)

A mixture of **32a** (0.15 g, 0.44 mmol) in DCM (5 mL) was added tert-butyldimethylsilyl chloride (0.13 g, 0.89mmol) and DIPEA (0.12 g, 0.89 mmol). The mixture was stirred at room temperature for 6 hr. The reaction was quenched with water and extracted with DCM (30ml*3). The organic layer was collected and dried over anhydrous MgSO₄ concentrated *in vacuo* and purified by silica gel chromatography (ethyl acetate: n-hexane = 1: 1) to afford product. Then this product was dissolved in tetrahydrofuran (5 ml) and added potassium tert-butoxide (0.02 g, 0.17 mmol) and ethyl iodide (0.77 ml) to the reaction solution and stirred at room temperature for 6 hours. The reaction was quenched with water and extracted with DCM (30ml*3). The organic layer was collected and dried over anhydrous MgSO₄ concentrated *in vacuo* and purified by silica gel chromatography (ethyl acetate: n-hexane = 1: 1) to afford product. Then this product was dissolved in DCM (3 ml) and added tetra-n-butylammonium fluoride (0.09 ml). The reaction solution was stirred at room temperature for 3hr. The reaction was quenched with water and extracted with DCM (30ml*3). The organic layer was collected and dried over anhydrous MgSO₄ concentrated *in vacuo* and purified by silica gel chromatography (ethyl acetate: n-hexane = 1: 1) to afford **32c** (0.02 g, 20%). ¹H-NMR (300MHz, MeOD): δ 1.25 (d, *J=* 7.2 Hz, 6H), 1.62 (t, *J=* 7.2 Hz, 3H), 2.71 (s, 3H), 3.19-3.28 (m, 1H), 4.38 (q, *J=* 7.2 Hz, 2H), 6.62 (s, 1H), 7.59-7.67 (m, 1H), 7.75-7.83 (m, 1H), 7.94-8.09 (m, 3H), 8.45 (s, 1H).

### 1-(6-amino-3,4-dihydroquinolin-1(2H)-yl)ethanone (119)

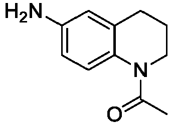

### 2,4-bis(benzyloxy)-5-isopropyl-N-(1,2,3,4-tetrahydroquinolin-6-yl)benzamide (120)

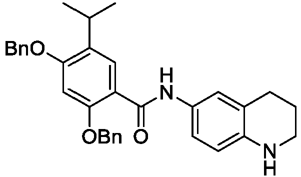

### 2,4-bis(benzyloxy)-5-isopropyl-N-methyl-N-(1,2,3,4-tetrahydroquinolin-6-yl)benzamide (121)

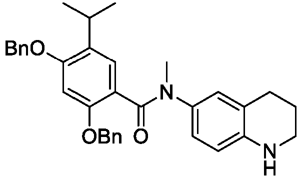

### 2,4-dihydroxy-5-isopropyl-N-methyl-N-(1,2,3,4-tetrahydroquinolin-6-yl)benzamide (33a)

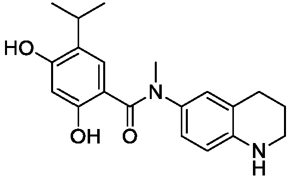

### N-ethyl-2,4-dihydroxy-5-isopropyl-N-(1,2,3,4-tetrahydroquinolin-6-yl)benzamide (33b)

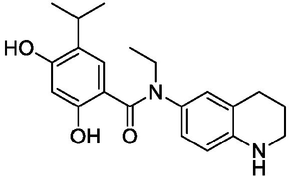

### 2,4-dihydroxy-5-isopropyl-N-methyl-N-(1-methyl-1,2,3,4-tetrahydroquinolin-6-yl)benzamide (33c)

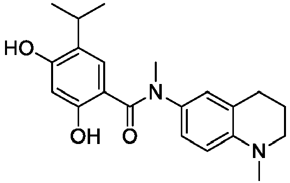

### N-ethyl-2,4-dihydroxy-5-isopropyl-N-(1-methyl-1,2,3,4-tetrahydroquinolin-6-yl)benzamide (33d)

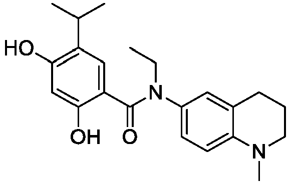

### 2,4-bis(benzyloxy)-5-isopropyl-N-(2-methylpyridin-3-yl)benzamide (124)

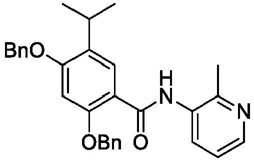

### 2,4-bis(benzyloxy)-N-(2-(benzyloxy)pyridin-3-yl)-5-isopropylbenzamide (125)

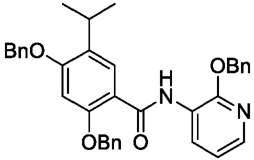

### N-ethyl-2,4-dihydroxy-5-isopropyl-N-(2-methylpyridin-3-yl)benzamide (34a)

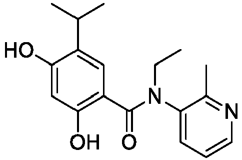

### N-(2-(benzyloxy)pyridin-3-yl)-N-ethyl-2,4-dihydroxy-5-isopropylbenzamide (34b)

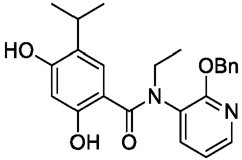

### N-(benzo[d]isoxazol-5-yl)-2,4-bis(benzyloxy)-5-isopropylbenzamide (127)

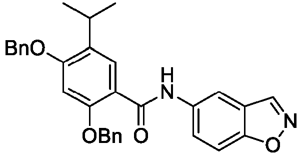

### N-(benzo[d]isoxazol-5-yl)-2,4-dihydroxy-5-isopropyl-N-methylbenzamide (35a)

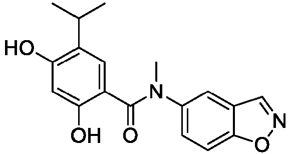

### N-(benzo[d]isoxazol-5-yl)-N-ethyl-2,4-dihydroxy-5-isopropylbenzamide (35b)

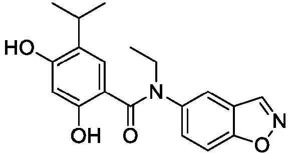

### (((4-isopropyl-6-nitro-1,3-phenylene)bis(oxy))bis(methylene))dibenzene (128)

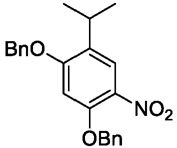

A mixture of **45** (3.00g, 9.04mmol), nitric acid (20ml) and acetic acid (50ml) was stirred for 10 min at room temperature. The reaction was quenched with water and extracted with CH₂Cl₂ (30ml*3). The organic layer was collected and dried over anhydrous MgSO₄ concentrated *in vacuo* to yield colorless oil product. The residue was purified by silica gel chromatography (ethyl acetate: n-hexane = 1:4, Rf= 0.4) to afford **128** (1.83g, 53.67%) as a yellow solid.¹H-NMR (500MHz, CDCl₃): δ 1.22 (d, *J=* 7 Hz, 6H), 3.28 (Sep, *J=* 7 Hz, 1H), 5.07 (s, 2H), 5.16 (s, 2H), 6.54 (s, 1H), 7.39 (m, 10H), 7.89 (s, 1H).

### 2,4-bis(benzyloxy)-5-isopropylaniline (129)

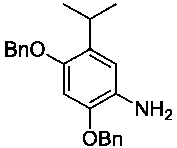

A mixture of **128** (1g, 2.65mmol), Fe powder (0.8g, 14.33mmol), NH₄Cl (0.6g, 11.11mmol), IPA (20ml) and H₂O (5ml), was stirred and reflux for 1.5h. The Fe powder was removed by ethyl acetate wash through celite packing. The organic layer was concentrated *in vacuo* and purified by silica gel chromatography (ethyl acetate: n-hexane = 1:2, Rf= 0.3) to afford **129** (0.67g, 72.83%) as a block solid.¹H-NMR (500MHz, CDCl₃): δ 1.18 (d, *J=* 7 Hz, 6H), 3.31 (Sep, *J=* 7 Hz, 1H), 4.34 (s, 2H), 4.96 (s, 2H), 5.02 (s, 2H), 6.56 (s, 1H), 6.66 (s, 1H), 7.36 (m, 10H).

### N-(2,4-bis(benzyloxy)-5-isopropylphenyl)-3-fluorobenzamide (130)

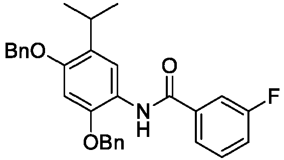

A mixture of **129** (1g, 7.14mmol), EDC.HCl (2g, 10.47mmol), HOBt (1.20g, 8.89mmol), NMM (2ml, 18.98mmol) and DMF (10ml) was stirred for 10 min then added 3-fluorobenzoic acid (2g, 5.76mmol) for overnight, the reaction was quenched with water and extracted with ethyl acetate (30ml*3). The organic layer was collected and dried over anhydrous MgSO₄ concentrated *in vacuo* to yield colorless oil product. The residue was purified by silica gel chromatography (ethyl acetate: n-hexane = 1:2, Rf= 0.6) to afford **130** (1.73g, 64.07%) as a block solid. ¹H-NMR (500MHz,CDCl₃): δ 1.30 (d, *J=* 7Hz, 6H), 3.42 (Sep, *J=* 7 Hz, 1H), 5.05 (s, 2H), 5.11 (s, 2H), 6.64 (s, 1H), 7.36 (m, 14H), 8.39 (s, 1H), 8.43 (s, 1H).

### N-(2,4-bis(benzyloxy)-5-isopropylphenyl)-4-fluorobenzamide (131)

A mixture of **129** (1g, 7.14mmol), EDC.HCl (2g, 10.47mmol), HOBt (1.20g, 8.89mmol), NMM (2ml, 18.98mmol) and DMF (10ml) was stirred for 10 min then added 4-fluorobenzoic acid (2g, 5.76mmol) for overnight, the reaction was quenched with water and extracted with ethyl acetate (30ml*3). The organic layer was collected and dried over anhydrous MgSO₄ concentrated *in vacuo* to yield colorless oil product. The residue was purified by silica gel chromatography (ethyl acetate: n-hexane = 1:4, Rf= 0.7) to afford **131** (1.64g, 60.74%) as a block solid. ¹H-NMR (500MHz, CDCl₃): δ1.25 (d, *J=* 7 Hz, 6H), 3.38 (Sep, *J*= 7Hz, 1H), 5.03 (s, 2H), 5.08 (s, 2H), 6.61 (s, 1H), 7.09 (m, 2H), 7.36 (m, 10H), 7.78 (m, 2H), 8.30 (s, 1H), 8.40 (s, 1H).

### N-(2,4-dihydroxy-5-isopropylphenyl)-3-fluorobenzamide (36a)

A mixture of **130** (0.35g, 0.75mmol), 10% palladium on activated carbon (0.04g, 0.04mmol) and MeOH (8ml) was under the hydrogen gas. The reaction was stirred at room temperature for 2h. The 10% palladium on activated carbon was removed by methyl alcohol wash through celite packing. The organic layer was concentrated *in vacuo* and purified by silica gel chromatography (ethyl acetate: n-hexane = 1:2, Rf= 0.4) to afford **36a** (0.19g, 86.36%) as a brown oil. ¹H-NMR (500MHz, MeOD): δ 1.19 (d, *J=* 7 Hz, 6H), 3.19 (Sep, *J=* 7 Hz, 1H), 6.41 (s, 1H), 7.28 (m, 1H), 7.67 (qui, *J*= 7.5 Hz, 1H), 7.75 (d, *J=* 7.5 Hz, 1H).

### N-(2,4-dihydroxy-5-isopropylphenyl)-3-fluoro-N-methylbenzamide (36b)

A mixture of **130** (0.37g, 0.79mmol), NaH (0.04g, 1mmole) and DMF (4ml) was added methyl iodide (0.1ml, 1.61mmol) at room temperature for 2h. The reaction was quenched with water and extracted with ethyl acetate (30ml*3). The organic layer was collected and dried over anhydrous MgSO₄ concentrated *in vacuo* and purified by silica gel chromatography (ethyl acetate: n-hexane = 1:2, Rf= 0.5) to get the oily product, then the oily product was dissolved in MeOH (8ml) and added 10% palladium on activated carbon (0.04g, 0.04mmol) under the hydrogen gas. The reaction was stirred at room temperature for 2h. The 10% palladium on activated carbon was removed by methyl alcohol wash through celite packing. The organic layer was concentrated *in vacuo* and purified by silica gel chromatography (ethyl acetate: n-hexane = 1:2, Rf= 0.2) to afford **36b** (0.15g, 62.50%) as a brown oil. ¹H-NMR (500MHz, MeOD): δ 0.90 (d, *J=* 7 Hz, 3H), 1.05 (d, *J=* 7 Hz, 3H), 3.00 (Sep, *J*= 7Hz, 1H), 3.29 (s, 3H), 6.24 (s, 1H), 6.61 (s, 1H), 6.94 (m, 1H), 7.00 (m, 1H), 7.08 (d, *J=* 7.5 Hz, 1H), 7.16 (m, 1H).

### N-(2,4-dihydroxy-5-isopropylphenyl)-N-ethyl-3-fluorobenzamide (36c)

A mixture of **130** (0.33g, 0.70mmol), NaH (0.04g, 1mmole) and DMF (4ml) was added ethyl iodide (0.1ml, 1.24mmol) at room temperature for 2h. The reaction was quenched with water and extracted with ethyl acetate (30ml*3). The organic layer was collected and dried over anhydrous MgSO₄ concentrated *in vacuo* and purified by silica gel chromatography (ethyl acetate: n-hexane = 1:2, Rf= 0.5) to get the oily product, then the oily product was dissolved in MeOH (7ml) and added 10% palladium on activated carbon (0.04g, 0.04mmol) under the hydrogen gas. The reaction was stirred at room temperature for 2h. The 10% palladium on activated carbon was removed by methyl alcohol wash through celite packing. The organic layer was concentrated *in vacuo* and purified by silica gel chromatography (ethyl acetate: n-hexane = 1:2, Rf= 0.3) to afford **36c** (0.13g, 59.09%) as a pink solid. m.p. 183.8- 184.1. ¹H-NMR (500MHz, MeOD): δ 0.92 (d, *J=* 7Hz, 3H), 1.06 (d, *J=* 7 Hz, 3H), 1.17 (t, *J=* 7 Hz, 3H), 3.01 (Sep, *J=* 7 Hz, 1H), 3.58 (Sex, *J*= 7 Hz, 1H), 4.01 (Sex, *J*= 7 Hz, 1H), 6.23 (s, 1H), 6.57 (s, 1H), 6.93 (m, 1H), 6.98 (m, 1H), 7.01 (m, 1H), 7.15 (m, 1H).

### N-(2,4-dihydroxy-5-isopropylphenyl)-3-fluoro-N-propylbenzamide (36d)

A mixture of **130** (0.34g, 0.72mmol), NaH (0.04g, 1mmole) and DMF (4ml) was added 1-iodopropane (0.1ml, 1.03mmol) at room temperature for 2h. The reaction was quenched with water and extracted with ethyl acetate (30ml*3). The organic layer was collected and dried over anhydrous MgSO₄ concentrated *in vacuo* and purified by silica gel chromatography (ethyl acetate: n-hexane = 1:2, Rf= 0.5) to get the oily product, then the oily product was dissolved in MeOH (9ml) and added 10% palladium on activated carbon (0.04g, 0.04mmol) under the hydrogen gas. The reaction was stirred at room temperature for 2h. The 10% palladium on activated carbon was removed by methyl alcohol wash through celite packing. The organic layer was concentrated *in vacuo* and purified by silica gel chromatography (ethyl acetate: n-hexane = 1:2, Rf= 0.3) to afford **36d** (0.14g, 58.33%) as a pink solid. m.p. 185.1- 186.4. ¹H-NMR (500MHz, MeOD): δ 0.92 (m, 6H), 1.06 (d, *J=* 7 Hz, 3H), 1.61 (m, 2H), 3.01 (qui, *J*= 7 Hz, 1H), 3.97 (m, 1H), 6.23 (s, 1H), 6.57 (s, 1H), 6.93 (m, 1H), 7.01 (m, 1H), 7.08 (m, 1H), 7.16 (m, 1H).

### N-(2,4-dihydroxy-5-isopropylphenyl)-3-fluoro-N-isopropylbenzamide (36e)

A mixture of **130** (0.34g, 0.72mmol), NaH (0.04g, 1mmole) and DMF (4ml) was added 2-iodopropane (0.1ml, 1.03mmol) at room temperature for 2h. The reaction was quenched with water and extracted with ethyl acetate (30ml*3). The organic layer was collected and dried over anhydrous MgSO₄ concentrated *in vacuo* and purified by silica gel chromatography (ethyl acetate: n-hexane = 1:2, Rf= 0.5) to get the oily product, then the oily product was dissolved in MeOH (7ml) and added 10% palladium on activated carbon (0.04g, 0.04mmol) under the hydrogen gas. The reaction was stirred at room temperature for 2h. The 10% palladium on activated carbon was removed by methyl alcohol wash through celite packing. The organic layer was concentrated *in vacuo* and purified by silica gel chromatography (ethyl acetate: n-hexane = 1:2, Rf= 0.3) to afford **36e** (0.13g, 54.17%) as a pink solid. m.p. 226.4-228.9. ¹H-NMR (500MHz, MeOD): δ 1.02 (d, *J=* 7 Hz, 3H), 1.12 (t, *J=* 7 Hz, 6H), 1.25 (d, *J=* 7 Hz, 3H), 3.05 (Sep, *J=* 7 Hz, 1H), 4.81 (m, 1H), 6.15 (s, 1H), 6.67 (s, 1H), 6.89 (m, 1H), 7.00 (d, *J=* 9.5 Hz, 1H), 7.06 (d, *J=* 7.5 Hz, 1H), 7.12 (m, 1H).

### N-(2,4-dihydroxy-5-isopropylphenyl)-4-fluorobenzamide (37a)

A mixture of **131** (0.34g, 0.72mmol), 10% palladium on activated carbon (0.04g, 0.04mmol) and MeOH (9ml) was under the hydrogen gas. The reaction was stirred at room temperature for 2h. The 10% palladium on activated carbon was removed by methyl alcohol wash through celite packing. The organic layer was concentrated *in vacuo* and purified by silica gel chromatography (ethyl acetate: n-hexane = 1:2, Rf= 0.3) to afford **37a** (0.17g, 80.95%)as a brown oil. ¹H-NMR (500MHz, CDCl₃): δ 1.20 (d, *J=* 7Hz, 6H), 3.14 (Sep, *J=* 7 Hz, 1H), 5.35 (s, 1H), 6.47 (s, 1H), 6.87 (s, 1H), 7.17 (m, 2H), 7.92 (m, 2H), 8.04 (s, 1H), 8.73 (s, 1H).

### N-(2,4-dihydroxy-5-isopropylphenyl)-4-fluoro-N-methylbenzamide (37b)

A mixture of **131** (0.37g, 0.79mmol), NaH (0.04g, 1mmole) and DMF (4ml) was added methyl iodide (0.1ml, 1.61mmol) at room temperature for 2h. The reaction was quenched with water and extracted with ethyl acetate (30ml*3). The organic layer was collected and dried over anhydrous MgSO₄ concentrated *in vacuo* and purified by silica gel chromatography (ethyl acetate: n-hexane = 1:2, Rf= 0.6) to get the oily product, then the oily product was dissolved in MeOH (7ml) and added 10% palladium on activated carbon (0.04g, 0.04mmol) under the hydrogen gas. The reaction was stirred at room temperature for 2h. The 10% palladium on activated carbon was removed by methyl alcohol wash through celite packing. The organic layer was concentrated *in vacuo* and purified by silica gel chromatography (ethyl acetate: n-hexane = 1:2, Rf= 0.3) to afford **37b** (0.14g, 58.33%) as a white solid. m.p. 180.1-180.9. ¹H-NMR (500MHz, MeOD): δ 0.90 (d, *J=* 7 Hz, 3H), 1.04 (d, *J=* 7 Hz, 3H), 3.00 (Sep, *J=* 1H), 3.29 (s, 3H), 6.24 (s, 1H), 6.58 (s, 1H), 6.88 (m, 2H), 7.31 (m, 1H).

### N-(2,4-dihydroxy-5-isopropylphenyl)-N-ethyl-4-fluorobenzamide (37c)

A mixture of **131** (0.32g, 0.68mmol), NaH (0.04g, 1mmole) and DMF (4ml) was added ethyl iodide (0.1ml, 1.24mmol) at room temperature for 2h. The reaction was quenched with water and extracted with ethyl acetate (30ml*3). The organic layer was collected and dried over anhydrous MgSO₄ concentrated *in vacuo* and purified by silica gel chromatography (ethyl acetate: n-hexane = 1:2, Rf= 0.5) to get the oily product, then the oily product was dissolved in MeOH (7ml) and added 10% palladium on activated carbon (0.04g, 0.04mmol)under the hydrogen gas. The reaction was stirred at room temperature for 2h. The 10% palladium on activated carbon was removed by methyl alcohol wash through celite packing. The organic layer was concentrated *in vacuo* and purified by silica gel chromatography (ethyl acetate: n-hexane = 1:2, Rf= 0.3) to afford **37c** (0.13g, 59.09%) as a white solid. m.p. 209.9-211.3. ¹H-NMR (500MHz, MeOD): δ 0.91 (d, *J=* 7 Hz, 3H), 1.05 (d, *J=* 7 Hz, 3H), 1.16 (t, *J=* 7 Hz, 3H), 3.02 (Sep, *J=* 7 Hz, 1H), 3.58 (6, *J=* 7 Hz, 1H), 4.00 (6, *J=* 7 Hz, 1H), 6.24 (s, 1H), 6.54 (s, 1H), 6.86 (m, 2H), 7.31 (m, 2H).

### N-(2,4-dihydroxy-5-isopropylphenyl)-4-fluoro-N-propylbenzamide (37d)

A mixture of **131** (0.36g, 0.77mmol), NaH (0.04g, 1mmole) and DMF (4ml) was added 1-iodopropane (0.1ml, 1.03mmol) at room temperature for 2h. The reaction was quenched with water and extracted with ethyl acetate (30ml*3). The organic layer was collected and dried over anhydrous MgSO₄ concentrated *in vacuo* and purified by silica gel chromatography (ethyl acetate: n-hexane = 1:2, Rf= 0.5) to get the oily product, then the oily product was dissolved in MeOH (8ml) and added 10% palladium on activated carbon (0.04g, 0.04mmol) under the hydrogen gas. The reaction was stirred at room temperature for 2h. The 10% palladium on activated carbon was removed by methyl alcohol wash through celite packing. The organic layer was concentrated *in vacuo* and purified by silica gel chromatography (ethyl acetate: n-hexane = 1:2, Rf= 0.3) to afford **37d** (0.15g, 57.69%) as a pink solid. m.p. 178.1-178.7. ¹H-NMR (500MHz, MeOD): δ 0.92 (m, 6H), 1.05 (d, *J=* 7 Hz 3H), 1.59 (m, 2H), 3.00 (Sex, *J*= 7 Hz, 1H), 4.45 (m, 1H), 3.96 (m, 1H), 6.23 (s, 1H), 6.54 (s, 1H), 6.86 (m, 2H), 7.29 (m, 2H).

### N-(2,4-dihydroxy-5-isopropylphenyl)-4-methoxybenzenesulfonamide (38a)

A mixture of **131** (0.8g, 2.3mmol), pyridine (2ml) and DCM (8ml) was added 4-methoxybenzesulfonyl chloride (0.7g, 3.39mmol) and stirred at room temperature for 2h. The reaction was quenched with water and extracted with ethyl acetate (30ml*3). The organic layer was collected and dried over anhydrous MgSO₄ concentrated *in vacuo* and purified by silica gel chromatography (ethyl acetate: n-hexane = 1:2, Rf= 0.6) to get the oily product, then the oily product was dissolved in MeOH (15ml) and added 10% palladium on activated carbon (0.10g, 0.09mmol) under the hydrogen gas. The reaction was stirred at room temperature for 2h. The 10% palladium on activated carbon was removed by methyl alcohol wash through celite packing. The organic layer was concentrated *in vacuo* and purified by silica gel chromatography (ethyl acetate: n-hexane = 1:2, Rf= 0.2) to afford **38a** (0.51g, 65.38%) as a block solid. m.p. 127.5-129.8. ¹H-NMR (500MHz, CDCl₃): δ 0.93 (d, *J=* 6.5 Hz, 6H), 2.96 (qui, *J*= 6.5 Hz, 1H), 3.82 (s, 3H), 6.23 (s, 1H), 6.32 (s, 1H), 6.40 (s, 1H), 6.88 (d, *J=* 9 Hz, 2H), 7.60 (d, *J=* 9 Hz, 2H).

### N-(2,4-dihydroxy-5-isopropylphenyl)-4-fluorobenzenesulfonamide (38b)

A mixture of **131** (0.45g, 1.30mmol), pyridine (2ml) and DCM (8ml) was added 4-fluorobenzenesulfonyl chloride (0.4g, 2.06mmol) and stirred at room temperature for 2h. The reaction was quenched with water and extracted with ethyl acetate (30ml*3). The organic layer was collected and dried over anhydrous MgSO₄ concentrated *in vacuo* and purified by silica gel chromatography (ethyl acetate: n-hexane = 1:2, Rf= 0.5) to get the oily product, then the oily product was dissolved in MeOH (7ml) and added 10% palladium on activated carbon (0.05g, 0.05mmol) under the hydrogen gas. The reaction was stirred at room temperature for 2h. The 10% palladium on activated carbon was removed by methyl alcohol wash through celite packing. The organic layer was concentrated *in vacuo* and purified by silica gel chromatography (ethyl acetate: n-hexane = 1:2, Rf= 0.3) to afford **38b** (0.27g, 64.29%) as a brown solid. m.p. 168.3-169.1. ¹H-NMR (500MHz, CDCl₃): δ 0.94 (d, *J=* 7 Hz, 6H), 2.98 (Sep, *J=* 7 Hz, 1H), 6.16 (s, 1H), 6.55 (s, 1H), 6.99 (m, 2H), 7.61 (m, 2H).

### 4-isopropyl-6-(3,4,5-trimethoxyphenethyl)benzene-1,3-diol (39a)

A mixture of 3,4,5-trimethoxybenzyl chloride (0.8g, 3.69mmol), triphenylphosphine (3g, 11.44mmol) and toluene (10ml) was refluxed for overnight. The reaction was filtered by hexane wash and took the precipitant and directly used in next step. A mixture of **46** (1g, 2.78mmol), NaH (0.15g, 3.75mmol) and toluene (15ml) was stirred for 10 min then added the former precipitant at room temperature for overnight. The residue was quenched with water and extracted with ethyl acetate (30ml*3). The organic layer was collected and dried over anhydrous MgSO₄ concentrated *in vacuo* and purified by silica gel chromatography (ethyl acetate: n-hexane = 1: 2, Rf= 0.6) to afford as an oily product and directly used in next step. A mixture of oily product, 10% palladium on activated carbon (0.1g, 0.09mmol) and MeOH (6ml) was under the hydrogen gas. The reaction was stirred at room temperature for 2h. The 10% palladium on activated carbon was removed by methyl alcohol wash through celite packing. The organic layer was concentrated *in vacuo* and pu0rified by silica gel chromatography (ethyl acetate: n-hexane = 1: 2, Rf= 0.3) to afford **39a** (0.54g, 56.25%) as a colorless oil. ¹H-NMR (500MHz, MeOD): δ 1.06 (d, *J=* 7 Hz, 6H), 2.74 (s, 4H), 3.09 (Sep, *J=* 7 Hz, 1H), 3.70 (s, 3H), 3.73 (s, 6H), 6.28 (s, 1H), 6.36 (s, 2H), 6.55 (s, 1H).

### 4-isopropyl-6-(4-methoxyphenethyl)benzene-1,3-diol (39b)

A mixture of 4-methoxybenzyl chloride (0.6g, 3.83mmol), triphenylphosphine (3g, 11.44mmol) and toluene (9ml) was refluxed for overnight. The reaction was filtered by hexane wash and took the precipitant and directly used in next step. A mixture of **46** (1g, 2.78mmol), NaH (0.15g, 3.75mmol) and toluene (15ml) was stirred for 10 min then added the former precipitant at room temperature for overnight. The residue was quenched with water and extracted with ethyl acetate (30ml*3). The organic layer was collected and dried over anhydrous MgSO₄ concentrated *in vacuo* and purified by silica gel chromatography (ethyl acetate: n-hexane = 1: 3, Rf= 0.7) to afford as an oily product and directly used in next step. A mixture of oily product, 10% palladium on activated carbon (0.1g, 0.09mmol) and MeOH(7ml) was under the hydrogen gas. The reaction was stirred at room temperature for 2h. The 10% palladium on activated carbon was removed by methyl alcohol wash through celite packing. The organic layer was concentrated *in vacuo* and purified by silica gel chromatography (ethyl acetate: n-hexane = 1: 3, Rf= 0.2) to afford **39b** (0.43g, 53.75%) as a brown oil. ¹H-NMR (500MHz, CDCl₃): δ 1.19 (d, *J=* 7 Hz, 6H), 2.80 (m, 4H), 3.06 (Sep, *J=* 7 Hz, 1H), 3.78 (s, 3H), 4.67 (s, 1H), 4.86 (s, 1H), 6.21 (s, 1H), 6.81 (m, 3H), 7.07 (d, *J=* 8.5 Hz, 2H).

### 4-(4-fluorophenethyl)-6-isopropylbenzene-1,3-diol (39c)

A mixture of 4-fluorobenzyl chloride (0.5g, 3.47mmol), triphenylphosphine (3g, 11.44mmol) and toluene (9ml) was refluxed for overnight. The reaction was filtered by hexane wash and took the precipitant and directly used in next step. A mixture of **46** (1g, 2.78mmol), NaH (0.15g, 3.75mmol) and toluene (15ml) was stirred for 10 min then added the former precipitant at room temperature for overnight. The residue was quenched with water and extracted with ethyl acetate (30ml*3). The organic layer was collected and dried over anhydrous MgSO₄ concentrated *in vacuo* and purified by silica gel chromatography (ethyl acetate: n-hexane = 1: 3, Rf= 0.6) to afford as an oily product and directly used in next step. A mixture of oily product, 10% palladium on activated carbon (0.1g, 0.09mmol) and MeOH(7ml) was under the hydrogen gas. The reaction was stirred at room temperature for 2h. The 10% palladium on activated carbon was removed by methyl alcohol wash through celite packing. The organic layer was concentrated *in vacuo* and purified by silica gel chromatography (ethyl acetate: n-hexane = 1: 3, Rf= 0.3) to afford **39c** (0.39g, 51.31%) as a pink solid. m.p. 82.6-83.3. ¹H-NMR (500MHz, CDCl₃): δ 1.17 (d, *J=* 6.5 Hz, 6H), 2.81 (m, 4H), 3.04 (qui, *J*= 7 Hz, 1H), 4.60 (s, 1H), 4.72 (s, 1H), 6.23 (s, 1H), 6.74 (s, 1H), 6.93 (m, 2H), 7.09 (m, 2H).

### 1-(2,4-dihydroxy-5-isopropylphenyl)ethanone (139)

A mixture of **45** (3.00g, 9.04mmol), 10% palladium on activated carbon (0.4g, 0.38mmol) and MeOH (15ml) was under the hydrogen gas and 40 psi at room temperature for overnight. The 10% palladium on activated carbon was removed by methyl alcohol wash through celite packing. The organic layer was concentrated *in vacuo* and purified by silica gel chromatography (ethyl acetate: n-hexane = 1: 4, Rf= 0.5) to afford as an oily product, and directly used in next step. A mixture of the oily product, ZnCl₂ (2.00g, 20mmol) and acetic acid (8ml) was refluxed for overnight. The reaction was quenched with water and extracted with ethyl acetate (30ml*3). The organic layer was collected and dried over anhydrous MgSO₄ concentrated *in vacuo* and purified by silica gel chromatography (ethyl acetate: n-hexane = 1: 4, Rf= 0.3) to afford **139** (1.02g, 58.29%) as an oily product. ¹H-NMR (500MHz, CDCl₃): δ 1.25 (d, *J=* 6.5 Hz, 6H), 2.57 (s, 3H), 3.13 (Sep, *J=* 7 Hz, 1H), 5.57 (s, 1H), 6.30 (s, 1H), 7.26 (s, 1H), 7.49 (s, 1H), 12.53 (s, 1H).

### 1-(5-isopropyl-2,4-bis((4-methoxybenzyl)oxy)phenyl)ethanone (140)

A mixture of **139** (1.02g, 5.25mmol), Cs₂CO₃ (5g, 15.35mmol), KI (cat.) and DMF (8ml) was added 4-methoxybenzyl chloride (2ml, 14.75mmol) at room temperature for overnight. The reaction was quenched with 6N NaOH (aq.) and extracted with ethyl acetate (30ml*3). The organic layer was collected and dried over anhydrous MgSO₄ concentrated *in vacuo* to and purified by silica gel chromatography (ethyl acetate: n-hexane = 1: 3, Rf= 0.4) to afford **140** (1.51g, 66.23%) as a yellow solid.¹H-NMR (500MHz, CDCl₃): δ 1.20 (d, *J=* 6.5 Hz, 6H), 2.53 (s, 3H), 3.24 (qui, *J*= 7 Hz, 1H), 3.82 (s, 6H), 5.03 (s, 2H), 5.04 (s, 2H), 6.53 (s, 1H), 6.92 (m, 4H), 7.32 (m, 4H), 7.74 (s, 1H).

### (E)-3-(3,4-dimethoxyphenyl)-1-(5-isopropyl-2,4-bis((4-methoxybenzyl)oxy)phenyl)prop-2-en-1-one (143)

A mixture of **140** (1.00g, 2.30mmol), 1N NaOH (5ml), MeOH (8ml) and DCM (3ml) was stirred for 10 min then added 3,4-dimethoxybenzaldehyde (0.6g, 3.61mmol) at room temperature for overnight. The reaction was quenched with water and extracted with CH₂Cl₂ (30ml*3). The organic layer was collected and dried over anhydrous MgSO₄ concentrated *in vacuo* to yield colorless oil product. The residue was purified by silica gel chromatography (ethyl acetate: n-hexane = 1: 2, Rf= 0.2) to afford **143** (0.68g, 50.75%) as a yellow solid. ¹H-NMR (500MHz, MeOD): δ 1.22 (d, *J=* 6.5 Hz, 6H), 3.28 (Sep, *J=* 7 Hz, 1H), 3.72 (d, *J*= 4.5 Hz, 6H), 3.79 (s, 3H), 3.87 (s, 3H), 5.02 (s, 2H), 5.06 (s, 2H), 6.76 (m, 3H), 6.90 (m, 4H), 7.33 (m, 4H), 7.52 (d, *J=* 16 Hz, 1H), 7.51 (d, *J=* 16 Hz, 1H), 7.77 (s, 1H).

### (E)-3-(2,5-dimethoxyphenyl)-1-(5-isopropyl-2,4-bis((4-methoxybenzyl)oxy)phenyl)prop-2-en-1-one (144)

A mixture of **140** (1.00g, 2.30mmol), 1N NaOH (6ml), MeOH (9ml) and DCM (3ml) was stirred for 10 min then added 2,5-dimethoxybenzaldehyde (0.6g, 3.61mmol) at room temperature for overnight. The reaction was quenched with water and extracted with CH₂Cl₂ (30ml*3). The organic layer was collected and dried over anhydrous MgSO₄ concentrated *in vacuo* to yield colorless oil product. The residue was purified by silica gel chromatography (ethyl acetate: n-hexane = 1:3, Rf= 0.2) to afford **144** (0.70g, 52.24%) as a yellow solid. ¹H-NMR (500MHz, CDCl₃): δ 1.25 (d, *J=* 6.5 Hz, 6H), 3.31 (Sep, *J=* 7 Hz, 1H), 3.66 (s, 3H), 3.74 (d, *J=* 7 Hz, 6H), 3.80 (s, 3H), 5.05 (s, 2H), 5.06 (s, 2H), 6.61 (s, 1H), 6.80 (m, 3H), 6.87 (m, 1H), 6.95 (m, 3H), 7.33 (m, 4H), 7.66 (d, *J=* 16 Hz, 1H), 7.76 (s, 1H), 8.04 (d, *J=* 16 Hz, 1H).

### (E)-1-(2,4-dihydroxy-5-isopropylphenyl)-3-(3,4-dimethoxyphenyl)prop-2-en-1-one (40a)

A mixture of **143** (0.6g, 1.03mmol) and DCM (20ml) was added titanium (IV) chloride (0.3ml, 2.73mmol) at 0°C for 1 h. The reaction was quenched with water and extracted with ethyl acetate (30ml*3). The organic layer was collected and dried over anhydrous MgSO₄ concentrated *in vacuo* and purified by silica gel chromatography (ethyl acetate: n-hexane = 1:3, Rf= 0.3) to afford **40a** (0.19g, 54.29%) as an yellow solid. m.p. 177.2- 178.1. ¹H-NMR (500MHz, MeOD): δ 1.27 (d, *J=* 7.5 Hz, 6H), 3.20 (Sep, *J=* 7 Hz, 1H), 3.88 (s, 2H), 3.91 (s, 2H), 6.29 (s, 1H), 7.00 (d, *J=* 8.5 Hz, 1H), 7.32 (m, 1H), 7.37 (d, *J=* 2 Hz, 1H), 7.64 (d, *J=* 15 Hz, 1H), 7.79 (m, 2H).

### (E)-1-(2,4-dihydroxy-5-isopropylphenyl)-3-(2,5-dimethoxyphenyl)prop-2-en-1-one (40b)

A mixture of **144** (0.72g, 1.24mmol) and DCM (25ml) was added titanium(IV) chloride (0.3ml, 2.73mmol) at 0°C for 1 h. The reaction was quenched with water and extracted with ethyl acetate (30ml*3). The organic layer was collected and dried over anhydrous MgSO₄ concentrated *in vacuo* and purified by silica gel chromatography (ethyl acetate: n-hexane = 1:2, Rf= 0.4) to afford **40b** (0.2g, 47.62%) as an orange solid. m.p. 140.3- 142.1. ¹H-NMR (500MHz, CDCl₃): δ 1.28 (d, *J=* 6.5 Hz, 6H), 3.17 (Sep, *J=* 7 Hz, 1H), 3.83 (s, 3H), 3.88 (s, 3H), 6.12 (s, 1H), 6.37 (s, 1H), 6.88 (d, *J=* 9 Hz, 1H), 6.95 (m, 1H), 7.16 (d, *J=* 3 Hz, 1H), 7.69 (m, 2H), 8.10 (d, *J=* 16 Hz, 1H), 13.29 (s, 1H).

### 2,4-dihydroxy-5-isopropylbenzaldehyde (145)

A mixture of **45** (3.00g, 9.04mmol), 10% palladium on activated carbon (0.4g, 0.38mmol) and MeOH (15ml) was under the hydrogen gas and 40 psi at room temperature for overnight. The 10% palladium on activated carbon was removed by methyl alcohol wash through celite packing. The organic layer was concentrated *in vacuo* and purified by silica gel chromatography (ethyl acetate: n-hexane = 1:4, Rf= 0.2) to afford as an oily product, and directly used in next step. A mixture of POCl₃ (4ml, 42.91mmol) and DMF (3.5ml, 45.40mmol) was stirred at 0°C for 10 min then added the oily product dissolved in DMF (4ml). The reaction was stirred at room temperature for overnight. The reaction was quenched with 6N NaOH (aq.) and extracted with ethyl acetate (30ml*3). The organic layer was collected and dried over anhydrous MgSO₄ concentrated *in vacuo* to yield a yellow product. The residue was without more purification to afford **145** (0.91g, 55.82%) as a yellow solid. ¹H-NMR (500MHz, MeOD): δ 1.20 (d, *J=* 6.5 Hz, 6H), 3.16 (Sex, *J*= 7 Hz, 1H), 6.27 (s, 1H), 7.38 (s, 1H), 9.68 (s, 1H).

### 5-isopropyl-2,4-bis((4-methoxybenzyl)oxy)benzaldehyde (146)

A mixture of **145** (1g, 5.56mmol), K₂CO₃ (2g, 14.47mmol), KI (cat.) and DMF (8ml) was added 4-methoxybenzyl chloride (2ml, 14.75mmol) at room temperature for overnight. The reaction was quenched with 6N NaOH (aq.) and extracted with ethyl acetate (30ml*3). The organic layer was collected and dried over anhydrous MgSO₄ concentrated *in vacuo* and purified by silica gel chromatography (ethyl acetate: n-hexane = 1:4, Rf= 0.4) to afford **146** (1.49g, 63.67%) as a yellow solid. ¹H-NMR (500MHz, CDCl₃): δ 1.19 (d, *J=* 7 Hz, 6H), 3.23 (Sex, *J*= 7 Hz, 1H), 3.82 (s, 3H), 3.83 (s, 3H), 5.04 (s, 2H), 5.06 (s, 2H), 6.53 (s, 1H), 6.92 (m, 4H), 7.31 (d, *J=* 8.5 Hz, 4H), 7.72 (s, 1H), 10.35 (s, 1H).

### (E)-3-(2,4-bis(benzyloxy)-5-isopropylphenyl)-1-(3,4,5-trimethoxyphenyl)prop-2-en-1-one (148)

A mixture of 1-(3,4,5-trimethoxyphenyl)ethanone (0.6g, 2.86mmol), 1N NaOH (5ml) and MeOH (10ml) was stirred for 10 min then added **146** (0.8g 1.90mmol) at room temperature for overnight. The reaction was quenched with water and extracted with CH₂Cl₂ (30ml*3). The organic layer was collected and dried over anhydrous MgSO₄ concentrated *in vacuo* to yield colorless oil product. The residue was purified by silica gel chromatography (ethyl acetate: n-hexane = 1:4, Rf= 0.2) to afford **148** (0.50g, 43.10%) as a orange solid. ¹H-NMR (500MHz, CDCl₃): δ 1.22 (d, *J=* 7 Hz, 6H), 3.28 (hept, *J=* 7 Hz, 1H), 3.79 (d, *J=* 2Hz, 9H), 3.81 (s, 3H), 3.90 (s, 3H), 5.02 (s, 2H), 5.04 (s, 2H), 6.59 (s, 1H), 6.90 (m, 4H), 7.17 (s, 2H), 7.33 (m, 4H), 7.43 (s, 1H), 7.53 (d, *J=* 15.5 Hz, 1H), 8.02 (d, *J=* 15.5 Hz, 1H).

### (E)-3-(2,4-dihydroxy-5-isopropylphenyl)-1-(3,4,5-trimethoxyphenyl)prop-2-en-1-one (41) A mixture of 148 (0.66g, 1.07mmol) and DCM (25ml) was added titanium(IV) chloride

(0.3ml, 2.73mmol) at 0°C for 1 h. The reaction was quenched with water and extracted with ethyl acetate(30ml*3).The organic layer was collected and dried over anhydrous MgSO₄ concentrated *in vacuo* and purified by silica gel chromatography (ethyl acetate: n-hexane = 1:1, Rf= 0.3) to afford **41** (0.19g, 47.50%) as an red solid. m.p. 114.8-115.9. ¹H-NMR (500MHz, CDCl₃): δ 1.19 (d, *J=* 7 Hz, 6H), 3.13 (Sep, *J=* 7 Hz, 1H), 3.88 (d, *J=* 2.5 Hz, 9H), 6.32 (s, 1H), 7.22 (s, 2H), 7.30 (s, 1H), 7.44 (d, *J=* 15.5 Hz, 1H), 8.03 (d, *J=* 15.5 Hz, 1H).

### Compound G-9

¹H NMR (300 MHz, CD₃OD): δ 1.25 (d, *J =* 6.9 Hz, 6H), 1.39-1.42 (m, 4H), 1.62-1.72 (m, 4H), 2.12 (t, *J =* 7.5 Hz, 2H), 2.38 (t, *J =* 7.5 Hz, 2H), 3.16-3.26 (m, 1H), 6.35 (s, 1H), 7.53-7.60 (m, 4H), 7.75 (s, 1H). LRMS *m*/*z:* calculated 480.49 found 480.2 (M+Na).

### Compound G-10

¹H NMR (300 MHz, CD₃OD): δ 0.82 (d, *J =* 6.6 Hz, 6H), 1.30-1.38 (m, 4H), 1.63-1.69 (m, 4H), 2.10 (t, *J =* 7.2 Hz, 2H), 2.36 (t, *J =* 7.2 Hz, 2H), 2.88-2.97 (m, 1H), 3.82 (s, 3H), 6.19 (s, 1H), 6.61 (s, 1H), 7.12 (d, *J=* 8.4 Hz, 2H), 7.56 (d, *J =* 8.7 Hz, 1H). LRMS *m*/*z:* calculated 494.52 found 494.2 (M+Na).

### Compound G-11

¹H NMR (300 MHz, CD₃OD): δ 0.82 (d, *J =* 6.9 Hz, 6H), 1.20 (t, *J =* 6.3Hz, 3H), 1.38-1.39 (m, 4H), 1.62-1.70 (m, 4H), 2.10 (t, *J =* 6.9 Hz, 2H), 2.37 (t, *J =* 7.2 Hz, 2H), 2.90-2.97 (m, 1H), 3.89-3.96 (m, 2H), 6.19 (s, 1H), 6.60 (s, 1H), 7.11 (d, *J =* 8.7 Hz, 2H), 7.57 (d, *J=* 8.7 Hz, 1H). LRMS *m*/*z:* calculated 508.55 found 508.2 (M+Na).

### Compound G-12

¹H NMR (300 MHz, CD₃OD): δ 0.85 (d, *J =* 6.9 Hz, 6H), 1.18 (d, *J =* 6.9Hz, 6H), 1.36 (m, 4H), 1.61-1.63 (m, 4H), 2.00-2.10 (m, 3H), 2.34 (t, *J =* 7.2 Hz, 2H), 2.85-2.98 (m, 1H), 4.93-4.97 (m, 1H), 6.13 (s, 1H), 6.58 (s, 1H), 7.07 (d, *J*= 8.7 Hz, 2H), 7.52 (d, *J*= 8.7 Hz, 1H).

### Compound G-13

¹H NMR (300 MHz, DMSO): δ 1.18 (d, *J =* 6.9 Hz, 6H), 1.22-1.28 (m, 4H), 1.44-1.59 (m, 4H), 1.93 (t, *J =* 7.5 Hz, 2H), 2.29 (t, *J =* 7.5 Hz, 2H), 3.06-3.24 (m, 1H), 6.36 (s, 1H), 7.21-7.35 (m, 4H), 7.76 (s, 1H), 7.95 (s, 1H), 8.65 (s, 1H), 9.90 (s, 1H), 10.14 (d, *J=* 8.4 Hz, 2H), 10.33 (s, 1H). LRMS *m*/*z:* calculated 480.49 found 480.1 (M+Na).

### Compound G-14

¹H NMR (300 MHz, DMSO): δ 0.77 (d, *J =* 6.9 Hz, 6H), 1.23 (bs, 8H), 1.42-1.53 (m, 4H), 1.90-1.95 (m, 2H), 2.24 (t, *J =* 7.5 Hz, 2H), 2.79-2.86 (m, 1H), 3.29 (s, 3H), 6.19 (s, 1H), 6.60 (s, 1H), 6.86 (d, *J=* 7.8 Hz, 2H), 7.23 (t, *J =* 8.1 Hz, 1H), 7.41 (d, *J*= 8.1 Hz, 1H), 7.50 (s, 1H), 8.64 (s,1H), 9.72 (s,1H), 9.78 (s,1H), 10.31 (s, 1H), 10.70 (s, 1H). LRMS *m*/*z:* calculated 494.52 found 494.2 (M+Na).

### Compound G-15

¹H NMR (300 MHz, DMSO): δ 0.75 (d, *J =* 6.9 Hz, 6H), 1.09 (t, *J=* 7.2 Hz, 3H), 1.23 (bs, 9H), 1.42-1.53 (m, 4H), 1.90-1.96 (m, 2H), 2.24 (t, *J =* 7.2 Hz, 2H), 2.78-2.85 (m, 1H), 3.74-3.80 (m, 2H), 6.18 (s, 1H), 6.57 (s, 1H), 6.83 (d, *J=* 9.0 Hz, 2H), 7.24 (t, *J=* 8.1 Hz, 1H), 7.43-7.48 (m, 2H), 8.64 (s, 1H), 9.72 (s, 1H), 9.90 (s, 1H), 10.31 (s, 1H), 10.83 (s, 1H). LRMS *m*/*z:* calculated 508.55 found 508.2 (M+Na).

### Compound G-16

¹H NMR (300 MHz, CD₃OD): δ 1.25 (d, *J =* 6.9 Hz, 7H), 1.29-1.34 (m, 4H), 1.49-1.58 (m, 2H), 1.65-1.72 (m, 2H) 2.02-2.04 (m, 2H), 2.44 (t, *J=* 7.5 Hz, 2H), 3.17-3.26 (m, 1H), 6.37 (s, 1H), 7.19-7.32 (m, 3H), 7.68 (s, 1H), 7.81 (d, *J=* 7.8 Hz, 7H). LRMS *m*/*z:* calculated 480.49 found 480.2 (M+Na).

### Compound G-17

¹H NMR (300 MHz, CD₃OD): δ 0.82 (d, *J =* 6.9 Hz, 6H), 1.36-1.44 (m, 2H), 1.61-1.75 (m, 4H), 2.11 (t, *J =* 7.2 Hz, 2H), 2.36 (t, *J =* 7.5 Hz, 2H), 2.88-2.97 (m, 1H), 3.40 (s, 3H), 6.20 (s, 1H), 6.61 (s, 1H), 7.11 (d, *J*= 9 Hz, 2H), 7.55 (d, *J=* 8.7 Hz, 1H). (sample didnt send for mass yet)

### Compound G-18

¹H NMR (300 MHz, CD₃OD): δ 0.76 (d, *J =* 6.9 Hz, 6H), 1.25-1.32 (m, 6H), 1.54-1.65 (m, 4H), 2.04 (t, *J =* 7.5 Hz, 2H), 2.30 (t, *J =* 7.5 Hz, 2H), 2.83-2.92 (m, 1H), 3.36 (s, 3H), 6.15 (s, 1H), 6.56 (s, 1H), 7.07 (d, *J =* 8.7 Hz, 2H), 7.51 (d, *J =* 8.7 Hz, 1H).

### EXAMPLE 2: In vitro inhibition of HSP90 with compounds of formula (I)

Certain compounds of formula (I) prepared in EXAMPLE 1 were evaluated using the above-described SRB assay.

Shown in Table 1 below are antiproliferative activities of compounds of formula (I) in inhibiting proliferation of lung A549, colorectal HCT116, liver Hep3B, breast MDA-MB-231, prostate PC3, oral KB, and HUVEC cells. In Table 1, the compounds numbered 18 to 32 labelled 'a', 21b, 24b, 26b, 20f, 23f are provided for reference only.

**Table 1. In vitro activity against human cancer cell lines and HUVEC cell, GI₅₀ (µM)**

| Compd. No. | Lung A549 | Colorectal HCT116 | Liver Hep3B | Breast MDA-MB-231 | Prostate PC3 | Oral KB | HUVEC |
|---|---|---|---|---|---|---|---|
| 18a | | | | | | >50 | |
| 18b | 0.07±0.01 | 0.09±0.01 | 0.20±0.03 | 0.09±0.01 | | 0.05±0.02 | 0.87±0.01 |
| 18c | | | | | | 0.51±0.17 | |
| 18d | | | | | | 0.28±0.00 | |
| 19a | | | | | | >50 | |
| 19b | 0.12±0.01 | 0.64±0.41 | 0.10±0.01 | 0.08±0.01 | | 0.04±0.01 | <0.1 |
| 19c | | | | | | 0.42±0.08 | |
| 19d | | | | | | 0.25±0.05 | |
| 20a | | | | | | 23.83±5.19 | |
| 20b | 0.17±0.01 | 1.00±0.49 | 0.18±0.04 | 0.07±0.01 | | 0.024±0.004 | 0.57±0.02 |
| 20c | 0.11±0.01 | 0.37±0.05 | 0.11±0.02 | 0.081+0.01 | | 0.032±0.004 | <0.1 |
| 20d | | | | | | 0.330±0.010 | |
| 20e | | | | | | 0.165±0.007 | |
| 20f | | | | | | 30.24±3.87 | |
| 20g | 0.28±0.02 | 0.70±0.06 | 0.57±0.32 | 0.10±0.02 | | 0.049±0.018 | 0.82±0.03 |
| 20h | | | | | | 0.076±0.006 | |
| 20i | | | | | | 0.400±0.000 | |
| 21a | 0.30±0.03 | | | | | | |
| 21b | >10 | | | | | | |
| 21c | 0.10±0.02 | | | | | | |
| 21d | 0.09±0.01 | | | | | | |
| 21e | 1.62±0.12 | | | | | | |
| 21f | 0.84±0.04 | | | | | | |
| 22a | >10 | | | | | | |
| 22b | >10 | | | | | | |
| 22c | >10 | | | | | | |
| 22d | >10 | | | | | | |
| 22e | >10 | | | | | | |
| 23a | | | | | | 32.13±3.29 | |
| 23b | 2.82±0.03 | 5.53±0.38 | | | | 0.036±0.001 | |
| 23c | | | | | | 0.041±0.010 | |
| 23d | | | | | | 0.473±0.085 | |
| 23e | | | | | | 0.460±0.014 | |
| 23h | | | | | | 1.46±0.04 | |
| 24b | >10 | >10 | | | >10 | | |
| 24c | 0.27 ± 0.03 | 0.71±0.08 | | | | | |
| 24d | 0.23 ± 0.03 | 0.44±0.19 | | | | | |
| 24e | 2.10 ± 0.17 | 3.76±0.23 | | | | | |
| 24f | 2.06 ± 0.12 | 3.14±0.87 | | | | | |
| 25a | >10 | | | | >10 | | |
| 25b | 0.11 ± 0.01 | | | | 0.10±0.01 | | |
| 26a | >10 | >10 | | | >10 | | |
| 26b | >10 | >10 | | | | | |
| 26c | 0.70 ± 0.04 | 1.15 ± 0.22 | | | | | |
| 26d | 0.38 ± 0.02 | 0.71 ± 0.06 | | | | | |
| 26e | 4.21 ±0.22 | 8.29 ± 0.29 | | | | | |
| 26f | 3.64±0.05 | 6.86± 0.48 | | | | | |
| 27a | | >10 | | | >10 | | |
| 27b | | 8.96±0.18 | | | 8.49±0.49 | | |
| 27c | | 3.49±0.15 | | | 6.68±0.55 | | |
| 27d | | >10 | | | >10 | | |
| 27e | | 8.77±0.10 | | | >10 | | |
| 28a | >10 | | | | >10 | | |
| 28b | >10 | | | | >10 | | |
| 28c | 0.28±0.05 | | | | 0.14±0.01 | | |
| 28d | 1.16±0.10 | 1.21± 0.13 | | | 2.33±0.04 | | |
| 29a | >10 | | | | >10 | | |
| 29b | >10 | >10 | | | >10 | | |
| 30a | >10 | | | | >10 | | |
| 30b | >10 | | | | >10 | | |
| 30c | 0.51±0.09 | | | | 0.46±0.13 | | |
| 31a | >10 | | | | >10 | | |
| 31b | 8.70±0.25 | >10 | | | 8.93±0.25 | | |
| 31c | 3.70±0.17 | | | | 2.40±0.27 | | |
| 32a | >10 | | | | >10 | | |
| 32c | 5.67 ± 0.06 | 5.99± 0.30 | | | 6.30±0.30 | | |
| 33a | | 5.79±0.11 | | | 7.66±0.42 | | |
| 33b | | 5.38±0.10 | | | 6.01±0.41 | | |
| 33c | | 6.51±0.09 | | | 8.50±0.45 | | |
| 33d | | 5.71±0.12 | | | 8.18±0.46 | | |
| 34a | >10 | | | | | | |
| 34b | 0.21±0.01 | | | | | | |
| 35a | | | | | | 0.265±0.021 | |
| 35b | | | | | | 0.080±0.014 | |

Several tested compounds and two reference HSP90 inhibitors, namely, BIIB021 and Tanespimycin (i.e., 17-AAG), were evaluated using the above-described HSP90 ATP-binding assay and SRB assay.

Among the tested compounds, compounds 18b, 19b, 20b, 20c, and 20g showed high antiproliferative activity against lung A549, colon HCT116, liver Hep3B, breast MDA-MB-231 cancer cell lines as shown in Table 2 below. These five compounds all exhibited better HSP90 enzyme inhibition activity than 17-AAG and better cellular activity against A549 cell line than BIIB021. Unexpectedly, compound 18b displayed a 4-fold improvement of antiproliferative activity as compared to that of 17-AAG in HCT116. Further, compound 18b exhibited a high safety margin with a therapeutic index of 12.4 (measured by GI₅₀(HUVEC)/GI₅₀(A549)). Compound 18b also showed high antiproliferative activity against leukemia HL60, lymphoblast MOLT4 and bone marrow K562.

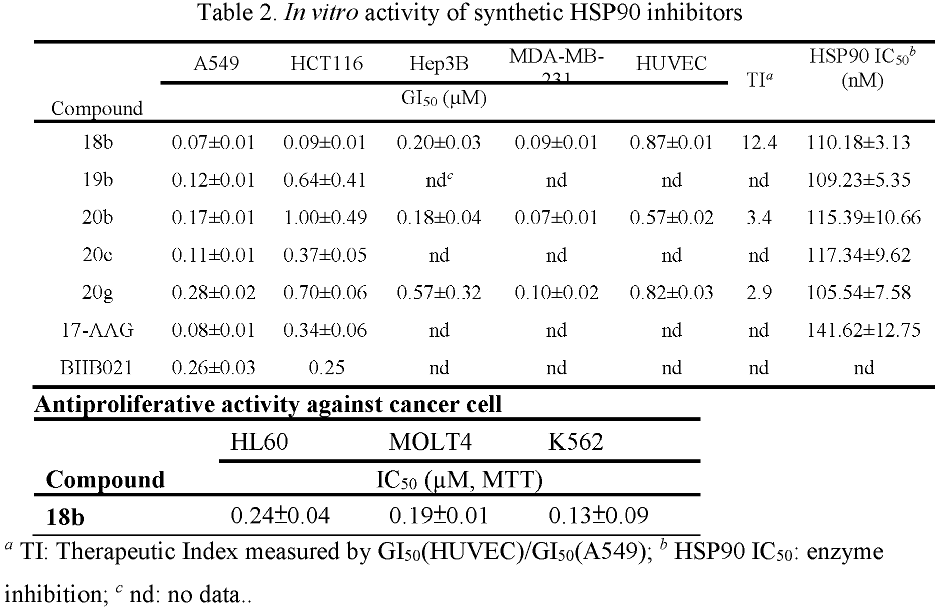

To confirm the inhibition of HSP90, western blotting analysis was performed to evaluate the levels HSP90 client proteins, i.e., EGFR, Akt, CDK-4, and HSP70, in HCT-116 tumor cells treated with compounds 18b, 20b, and 20g. Total cellular extracts were obtained 24 hours after treatment. It was observed that all tested compounds were effective, causing substantial depletion of the examined client proteins and inducing an increase in the expression levels of the chaperone HSP70, which confirmed the inhibition of HSP90.

These results indicate that compounds of formula (I) have high activity in inhibiting HSP90.

### EXAMPLE 3: In vivo efficacy of compounds of formula (I)

An *in vivo* study was conducted to evaluate the efficacy of compounds of formula (I) in suppressing tumor growth in a human colon HCT116 xenograft model.

As shown in Table 3 below, among five tested compounds, compounds 18b and 20g showed marked anticancer activity with tumor growth inhibition (TGI) values of 74.3% and 83.9%, respectively, via oral administration (100mg/kg, daily) in the human colon HCT116 xenograft model (n =7-8). Compound 20g caused 10.4% weight loss and exhibited narrower therapeutic index as compared to compound 18b. Further, compound 18b unexpectedly exhibited dose-dependent tumor growth inhibition, the highest maximumtolerated dose (MTD) of 250 mg/kg, and a higher therapeutic index of 10, as compared to other tested compounds.

**Table 3. Anti-tumor activity of HSP90 inhibitors in an HCT116 xenograft model (n =7-8).**

| Compound | Dosage (mg/kg) | Schedule | Route | TGI*^{a}* (%) | Loss of Body | MTD*^{b}* (mg/kg) | TI*^{c}* |
|---|---|---|---|---|---|---|---|
| 18b | 25 | qd | po | 55.6 | 0% | 250 | 10 |
| 18b | 100 | qd | po | 74.3 | 0% | - | - |
| 19b | 100 | qd | po | 29.1 | -3.1% Day 5 | - | - |
| 20b | 100 | qd x7/ 5 on 2 off | po | 37.7 | -8.5% Day 5 | < 250 | < 2.5 |
| 20c | 100 | qd x7/ 5 on 2 off | po | 33.1 | -15.4% Day 7 | 100 | 1 |
| 20g | 100 | qd | po | 83.9 | -10.4% Day 7 | < 250 | < 2.5 |

| | | | | | | | |
|---|---|---|---|---|---|---|---|
| *^{a}* TGI: Tumor growth inhibition. *^{b}* MTD: Maximum Tolerated Dose. *^{c}* TI: Therapeutic Index. TI is defined as the ratio of the maximum tolerated dose (MTD) to the minimum efficacious dose (MED). | | | | | | | |

Compound 18b was also evaluated via daily oral administration in the human lung NSCLC A549 cancer xenograft model. This compound unexpectedly suppressed the tumor growth with TGIs of 46.7%, 46.9%, and 50.9% at dosages of 25, 50, and 100 mg/kg, respectively, without significant body weight loss.

These results indicate that compounds of formula (I) have high *in vivo* efficacy in suppressing tumor growth.

### EXAMPLE 4: Evaluation of pharmacokinetic properties of compounds of formula (I)

A pharmacokinetic study was conducted to evaluate the pharmacokinetic properties of compounds of formula (I). Pharmacokinetics is proposed to study the absorption, the distribution, the biotrasformations and the elimination of drugs in man and animals (Rescigno A, Segre G. Drug and Tracer Kinetics. Blaisdell, Waltham (Mass) 1966).

Five compounds of formula (I), i.e., compounds 18b, 19b, 20b, 20c, and 20g, were evaluated for their pharmacokinetic profiles in rat. See Table 4 below. It was observed that compound 18b, when orally dosed at 20 mg/kg, exhibited a long half-life of about 27.7 hours, a low clearance of about 64 ml/min/kg, a high AUC of about 1310 ng/mL* hr, and a good oral bioavailability of 25%.

**Table 4. Pharmacokinetic profiles of HSP90 inhibitors in rat.**

| Compound | IV (2mg/kg) | | | | PO (20mg/kg) | | | | |
|---|---|---|---|---|---|---|---|---|---|
| | T_{1/2} (hr) | CL (ml/min/kg ) | Vₛₛ (1/kg) | AUC_{(0-inf)} (ng/mL* hr) | T_{1/2} (hr) | Cₘₐₓ (ng/ml) | T ₘₐₓ (hr) | AUC_{(0-inf)} (ng/mL* hr) | F (% ) |
| 18b | 4.0±1. 7 | 64 ± 1.8 | 5.5± 1.1 | 520 ± 14 | 27.7±1 3 | 40 ± 13 | 6 ± 2 | 1310±25 2 | 25 |
| 19b | 8.1±4. 7 | 75 ± 4.8 | 12.0±8. 9 | 442 ± 49 | 11± 7.6 | 51± 9 | 0.3±0.1 | 485 ± 103 | 11 |
| 20b | 1.3±0. 3 | 237 ± 37 | 18.0±4. 5 | 142 ± 25 | 7.5± 1.3 | 82 ± 34 | 2.2±1. 8 | 533 ± 154 | 37 |
| 20c | 1.5±0. 5 | 170 ± 64 | 9.0± 2.8 | 234±11 4 | 5.9±1.6 | 67 ± 23 | 2.7±1. 2 | 499 ± 38 | 22 |
| 20g | 0.7±0. 1 | 117 ± 23 | 4.3 ± 1.3 | 288 ± 59 | 2.2±0.6 | 12.8±8.3 | 0.5±0.4 | 36 ± 5 | 1.3 |

These results indicate that compounds of formula (I) have good pharmacokinetic properties suitable for oral administration.

### OTHER EMBODIMENTS

All of the features disclosed in this specification may be combined in any combination.

From the above description, one skilled in the art can easily ascertain the essential characteristics of the present invention, and without departing from the scope thereof which is determined by the following claims, can make various changes and modifications of the invention to adapt it to various usages and conditions. Thus, other embodiments are also within the scope of the following claims.

## Claims

1. A compound which is of formula (I): 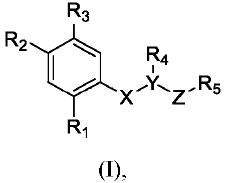 wherein
X is CO;
Y is N;
Z is:
(i) aryl selected from phenyl, 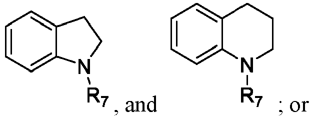
(ii) heteroaryl selected from the group consisting of 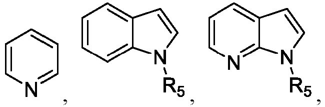 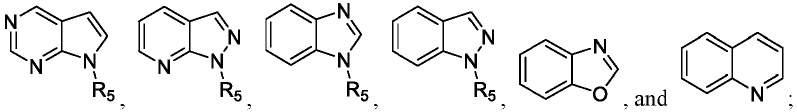
each of R₁ and R₂, independently, is OH or NH₂;
R₃ is C₁₋₆ alkyl or halogen;
R₄ is C₁₋₆ alkyl, C₃₋₈ cycloalkyl, C₁₋₆ alkylamine, or C₁₋₆ alkylalcohol; and
R₅ is CF₃, CN, NO₂, NR₆R₇, -NHCO(CH₂)ₙCONHOH, -CONH(CH₂)ₙCONHOH, CONHOH, CO₂NH₂, C₁₋₆ alkoxyl, C₃₋₁₀ cycloalkyl, C₃₋₁₀ heterocycloalkyl, aryl, or heteroaryl when Z is aryl, providing that when Z is phenyl, R₅ is -NHCO(CH₂)ₙCONHOH or - CONH(CH₂)ₙCONHOH; or
R₅ is H, halogen, CF₃, CN, NO₂, NR₆R₇, -NHCO(CH₂)ₙCONHOH, -CONH(CH₂)ₙCONHOH, CONHOH, CO₂NH₂, CH₂NR₆R₇, C₁₋₆ alkyl, C₁₋₆ alkoxyl, C₃₋₁₀ cycloalkyl, C₃₋₁₀ heterocycloalkyl, aryl, or heteroaryl when Z is heteroaryl,
in which
each of R₆ and R₇, independently, is H, C₁₋₆ alkyl, C₁₋₆ alkoxyl, C₃₋₁₀ cycloalkyl, C₃₋₁₀ heterocycloalkyl, aryl, or heteroaryl; or R₆, together with R₇ and the nitrogen atom bonded to R₆ and R₇, is C₃₋₁₀ heterocycloalkyl; or R₆, together with Z and the nitrogen atom bonded to R₆ and R₇, forms a fused bicycle; and
n is 5 to 7.

2. The compound of claim 1, wherein each of R₁ and R₂ is OH and R₃ is isopropyl.

3. The compound of any one of claims 1 to 2, wherein:
(i) Z is phenyl and R₅ is -NHCO(CH₂)ₙCONHOH or -CONH(CH₂)ₙCONHOH; or
(ii) Z is 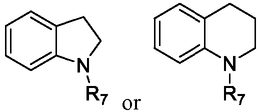 and R₇ is C₁₋₆ alkyl.

4. The compound of any one of claims 1 to 2, wherein Z is 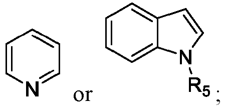 optionally, wherein Z is 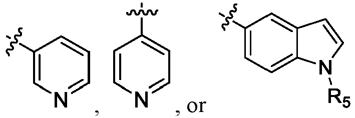 and R₄ is C₁₋₆ alkyl.

5. The compound of any one of claims 1 to 2 and 4, wherein the compound is one of the following compounds:

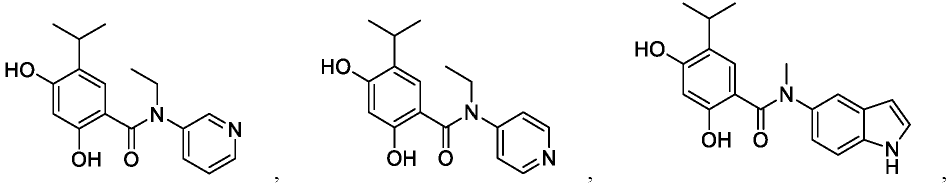

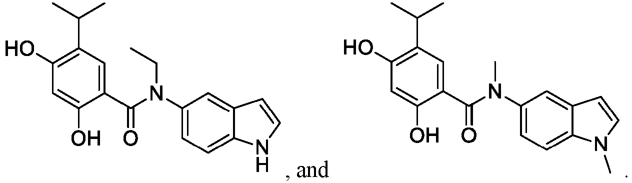

6. A pharmaceutical composition comprising a compound of any one of claims 1 to 5 and a pharmaceutically acceptable carrier.

7. A compound of formula (I) according to claim 1 for use in treating a medical condition associated with HSP90,
wherein the medical condition is cancer; optionally, wherein the cancer is breast cancer, nonsmall cell lung cancer, gastric cancer, lymphoma, or multiple myeloma, and optionally, wherein the compound is for oral administration.

8. The compound for use of claim 7, wherein:
each of R₁ and R₂ is OH, and R₃ is isopropyl.

9. The compound for use of claim 7 or 8, wherein Z is heteroaryl selected from the group consisting of

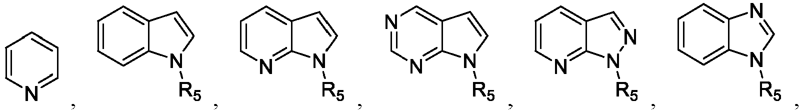

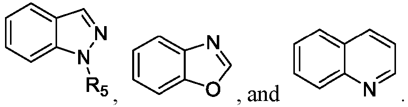

10. The compound for use of any one of claims 7 to 9, wherein the compound is one of the following compounds: 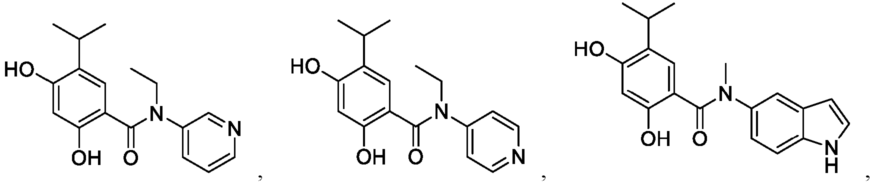 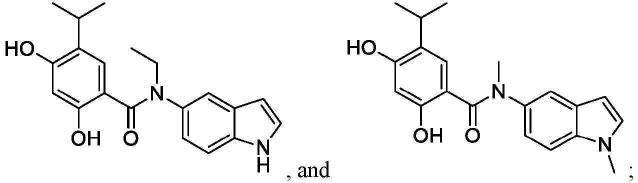 preferably, wherein the compound is

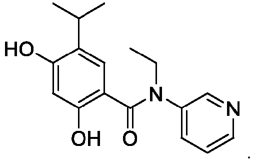

## Patentansprüche

1. Verbindung der Formel (I): 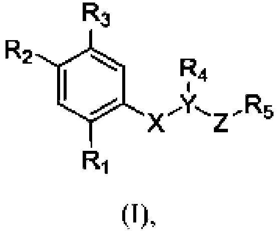 wobei
X CO ist;
Y N ist;
Z Folgendes ist:
(i) Aryl, ausgewählt aus Phenyl, 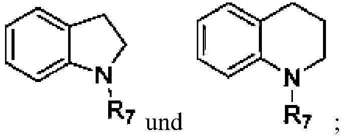 oder
(ii) Heteroaryl, ausgewählt aus der Gruppe, bestehend aus 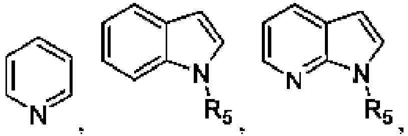 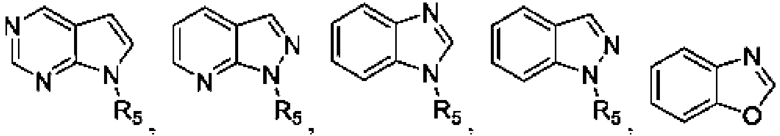 und 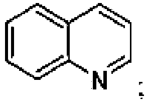
R₁ und R₂ jeweils unabhängig voneinander OH oder NH₂ sind;
R₃ C₁₋₆-Alkyl oder Halogen ist;
R₄ C₁₋₆-Alkyl, C₃₋₈-Cycloalkyl, C₁₋₆-Alkylamin oder C₁₋₆-Alkylalkohol ist; und
R₅ CF₃, CN, NO₂, NR₆R₇, -NHCO(CH₂)ₙCONHOH, -CONH(CH₂)ₙCONHOH, CONHOH, CO₂NH₂, C₁₋₆-Alkoxyl, C₃₋₁₀-Cycloalkyl, C₃₋₁₀-Heterocycloalkyl, Aryl oder Heteroaryl ist, wenn Z Aryl ist, mit der Maßgabe, dass, wenn Z Phenyl ist, R₅ - NHCO(CH₂)ₙCONHOH oder CONH(CH₂)ₙCONHOH ist; oder
R₅ H, Halogen, CF₃, CN, NO₂, NR₆R₇, -NHCO(CH₂)ₙCONHOH, - CONH(CH₂)ₙCONHOH, CONHOH, CO₂NH₂, CH₂NR₆R₇, C₁₋₆-Alkyl, C₁₋₆-Alkoxyl, C₃₋₁₀-Cycloalkyl, C₃₋₁₀-Heterocycloalkyl, Aryl oder Heteroaryl ist, wenn Z Heteroaryl ist,
wobei
R₆ und R₇ jeweils unabhängig voneinander H, C₁₋₆-Alkyl, C₁₋₆-Alkoxyl, C₃₋₁₀-Cycloalkyl, C₃₋₁₀-Heterocycloalkyl, Aryl oder Heteroaryl sind; oder R₆ zusammen mit R₇ und dem an R₆ und R₇ gebundenen Stickstoffatom ein C₃₋₁₀-Heterocycloalkyl ist; oder R₆ zusammen mit Z und dem an R₆ und R₇ gebundenen Stickstoffatom einen kondensierten Bicyclus bildet; und
n 5 bis 7 beträgt.

2. Verbindung nach Anspruch 1, wobei R₁ und R₂ jeweils OH sind und R₃ Isopropyl ist.

3. Verbindung nach einem der Ansprüche 1 bis 2, wobei:
(i) Z Phenyl ist und R₅ -NHCO(CH₂)ₙCONHOH oder -CONH(CH₂)ₙCONHOH ist; oder
(ii) Z 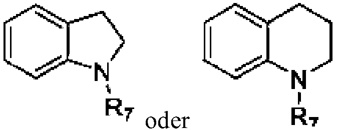 ist und R₇ C₁₋₆-Alkyl ist.

4. Verbindung nach einem der Ansprüche 1 bis 2, wobei Z 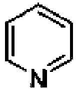 oder 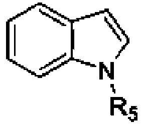 ist; optional wobei Z 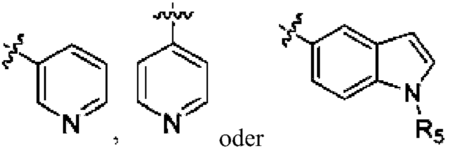 ist und R₄ C₁₋₆-Alkyl ist.

5. Verbindung nach einem der Ansprüche 1 bis 2 und 4, wobei die Verbindung eine der folgenden Verbindungen ist:

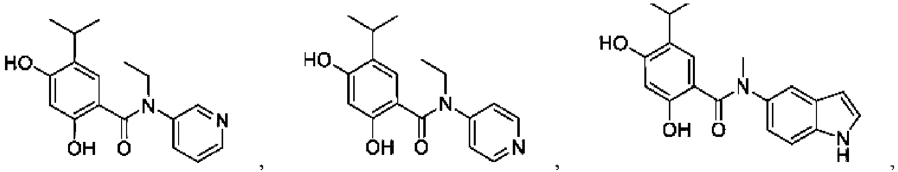

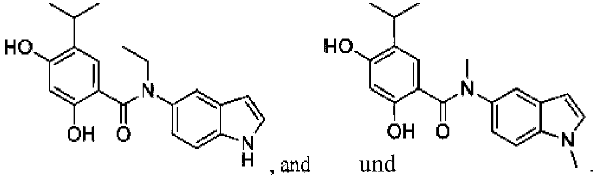

6. Pharmazeutische Zusammensetzung, die eine Verbindung nach einem der Ansprüche 1 bis 5 umfasst und einen annehmbaren Träger enthält.

7. Verbindung der Formel (I) nach Anspruch 1 zur Verwendung bei der Behandlung einer HSP90 zugeordneten Erkrankung,
wobei die Erkrankung Krebs ist; optional, wobei der Krebs Brustkrebs, nichtkleinzelliger Lungenkrebs, Magenkrebs, Lymphom oder Multiples Myelom ist, und optional, wobei die Verbindung zur oralen Verabreichung bestimmt ist.

8. Verbindung zur Verwendung nach Anspruch 7, wobei:
R₁ und R₂ jeweils OH sind und R₃ Isopropyl ist.

9. Verbindung zur Verwendung nach Anspruch 7 oder 8, wobei Z ein Heteroarylrest ist, ausgewählt aus der Gruppe bestehend aus

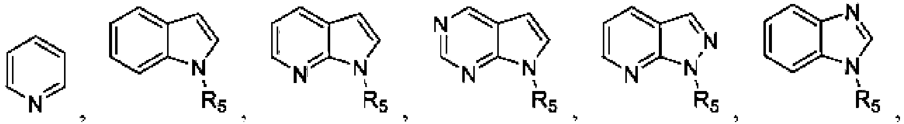

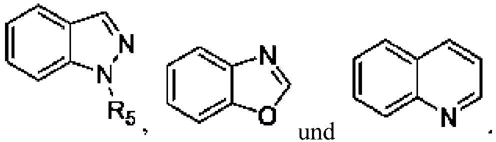

10. Verbindung zur Verwendung nach einem der Ansprüche 7 bis 9, wobei die Verbindung eine der folgenden Verbindungen ist: 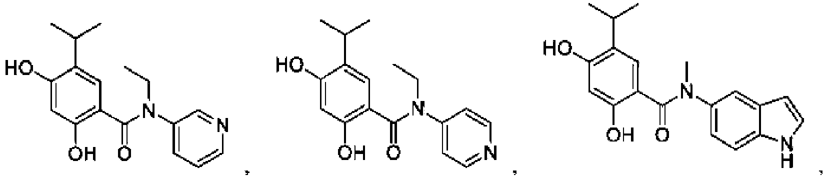 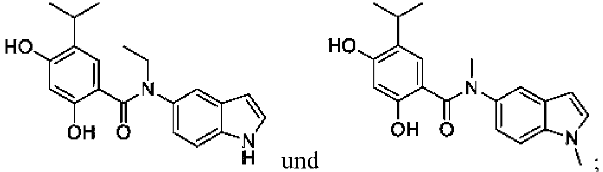 wobei die Verbindung vorzugsweise 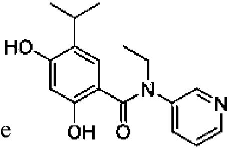 ist.

## Revendications

1. Composé de formule (I) : 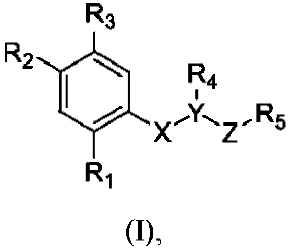 dans lequel :
X est CO ;
Y est N ;
Z est:
(i) aryle choisi parmi phényle, 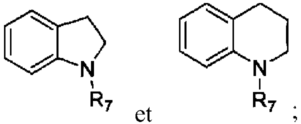 ou
(ii) hétéroaryle choisi dans le groupe constitué de 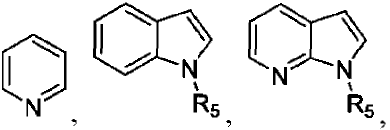 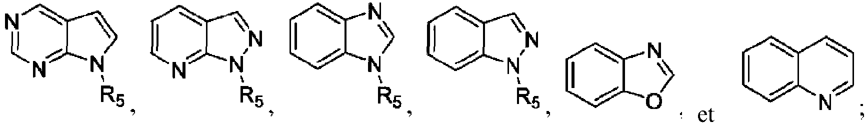
chacun de R₁ et R₂, indépendamment, est OH ou NH₂ ;
R₃ est un alkyle en C₁₋₆ ou un halogène ;
R₄ est un alkyle C₁₋₆, un cycloalkyle C₃₋₈, une alkylamine en C₁₋₆ ou un alkylalcool en C₁₋₆ ; et
R₅ est CF₃, CN, NO₂, NR₆R₇, -NHCO(CH₂)ₙCONHOH, -CONH(CH₂)ₙCONHOH, CONHOH, CO₂NH₂, un alkoxyle en C₁₋₆, un cycloalkyle en C₃₋₁₀, un hétérocycloalkyle en C₃₋₁₀, un aryle, ou hétéroaryle lorsque Z est aryle, à condition que lorsque Z est phényle, R₅ soit -NHCO(CH₂)ₙCONHOH ou -CONH(CH₂)ₙCONHOH ; ou
R₅ est H, un halogène, CF₃, CN, NO₂, NR₆R₇, -NHCO(CH₂)ₙCONHOH, - CONH(CH₂)ₙCONHOH, CONHOH, CO₂NH₂, CH₂NR₆R₇, un alkyle en C₁₋₆, un alkoxyle en C₁₋₆, un cycloalkyle en C₃₋₁₀, un hétérocycloalkye en C₃₋₁₀, aryle, ou hétéroaryle lorsque Z est hétéroaryle,
dans lequel
chacun de R₆ et R₇, indépendamment, est H, un alkyle en C₁₋₆, un alkoxyle en C₁₋₆, un cycloalkyle en C₃₋₁₀, un hétérocycloalkyle en C₃₋₁₀, aryle, ou hétéroaryle ; ou R₆, conjointement avec R₇ et l'atome d'azote lié à R₆ et R₇, est un hétérocycloalkyle en C₃₋₁₀ ; ou R₆, conjointement avec Z et l'atome d'azote lié à R₆ et R₇, forme un bicycle fusionné ; et
n est de 5 à 7.

2. Composé selon la revendication 1, dans lequel chacun de R₁ et R₂ est OH et R₃ est isopropyle.

3. Composé selon l'une quelconque des revendications 1 à 2, dans lequel :
(i) Z est phényle et R₅ est -NHCO(CH₂)ₙCONHOH ou -CONH(CH₂)ₙCONHOH ; ou
(ii) Z est 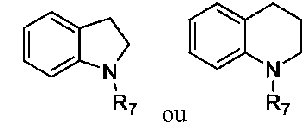 et R₇ est un alkyle en C₁₋₆.

4. Composé selon l'une quelconque des revendications 1 à 2, dans lequel Z est 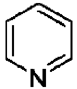 ou 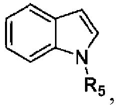 éventuellement,
dans lequel Z est 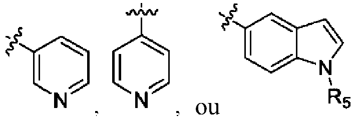 et R₄ est un alkyle en C₁₋₆.

5. Composé selon l'une quelconque des revendications 1 à 2 et 4, dans lequel le composé est un parmi les composés suivants :

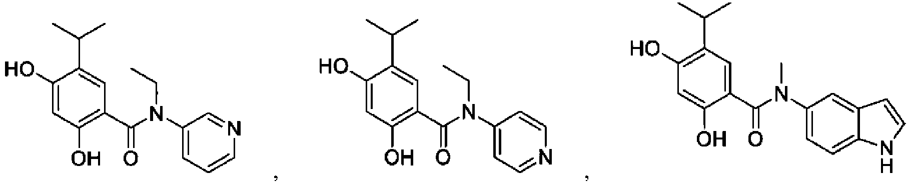

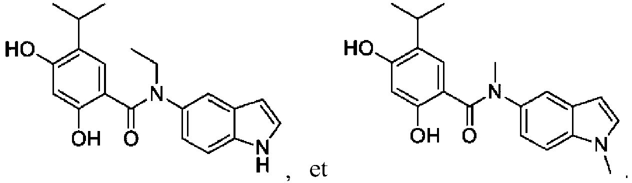

6. Composition pharmaceutique comprenant un composé selon l'une quelconque des revendications 1 à 5 et un véhicule pharmaceutiquement acceptable.

7. Composé de formule (I) selon la revendication 1 destiné à être utilisé dans le traitement d'un état médical associé à HSP90,
dans lequel l'état médical est un cancer ; éventuellement, le cancer étant un cancer du sein, un cancer du poumon non à petites cellules, un cancer gastrique, un lymphome, ou un myélome multiple, et optionnellement, dans lequel le composé est destiné à une administration orale.

8. Composé destiné à l'utilisation selon la revendication 7, dans lequel :
chacun de R₁ et R₂ est OH, et R₃ est un isopropyle.

9. Composé destiné à l'utilisation selon la revendication 7 ou 8, dans lequel Z est un hétéroaryle choisi dans le groupe constitué de

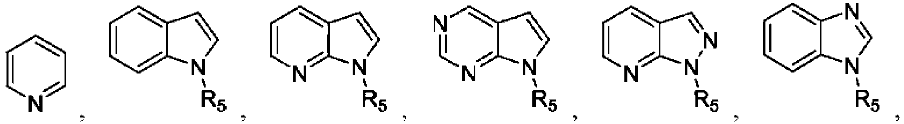

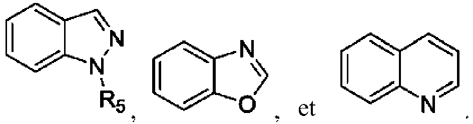

10. Composé destiné à l'utilisation selon l'une quelconque des revendications 7 à 9, dans lequel le composé est un parmi les composés suivants : 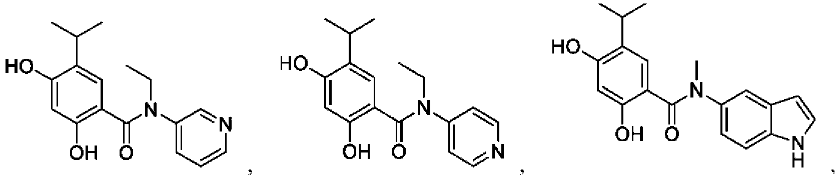 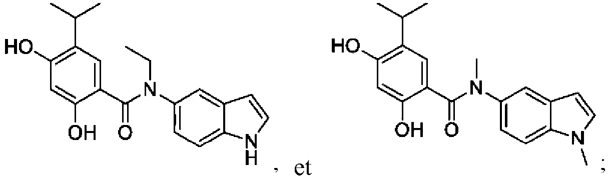 de préférence, le composé étant
